# EUROPEAN PATENT SPECIFICATION

(11) **EP 4 011 897 B1**
(45) Date of publication and mention of the grant of the patent: **04.11.2026**
(21) Application number: 20851145.1
(22) Date of filing: 05.08.2020
(51) Int. Cl.: C07J 43/00, C07J 5/00, C07J 7/00, C07D 231/14, A61K 31/57, A61K 31/573, A61K 31/58, A61P 25/00, C07D 231/12, C07D 249/04, C07D 257/04, C07D 403/06, C07D 403/08, C07D 413/04

(54) **SALT AND CRYSTAL FORM OF STEROID DERIVATIVE REGULATOR**
SALZ UND KRISTALLFORM EINES STEROIDDERIVATREGULATORS
SEL ET FORME CRISTALLINE D'UN RÉGULATEUR DE DÉRIVÉ DE STÉROÏDE

(30) Priority: 07.08.2019 CN 201910726659
(43) Date of publication of application: 15.06.2022
(73) Proprietor: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: LI, Zongbin, Shanghai 201203 (CN); KUANG, Yuanzhuo, Shanghai 201203 (CN); GUO, Linsong, Shanghai 201203 (CN)
(74) Representative: EP&C
(86) International application number: PCT/CN2020/107061
(87) International publication number: WO 2021/023213

(56) References cited:
- EP-A1- 3 750 908
- WO-A1-2016/084790
- WO-A1-2018/013615
- WO-A1-2019/113494
- WO-A1-2019/126741
- WO-A1-2019/154247
- WO-A1-2019/154257
- WO-A1-2019/154257
- WO-A1-93/03732
- WO-A1-95/21617
- CN-A- 104 136 452
- CN-A- 105 339 381
- CN-A- 110 088 091
- SHEIKHOLESLAMZADEH EHSAN ET AL: "Optimal Solvent Screening for the Crystallization of Pharmaceutical Compounds from Multisolvent Systems", INDUSTRIAL & ENGINEERING CHEMISTRY RESEARCH, vol. 51, no. 42, 3 October 2012 (2012-10-03), pages 13792 - 13802, XP093078644, ISSN: 0888-5885, DOI: 10.1021/ie3014742

## Description

### FIELD OF THE INVENTION

The present invention belongs to the field of drug synthesis, and in particular relates to a salt of a steroid derivative regulator, a crystal form thereof, a method for preparing the same, and a use thereof.

### BACKGROUND OF THE INVENTION

GABA_{A} receptor is a chemically-gated channel on the cell membrane and belongs to ionic receptors. GABA_{A} receptor is widely distributed in the nervous system, and can bind to inhibitory neurotransmitter GABA (gamma-aminobutyric acid), leading to the opening of chloride channels and inhibition of neurons. GABA_{A} receptor regulator (tetrahydroprogesterone) is a positive regulator of GABA_{A} receptor. The binding of tetrahydroprogesterone to intrasynaptic GABA_{A} receptor regulator can increase the opening frequency of chloride channel on the receptor and the influx of chloride ion, thereby increasing the Phasic current, producing a rapid inhibitory effect, reducing nerve excitability, and providing an anti-anxiety and anti-depression effect. The binding of tetrahydroprogesterone to extrasynaptic GABA_{A} receptor provides a continuous chloride ion current, and mediates a lasting and sustained inhibitory effect. Tetrahydroprogesterone can also increase the content of brain derived neurotrophic factor (BDNF), promote the regeneration of hippocampal neurons, and provide a neuroprotective effect, thereby improving anxiety and depression symptoms; but the specific mechanism of action is not clear yet.

Major depressive disorder (MDD) is a common, chronic and recurrent disease. The burden and adverse consequence caused by it are becoming more and more serious. In the past 40 years, the research and clinical application of antidepressants have greatly developed. However, most antidepressants (fluoxetine, paroxetine, sertraline, fluvoxamine, citalopram, etc.) take 2 to 4 weeks to have an effect. The clinical treatment of major depressive disorder patients, especially patients with suicidal tendency, often needs to be prompt and rapid, thus there is an urgent need to develop fast-acting antidepressants.

In the past two decades, there has been little innovation in the research and development of depression treatment. The development goal of GABA_{A} receptor regulators is to change the expectation of patients by changing the treatment regimen of MDD. If successfully developed, the GABA_{A} receptor regulator may become the first drug that provides a truly new mechanism of action for the treatment of depression in more than two decades. At present, foreign pharmaceutical companies, including Sage Therapeutics and Marinus etc., are doing their best to develop GABA_{A} receptor regulators.

Published patent applications related to GABA_{A} receptor regulators include: WO2003077919, WO2014169833, WO2016061537, WO2015180679, WO2015027227 and, in particular, WO2018013615, the latter document disclosing a CNS active 16-methyl substituted steroid compound.

GABA_{A} receptor regulators, as a popular target in the pharmaceutical industry, currently have a good application prospect.

First, GABA_{A} receptor regulators can be applied to major depressive disorder (MDD). The annual incidence of MDD in China is about 2%, thus there is a huge market potential.

Second, existing antidepressants take a long time, commonly 3 to 4 weeks, to have an effect, have a high failure rate up to 40%, and require long-term medication. GABA_{A} receptor regulators can provide a significant antidepressant effect within 24 hours, and the effect can last for several days to two weeks.

Third, GABA_{A} receptor regulators can meet the treatment need of MDD patients with oral administration once a day.

The PCT patent applications (PCT/CN2019/074134 and PCT/CN2019/074108) of Jiangsu Hansoh Pharmaceutical Group Co., Ltd. disclose the structure of a series of steroid derivative regulators. In the subsequent research and development, in order to obtain a product that can be readily processed, filtered and dried and to achieve features such as convenient storage and long-term stability, the present invention has conducted a comprehensive study on the salts of the above substances, and is dedicated to obtaining the most suitable salts and crystal forms.

### SUMMARY OF THE INVENTION

The objective of the present invention is to provide an acid addition salt of formula (Ia), having a structure as shown in formula (Ia): 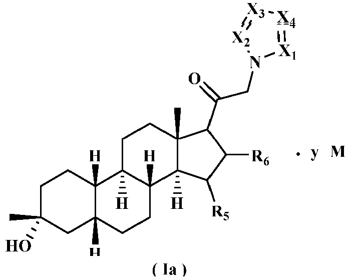 wherein:
X₁ is selected from the group consisting of CR₁ and N, and preferably N;
X₂ is selected from the group consisting of CR₂ and N;
X₃ is selected from the group consisting of CR₃ and N;
X₄ is selected from the group consisting of CR₄ and N;
R₁ and R₂ are each independently selected from the group consisting of hydrogen, deuterium, cyano, halogen, nitro, amino, C₁₋₈ alkyl, C₁₋₈ haloalkyl, C₁₋₈ alkoxy, C₁₋₈ hydroxyalkyl, cyano-substituted C₁₋₈ alkyl, C₃₋₈ cycloalkyl, cyano-substituted C₃₋₈ cycloalkyl, 3 to 8 membered heterocyclyl, C₆₋₁₀ aryl and 5 to 10 membered heteroaryl;
R₃ and R₄ are each independently selected from the group consisting of hydrogen, deuterium, cyano, halogen, nitro, amino, C₁₋₈ alkyl, C₁₋₈ haloalkyl, C₁₋₈ alkoxy, C₁₋₈ hydroxyalkyl, cyano-substituted C₁₋₈ alkyl, C₃₋₈ cycloalkyl, cyano-substituted C₃₋₈ cycloalkyl, 3 to 8 membered heterocyclyl, C₆₋₁₀ aryl, 5 to 10 membered heteroaryl, - (CH₂)ₙRₐ, -(CH₂)ₙORₐ, -(CH₂)ₙSRₐ, -(CH₂)ₙC(O)Rₐ, -S(O)Rₐ, -S(O)₂Rₐ, -S(O)(=NH)Rₐ, - C(O)ORₐ and -C(O)O(CH₂)ₙNRₐR_{b}, wherein the C₁₋₈ alkyl, C₁₋₈ haloalkyl, C₁₋₈ hydroxyalkyl, cyano-substituted C₁₋₈ alkyl, C₁₋₈ alkoxy, C₃₋₈ cycloalkyl, cyano-substituted C₃₋₈ cycloalkyl, 3 to 8 membered heterocyclyl, C₆₋₁₀ aryl and 5 to 10 membered heteroaryl can be each optionally further substituted;
R₅ is independently selected from the group consisting of C₁₋₈ alkyl, C₂₋₈ alkenyl, C₂₋₈ alkynyl, C₁₋₈ haloalkyl, C₁₋₈ alkoxy, C₁₋₈ hydroxyalkyl, cyano-substituted C₁₋₈ alkyl, C₁₋₈ alkylthio, C₃₋₈ cycloalkyl, cyano-substituted C₃₋₈ cycloalkyl, 3 to 8 membered heterocyclyl, C₆₋₁₀ aryl, 5 to 10 membered heteroaryl, -(CH₂)ₙRₐ, -(CH₂)ₙORₐ, -S(O)Rₐ, - S(O)₂Rₐ and -(CH₂)ₙNRₐR_{b}, wherein the C₁₋₈ alkyl, C₂₋₈ alkenyl, C₂₋₈ alkynyl, C₁₋₈ haloalkyl, C₁₋₈ hydroxyalkyl, cyano-substituted C₁₋₈ alkyl, C₁₋₈ alkoxy, C₃₋₈ cycloalkyl, cyano-substituted C₃₋₈ cycloalkyl, 3 to 8 membered heterocyclyl, C₆₋₁₀ aryl and 5 to 10 membered heteroaryl can be each optionally further substituted; R₆ is hydrogen;
or, R₅ and R₆ together with the carbon atoms to which they are attached form a C₃₋₈ cycloalkyl or 3 to 8 membered heterocyclyl, wherein the C₃₋₈ cycloalkyl and 3 to 8 membered heterocyclyl can be each optionally further substituted;
Rₐ and R_{b} are each independently selected from the group consisting of hydrogen, deuterium, halogen, cyano, hydroxy, amino, nitro, C₁₋₈ alkyl, C₁₋₈ alkoxy, C₁₋₈ hydroxyalkyl, C₁₋₈ deuterated alkyl, C₁₋₈ haloalkyl, C₃₋₈ cycloalkyl, 3 to 8 membered heterocyclyl, C₆₋₁₀ aryl and 5 to 10 membered heteroaryl, wherein the C₁₋₈ alkyl, C₁₋₈ deuterated alkyl, C₁₋₈ haloalkyl, C₃₋₈ cycloalkyl, 3 to 8 membered heterocyclyl, C₆₋₁₀ aryl and 5 to 10 membered heteroaryl can be each optionally further substituted;
M is an inorganic acid or organic acid, wherein the inorganic acid is selected from the group consisting of hydrochloric acid, sulfuric acid, nitric acid, hydrobromic acid, hydrofluoric acid, hydroiodic acid and phosphoric acid, and the organic acid is selected from the group consisting of 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, capric acid, caproic acid, caprylic acid, cinnamic acid, citric acid, cyclamic acid, camphorsulfonic acid, aspartic acid, camphor acid, gluconic acid, glucuronic acid, glutamic acid, erythorbic acid, lactic acid, malic acid, mandelic acid, pyroglutamic acid, tartaric acid, lauryl sulfuric acid, dibenzoyl tartaric acid, ethane-1,2-disulfonic acid, ethanesulfonic acid, formic acid, fumaric acid, galactonic acid, gentisic acid, glutaric acid, 2-ketoglutaric acid, glycolic acid, hippuric acid, isethionic acid, lactobionic acid, ascorbic acid, asparagic acid, lauric acid, camphor acid, maleic acid, malonic acid, D-tartaric acid, pamoic acid, methanesulfonic acid, 1,5-naphthalenedisulfonic acid, naphthalene-2-sulfonic acid, nicotinic acid, oleic acid, orotic acid, oxalic acid, palmitic acid, embonic acid, propionic acid, salicylic acid, 4-aminosalicylic acid, sebacic acid, stearic acid, succinic acid, thiocyanic acid, undecylenic acid, trifluoroacetic acid, benzenesulfonic acid, p-toluenesulfonic acid and L-malic acid; and
y is selected from the group consisting of 1, 2 and 3;
n is an integer from 0 to 6.

In a preferred embodiment of the present invention, in the acid addition salt of formula (Ia):
R₁ and R₂ are each independently selected from the group consisting of hydrogen, cyano, halogen, C₁₋₆ alkyl and C₃₋₆ cycloalkyl, and preferably hydrogen, cyano, halogen, C₁₋₃ alkyl and C₃₋₆ cycloalkyl;
R₃ and R₄ are each independently selected from the group consisting of hydrogen, cyano, halogen, nitro, C₁₋₆ alkyl, C₁₋₆ haloalkyl, C₁₋₆ hydroxyalkyl, cyano-substituted C₁₋₆ alkyl, C₃₋₆ cycloalkyl, cyano-substituted C₃₋₆ cycloalkyl, 5 or 6 membered heteroaryl, - (CH₂)ₙSRₐ, -(CH₂)ₙC(O)Rₐ, -S(O)Rₐ, -S(O)₂Rₐ, -S(O)(=NH)Rₐ, -C(O)ORₐ and - C(O)O(CH₂)ₙNRₐR_{b};
R₅ is selected from the group consisting ofC₁₋₆ alkyl, C₁₋₆ haloalkyl, C₁₋₆ hydroxyalkyl, cyano-substituted C₁₋₆ alkyl, C₁₋₆ alkoxy, C₁₋₆ alkylthio, C₃₋₆ cycloalkyl, cyano-substituted C₃₋₆ cycloalkyl, 3 to 6 membered heterocyclyl, C₆₋₁₀ aryl, 5 to 10 membered heteroaryl, -(CH₂)ₙRₐ, -(CH₂)ₙORₐ, -S(O)₂Rₐ and -(CH₂)ₙNRₐR_{b}, wherein the 5 to 10 membered heteroaryl can be optionally further substituted;
R₆ is hydrogen;
or, R₅ and R₆ together with the carbon atoms to which they are attached form a C₃₋₈ cycloalkyl, wherein the C₃₋₈ cycloalkyl can be optionally further substituted;
Rₐ and R_{b} are each independently selected from the group consisting of hydrogen, halogen, cyano, nitro, C₁₋₆ alkyl, 3 to 6 membered heterocyclyl and 5 to 10 membered heteroaryl, wherein the C₁₋₆ alkyl, 3 to 6 membered heterocyclyl and 5 to 10 membered heteroaryl can be each optionally further substituted;
M is selected from the group consisting of hydrochloric acid, sulfuric acid, nitric acid, hydrobromic acid, phosphoric acid, methanesulfonic acid, 1,5-naphthalenedisulfonic acid and *p*-toluenesulfonic acid; and
y is selected from the group consisting of 1 and 2;
n is an integer from 0 to 3.

In a preferred embodiment of the present invention, in the acid addition salt of formula (Ia):
R₁ and R₂ are each independently selected from the group consisting of hydrogen, fluorine, chlorine, cyano, methyl and cyclopropyl;
R₃ and R₄ are each independently selected from the group consisting of hydrogen, halogen, cyano, nitro, C₁₋₃ alkyl, cyano-substituted C₁₋₃ alkyl, halogen-substituted C₁₋₃ alkyl, C₁₋₃ hydroxyalkyl, C₃₋₆ cycloalkyl, cyano-substituted C₃₋₆ cycloalkyl, oxazolyl, - C(O)Rₐ, -SC₁₋₃ alkyl, -S(O)C₁₋₃ alkyl, -S(O)₂C₁₋₃ alkyl, -C(O)OC₁₋₃ alkyl, -S(O)(=NH)C₁₋₃ alkyl and -C(O)O(CH₂)ₙN(CH₃)C₁₋₃ alkyl, and preferably hydrogen, fluorine, chlorine, cyano, nitro, trifluoromethyl, cyclopropyl, cyano-substituted cyclopropyl, cyano-substituted isopropyl, hydroxyisopropyl, oxazolyl, -C(O)Rₐ, -SCH₃, -S(O)CH₃, - S(O)₂CH₃, -C(O)OCH₂CH₃, -S(O)(=NH)CH₃ and -C(O)O(CH₂)₂NCH₃(CH₃);
R₅ is selected from the group consisting of C₁₋₃ alkyl, C₁₋₃ haloalkyl, C₁₋₃ alkoxy, C₁₋₃ hydroxyalkyl, cyano-substituted C₁₋₃ alkyl, C₃₋₆ cycloalkyl, cyano-substituted C₃₋₆ cycloalkyl, 3 to 6 membered nitrogen-containing heterocyclyl, C₆₋₁₀ aryl, 5 to 10 membered heteroaryl, C₁₋₃ alkylthio, -(CH₂)ₙRₐ, -(CH₂)ₙORₐ, -S(O)₂Rₐ and -(CH₂)ₙN(Rₐ)₂, and preferably hydrogen, fluorine, chlorine, cyano, methyl, ethyl, isopropyl, fluoromethyl, trifluoromethyl, methoxy, cyclopropyl, cyclobutyl, methyl-substituted pyrazolyl, phenyl, -SCH₃, -(CH₂)ₙRₐ, -NC(CH₃)₂, -S(O)₂C(CH₃)₂ and -CH₂N(CH₃)₂;
R₆ is hydrogen;
or, R₅ and R₆ together with the carbon atoms to which they are attached form a C₃₋₆ cycloalkyl, and preferably cyclopropyl, wherein the cyclopropyl is optionally substituted by fluorine;
Rₐ is selected from the group consisting of hydrogen, nitro, C₁₋₃ alkyl, azetidinyl and pyrrolidinyl, wherein the azetidinyl and pyrrolidinyl are each optionally substituted by halogen, and preferably by fluorine;
M is selected from the group consisting of hydrochloric acid, sulfuric acid, methanesulfonic acid, *p*-toluenesulfonic acid, hydrobromic acid, nitric acid and 1,5-naphthalenedisulfonic acid, and preferably methanesulfonic acid;
y is 1 or 2;
n is selected from the group consisting of 0, 1 and 2.

In a preferred embodiment of the present invention, the acid addition salt of formula (Ia) has a structure as shown in formula (IIa):

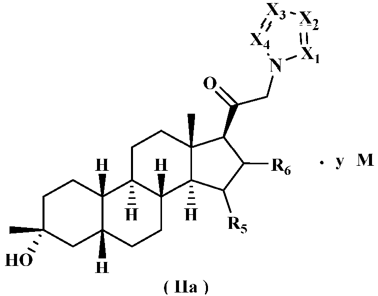

In a preferred embodiment of the present invention, the acid addition salt of formula (Ia) has a structure as shown in formula (IIIa): 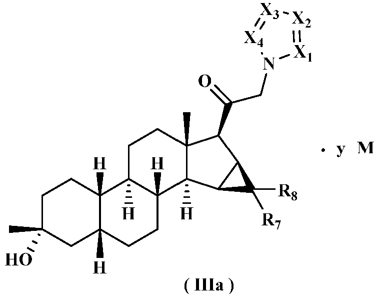 wherein:
R₇ and R₈ are each independently selected from the group consisting of hydrogen, fluorine, chlorine, bromine and methyl, and preferably hydrogen and fluorine.

In a preferred embodiment of the present invention, the acid addition salt of formula (Ia) has a structure as shown in formula (IVa):

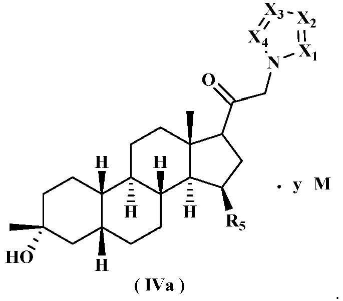

In a preferred embodiment of the present invention, the acid addition salt of formula (Ia) has a structure as shown in formula (Va):

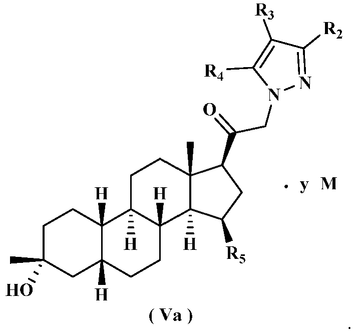

In a preferred embodiment of the present invention, the acid addition salt of formula (Ia) has a structure as follows:

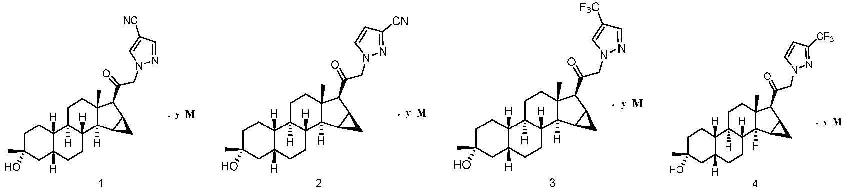

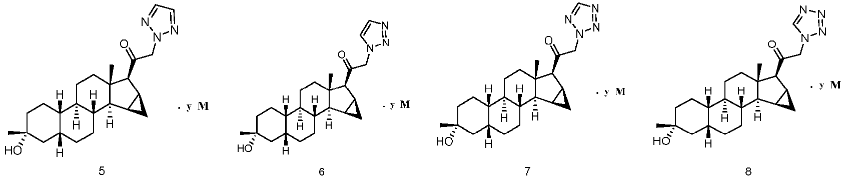

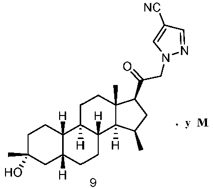

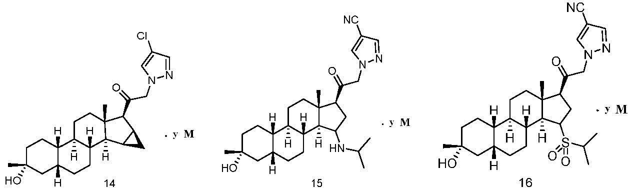

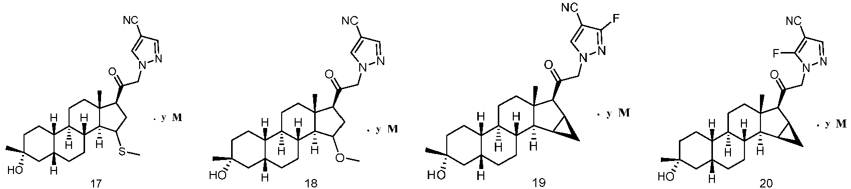

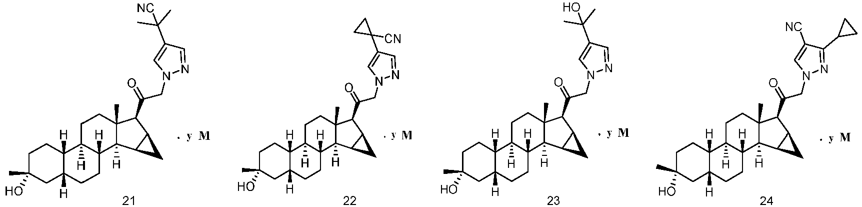

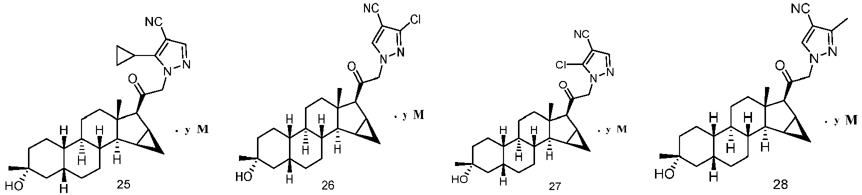

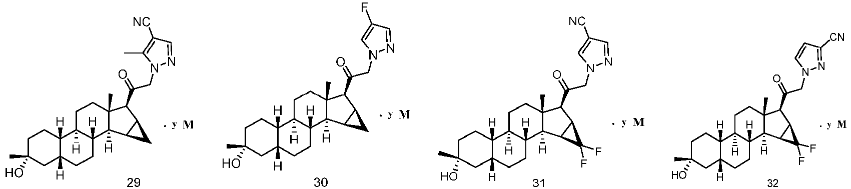

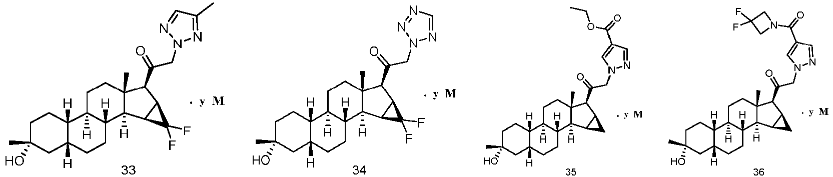

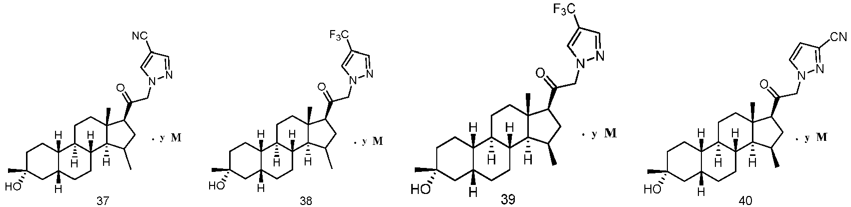

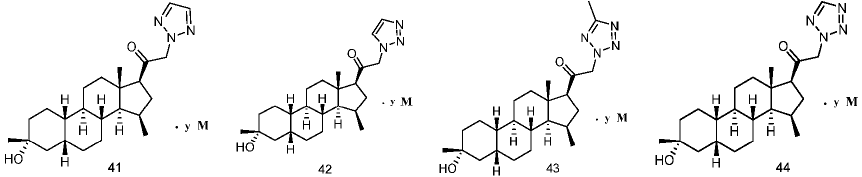

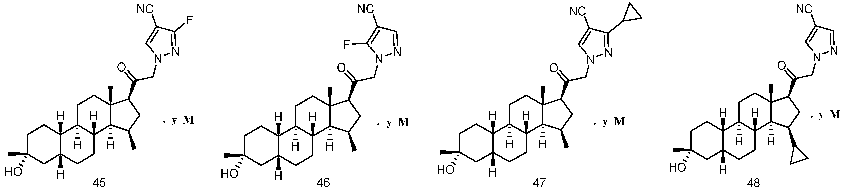

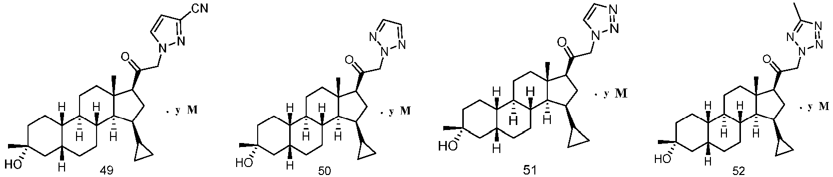

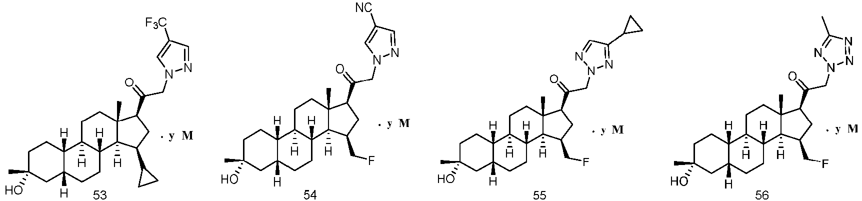

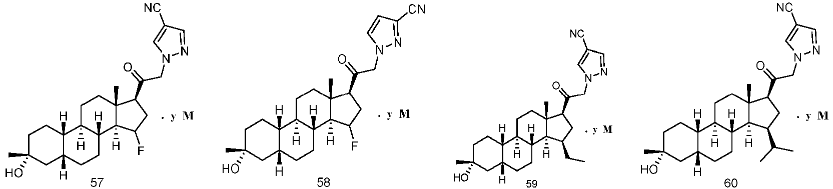

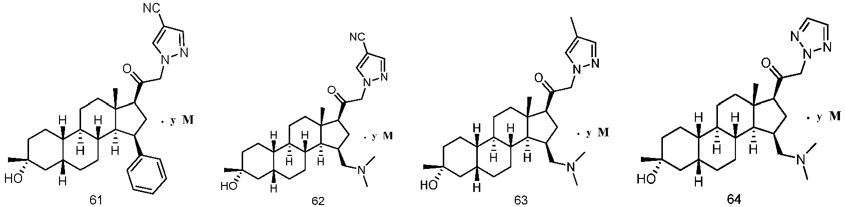

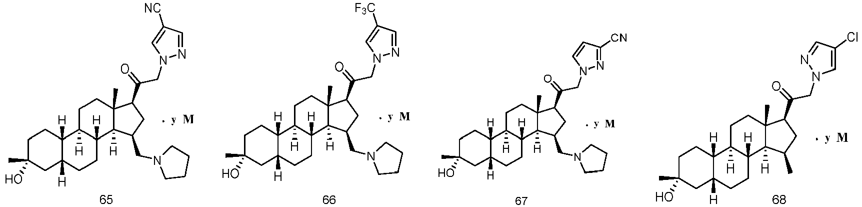

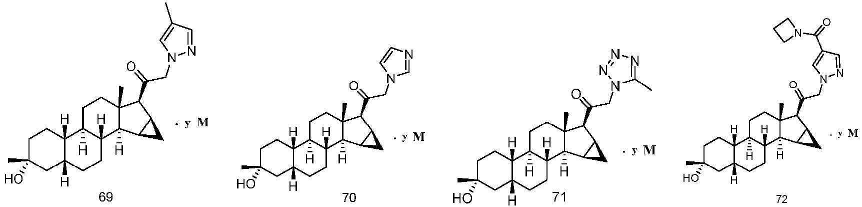

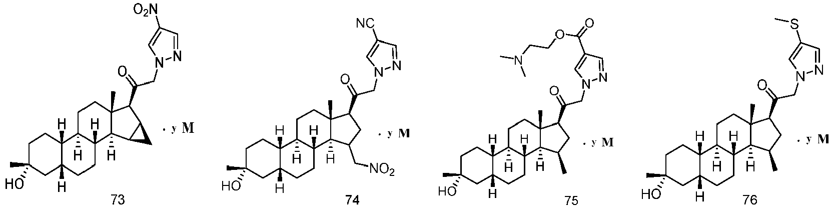

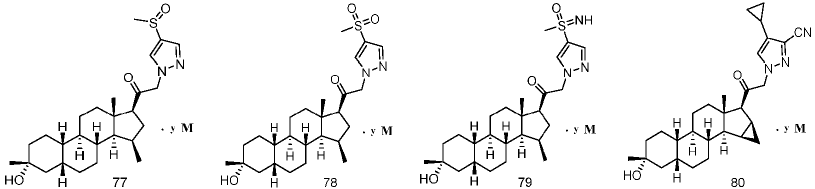

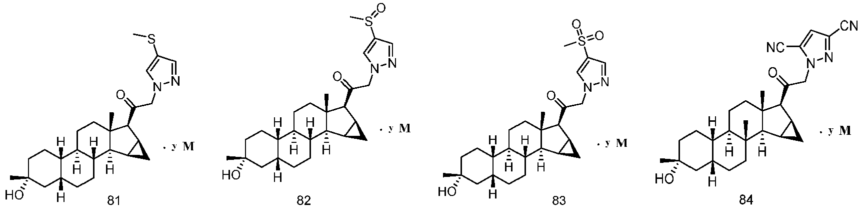

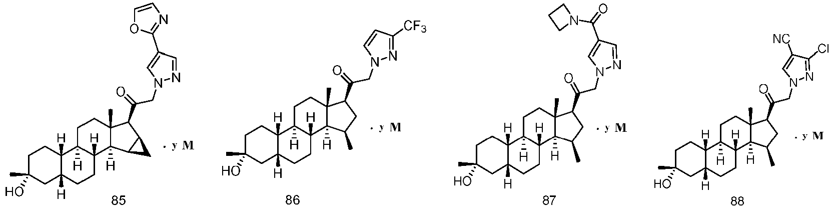

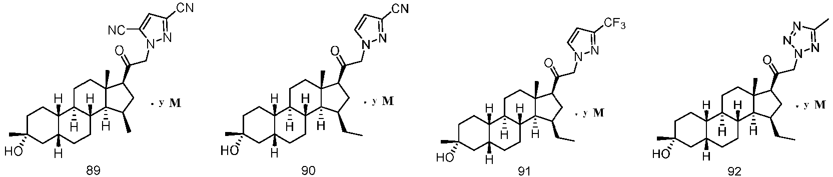

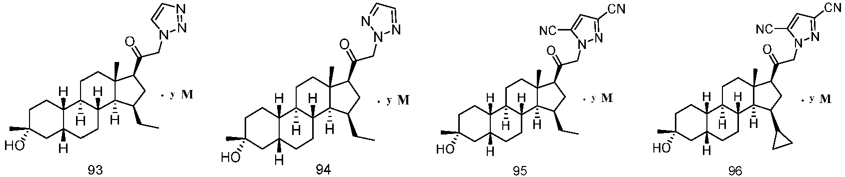

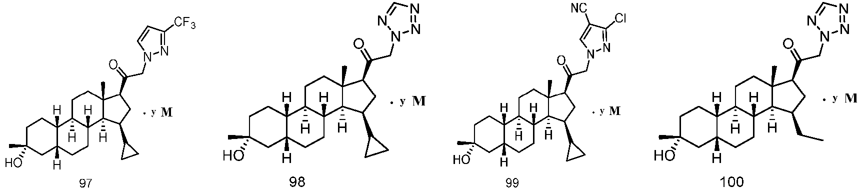

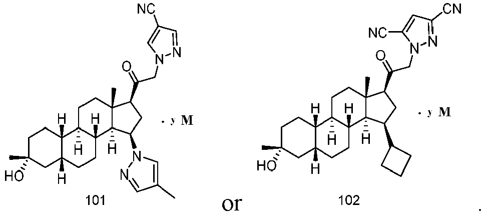

In certain embodiments of the present invention, the acid addition salt of formula (Ia) has a structure as follows:

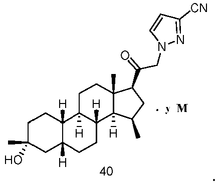

In a preferred embodiment of the present invention, the acid addition salt of formula (Ia) has a structure as shown in formula (VIa):

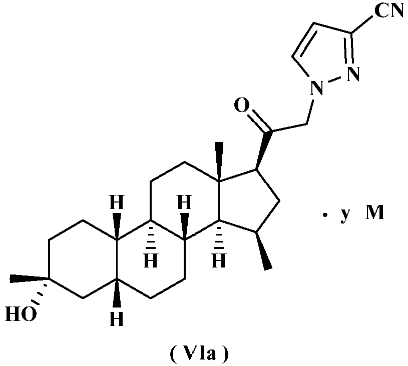

M is methanesulfonic acid, and y is 1.

In a preferred embodiment of the present invention, the compound of formula (Ia) is characterized in that the compound of formula (VIa) is amorphous.

Disclosed herein but not defining the scope of the invention is a crystal form of the compound of formula (I), having a structure as shown in formula (I): 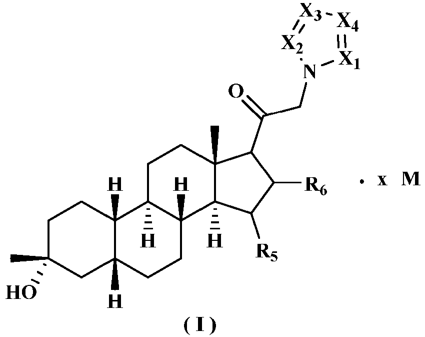 wherein:
X₁ is selected from the group consisting of CR₁ and N, and preferably N;
X₂ is selected from the group consisting of CR₂ and N;
X₃ is selected from the group consisting of CR₃ and N;
X₄ is selected from the group consisting of CR₄ and N;
R₁ and R₂ are each independently selected from the group consisting of hydrogen, deuterium, cyano, halogen, nitro, amino, C₁₋₈ alkyl, C₁₋₈ haloalkyl, C₁₋₈ alkoxy, C₁₋₈ hydroxyalkyl, cyano-substituted C₁₋₈ alkyl, C₃₋₈ cycloalkyl, cyano-substituted C₃₋₈ cycloalkyl, 3 to 8 membered heterocyclyl, C₆₋₁₀ aryl and 5 to 10 membered heteroaryl;
R₃ and R₄ are each independently selected from the group consisting of hydrogen, deuterium, cyano, halogen, nitro, amino, C₁₋₈ alkyl, C₁₋₈ haloalkyl, C₁₋₈ alkoxy, C₁₋₈ hydroxyalkyl, cyano-substituted C₁₋₈ alkyl, C₃₋₈ cycloalkyl, cyano-substituted C₃₋₈ cycloalkyl, 3 to 8 membered heterocyclyl, C₆₋₁₀ aryl, 5 to 10 membered heteroaryl, - (CH₂)ₙRₐ, -(CH₂)ₙORₐ, -(CH₂)ₙSRₐ, -(CH₂)ₙC(O)Rₐ, -S(O)Rₐ, -S(O)₂Rₐ, -S(O)(=NH)Rₐ, - C(O)ORₐ and -C(O)O(CH₂)ₙNRₐR_{b}, wherein the C₁₋₈ alkyl, C₁₋₈ haloalkyl, C₁₋₈ hydroxyalkyl, cyano-substituted C₁₋₈ alkyl, C₁₋₈ alkoxy, C₃₋₈ cycloalkyl, cyano-substituted C₃₋₈ cycloalkyl, 3 to 8 membered heterocyclyl, C₆₋₁₀ aryl and 5 to 10 membered heteroaryl can be each optionally further substituted;
R₅ is independently selected from the group consisting of C₁₋₈ alkyl, C₂₋₈ alkenyl, C₂₋₈ alkynyl, C₁₋₈ haloalkyl, C₁₋₈ alkoxy, C₁₋₈ hydroxyalkyl, cyano-substituted C₁₋₈ alkyl, C₁₋₈ alkylthio, C₃₋₈ cycloalkyl, cyano-substituted C₃₋₈ cycloalkyl, 3 to 8 membered heterocyclyl, C₆₋₁₀ aryl, 5 to 10 membered heteroaryl, -(CH₂)ₙRₐ, -(CH₂)ₙORₐ, -S(O)Rₐ, - S(O)₂Rₐ and -(CH₂)ₙNRₐR_{b}, wherein the C₁₋₈ alkyl, C₂₋₈ alkenyl, C₂₋₈ alkynyl, C₁₋₈ haloalkyl, C₁₋₈ hydroxyalkyl, cyano-substituted C₁₋₈ alkyl, C₁₋₈ alkoxy, C₃₋₈ cycloalkyl, cyano-substituted C₃₋₈ cycloalkyl, 3 to 8 membered heterocyclyl, C₆₋₁₀ aryl and 5 to 10 membered heteroaryl can be each optionally further substituted; R₆ is hydrogen;
or, R₅ and R₆ together with the carbon atoms to which they are attached form a C₃₋₈ cycloalkyl or 3 to 8 membered heterocyclyl, wherein the C₃₋₈ cycloalkyl and 3 to 8 membered heterocyclyl can be each optionally further substituted;
Rₐ and R_{b} are each independently selected from the group consisting of hydrogen, deuterium, halogen, cyano, hydroxy, amino, nitro, C₁₋₈ alkyl, C₁₋₈ alkoxy, C₁₋₈ hydroxyalkyl, C₁₋₈ deuterated alkyl, C₁₋₈ haloalkyl, C₃₋₈ cycloalkyl, 3 to 8 membered heterocyclyl, C₆₋₁₀ aryl and 5 to 10 membered heteroaryl, wherein the C₁₋₈ alkyl, C₁₋₈ deuterated alkyl, C₁₋₈ haloalkyl, C₃₋₈ cycloalkyl, 3 to 8 membered heterocyclyl, C₆₋₁₀ aryl and 5 to 10 membered heteroaryl can be each optionally further substituted;
M is an inorganic acid or organic acid, wherein the inorganic acid is selected from the group consisting of hydrochloric acid, sulfuric acid, nitric acid, hydrobromic acid, hydrofluoric acid, hydroiodic acid and phosphoric acid, and the organic acid is selected from the group consisting of 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, capric acid, caproic acid, caprylic acid, cinnamic acid, citric acid, cyclamic acid, camphorsulfonic acid, aspartic acid, camphor acid, gluconic acid, glucuronic acid, glutamic acid, erythorbic acid, lactic acid, malic acid, mandelic acid, pyroglutamic acid, tartaric acid, lauryl sulfuric acid, dibenzoyl tartaric acid, ethane-1,2-disulfonic acid, ethanesulfonic acid, formic acid, fumaric acid, galactonic acid, gentisic acid, glutaric acid, 2-ketoglutaric acid, glycolic acid, hippuric acid, isethionic acid, lactobionic acid, ascorbic acid, asparagic acid, lauric acid, camphor acid, maleic acid, malonic acid, D-tartaric acid, pamoic acid, methanesulfonic acid, 1,5-naphthalenedisulfonic acid, naphthalene-2-sulfonic acid, nicotinic acid, oleic acid, orotic acid, oxalic acid, palmitic acid, embonic acid, propionic acid, salicylic acid, 4-aminosalicylic acid, sebacic acid, stearic acid, succinic acid, thiocyanic acid, undecylenic acid, trifluoroacetic acid, benzenesulfonic acid, *p*-toluenesulfonic acid and L-malic acid; and
x is selected from the group consisting of 0, 1, 2 and 3;
n is an integer from 0 to 6.

Disclosed herein but not defining the scope of the invention is a crystal form of the compound of formula (I) characterized in that,
R₁ and R₂ are each independently selected from the group consisting of hydrogen, cyano, halogen, C₁₋₆ alkyl and C₃₋₆ cycloalkyl, and preferably hydrogen, cyano, halogen, C₁₋₃ alkyl and C₃₋₆ cycloalkyl;
R₃ and R₄ are each independently selected from the group consisting of hydrogen, cyano, halogen, nitro, C₁₋₆ alkyl, C₁₋₆ haloalkyl, C₁₋₆ hydroxyalkyl, cyano-substituted C₁₋₆ alkyl, C₃₋₆ cycloalkyl, cyano-substituted C₃₋₆ cycloalkyl, 5 or 6 membered heteroaryl, - (CH₂)ₙSRₐ, -(CH₂)ₙC(O)Rₐ, -S(O)Rₐ, -S(O)₂Rₐ, -S(O)(=NH)Rₐ, -C(O)ORₐ and - C(O)O(CH₂)ₙNRₐR_{b};
R₅ is selected from the group consisting of C₁₋₆ alkyl, C₁₋₆ haloalkyl, C₁₋₆ hydroxyalkyl, cyano-substituted C₁₋₆ alkyl, C₁₋₆ alkoxy, C₁₋₆ alkylthio, C₃₋₆ cycloalkyl, cyano-substituted C₃₋₆ cycloalkyl, 3 to 6 membered heterocyclyl, C₆₋₁₀ aryl, 5 to 10 membered heteroaryl, -(CH₂)ₙRₐ, -(CH₂)ₙORₐ, -S(O)₂Rₐ and -(CH₂)ₙNRₐR_{b}, wherein the 5 to 10 membered heteroaryl can be optionally further substituted;
R₆ is hydrogen;
or, R₅ and R₆ together with the carbon atoms to which they are attached form a C₃₋₈ cycloalkyl, wherein the C₃₋₈ cycloalkyl can be optionally further substituted;
Rₐ and R_{b} are each independently selected from the group consisting of hydrogen, halogen, cyano, nitro, C₁₋₆ alkyl, 3 to 6 membered heterocyclyl and 5 to 10 membered heteroaryl, wherein the C₁₋₆ alkyl, 3 to 6 membered heterocyclyl and 5 to 10 membered heteroaryl can be each optionally further substituted;
M is selected from the group consisting of hydrochloric acid, sulfuric acid, nitric acid, hydrobromic acid, phosphoric acid, methanesulfonic acid, 1,5-naphthalenedisulfonic acid and *p*-toluenesulfonic acid; and
x is selected from the group consisting of 1 and 2;
n is an integer from 0 to 3.

Disclosed herein but not defining the scope of the invention is a crystal form of the compound of formula (I) characterized in that,
R₁ and R₂ are each independently selected from the group consisting of hydrogen, fluorine, chlorine, cyano, methyl and cyclopropyl;
R₃ and R₄ are each independently selected from the group consisting of hydrogen, halogen, cyano, nitro, C₁₋₃ alkyl, cyano-substituted C₁₋₃ alkyl, halogen-substituted C₁₋₃ alkyl, C₁₋₃ hydroxyalkyl, C₃₋₆ cycloalkyl, cyano-substituted C₃₋₆ cycloalkyl, oxazolyl, - C(O)Rₐ, -SC₁₋₃ alkyl, -S(O)C₁₋₃ alkyl, -S(O)₂C₁₋₃ alkyl, -C(O)OC₁₋₃ alkyl, -S(O)(=NH)C₁₋₃ alkyl and -C(O)O(CH₂)ₙN(CH₃)C₁₋₃ alkyl, and preferably hydrogen, fluorine, chlorine, cyano, nitro, trifluoromethyl, cyclopropyl, cyano-substituted cyclopropyl, cyano-substituted isopropyl, hydroxyisopropyl, oxazolyl, -C(O)Rₐ, -SCH₃, -S(O)CH₃, - S(O)₂CH₃, -C(O)OCH₂CH₃, -S(O)(=NH)CH₃ and -C(O)O(CH₂)₂NCH₃(CH₃);
R₅ is selected from the group consisting of C₁₋₃ alkyl, C₁₋₃ haloalkyl, C₁₋₃ alkoxy, C₁₋₃ hydroxyalkyl, cyano-substituted C₁₋₃ alkyl, C₃₋₆ cycloalkyl, cyano-substituted C₃₋₆ cycloalkyl, 3 to 6 membered nitrogen-containing heterocyclyl, C₆₋₁₀ aryl, 5 to 10 membered heteroaryl, C₁₋₃ alkylthio, -(CH₂)ₙRₐ, -(CH₂)ₙORₐ, -S(O)₂Rₐ and -(CH₂)ₙN(Rₐ)₂, and preferably hydrogen, fluorine, chlorine, cyano, methyl, ethyl, isopropyl, fluoromethyl, trifluoromethyl, methoxy, cyclopropyl, cyclobutyl, methyl-substituted pyrazolyl, phenyl, -SCH₃, -(CH₂)ₙRₐ, -NC(CH₃)₂, -S(O)₂C(CH₃)₂ and -CH₂N(CH₃)₂;
R₆ is hydrogen;
or, R₅ and R₆ together with the carbon atoms to which they are attached form a C₃₋₆ cycloalkyl, and preferably cyclopropyl, wherein the cyclopropyl is optionally substituted by halogen, and preferably by fluorine;
Rₐ is selected from the group consisting of hydrogen, nitro, C₁₋₃ alkyl, azetidinyl and pyrrolidinyl, wherein the azetidinyl and pyrrolidinyl are each optionally substituted by halogen, and preferably by fluorine;
M is selected from the group consisting of hydrochloric acid, sulfuric acid, methanesulfonic acid, p-toluenesulfonic acid, hydrobromic acid, nitric acid and 1,5-naphthalenedisulfonic acid, and preferably methanesulfonic acid;
x is 0, 1 or 2;
n is selected from the group consisting of 0, 1 and 2.

Disclosed herein but not defining the scope of the invention is a crystal form of the compound of formula (I) having a structure as shown in formula (II):

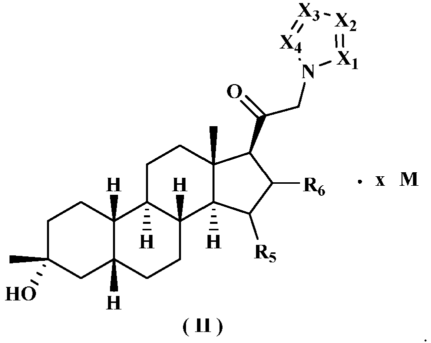

Disclosed herein but not defining the scope of the invention is a crystal form of the compound of formula (I) having a structure as shown in formula (III): 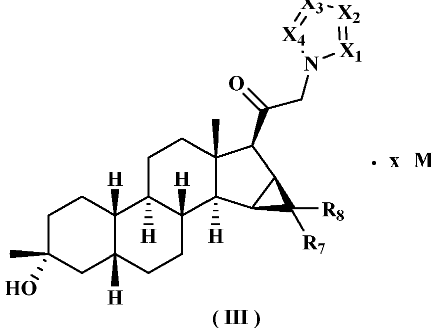 wherein:
R₇ and R₈ are each independently selected from the group consisting of hydrogen, fluorine, chlorine, bromine and methyl, and preferably hydrogen and fluorine.

Disclosed herein but not defining the scope of the invention is a crystal form of the compound of formula (I) having a structure as shown in formula (IV):

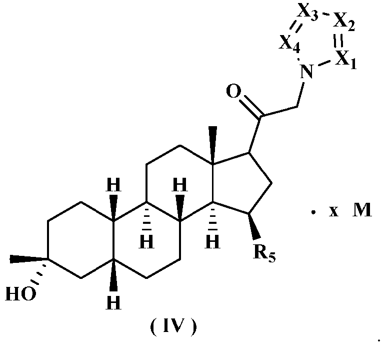

Disclosed herein but not defining the scope of the invention is a crystal form of the compound of formula (I) having a structure as shown in formula (V):

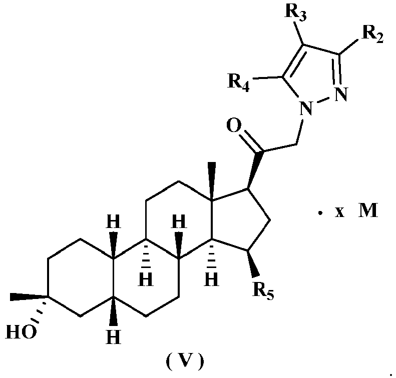

In a preferred embodiment of the present invention, in the crystal form of the compound of formula (Ia), the structure of the compound is as follows:

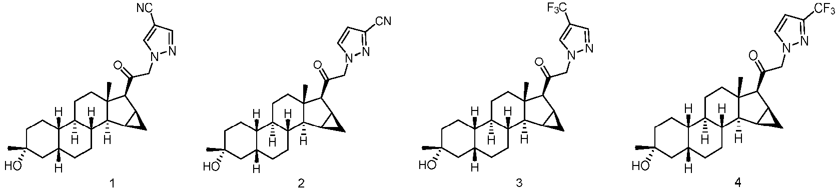

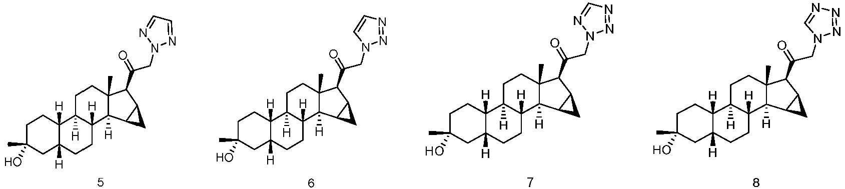

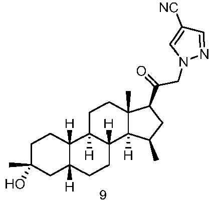

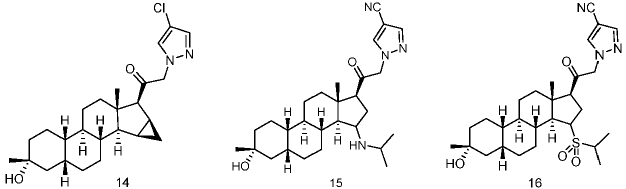

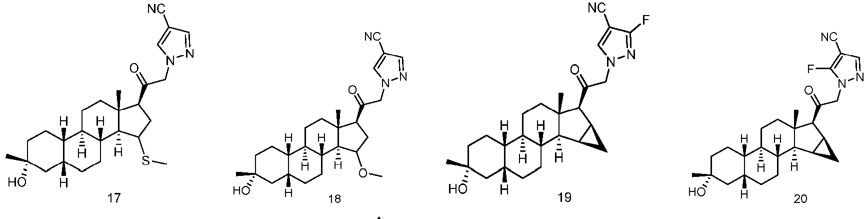

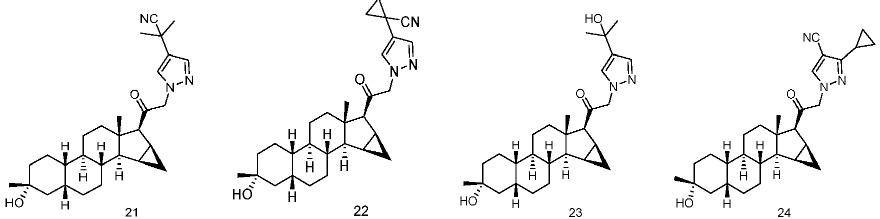

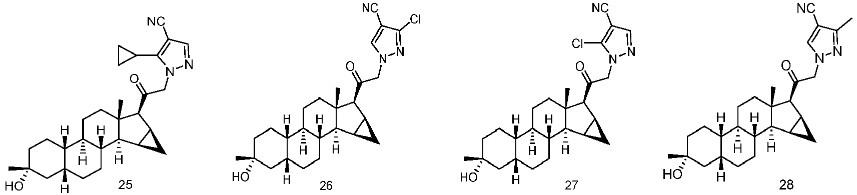

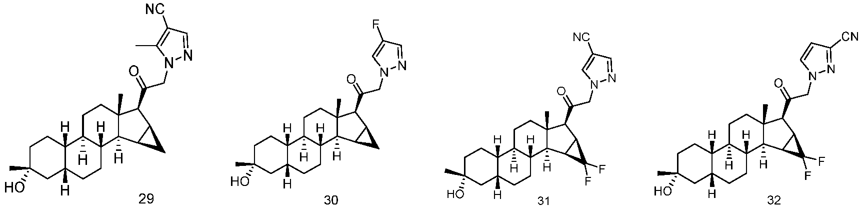

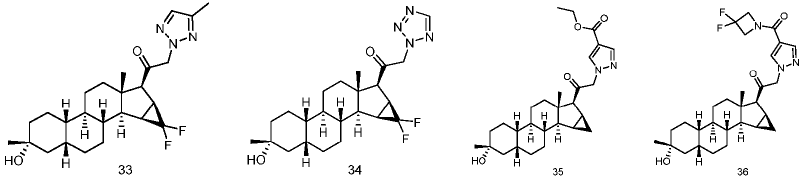

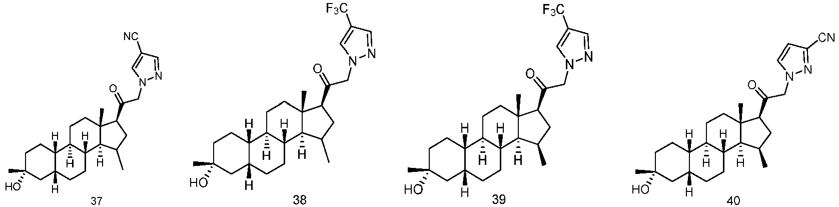

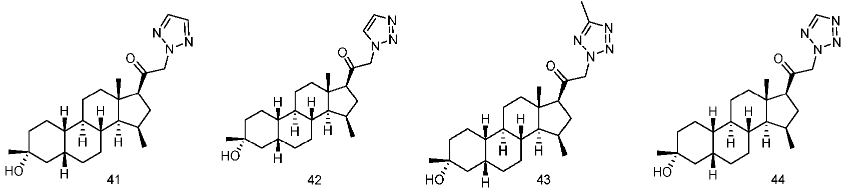

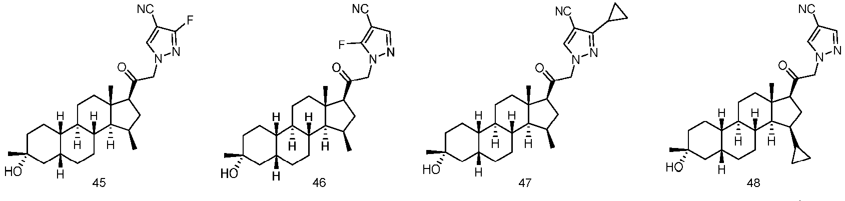

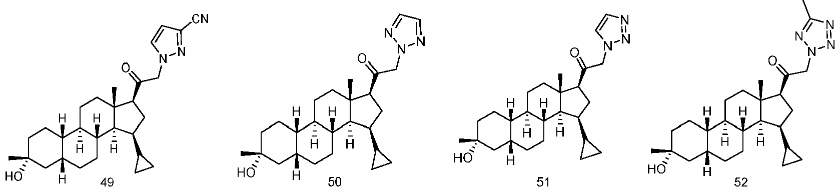

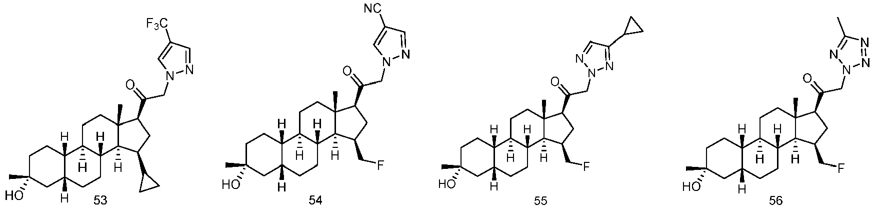

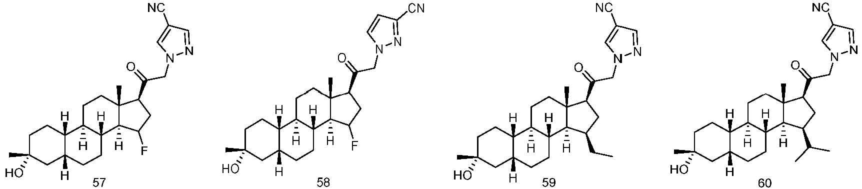

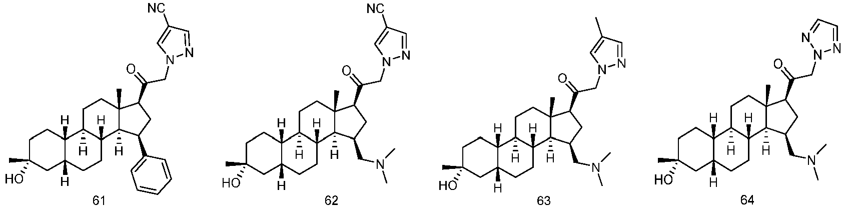

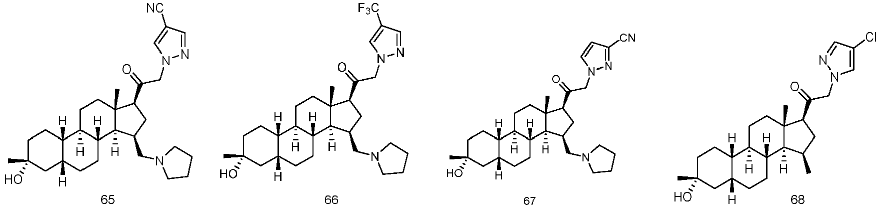

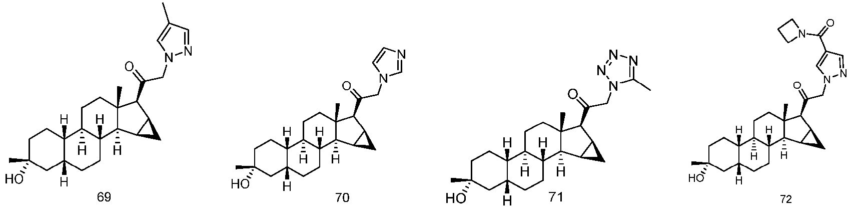

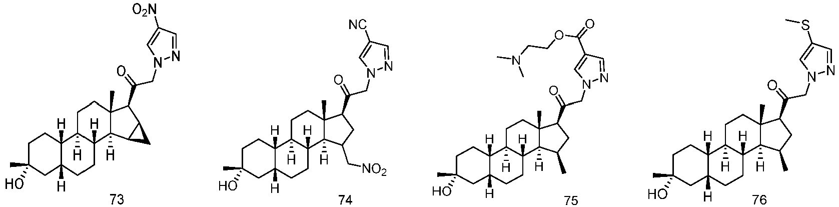

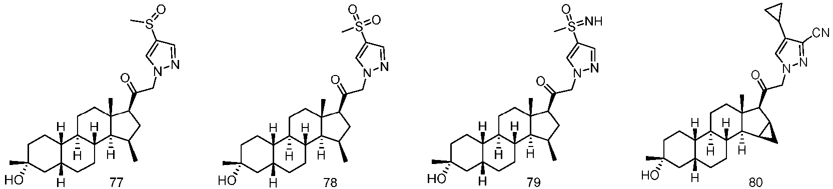

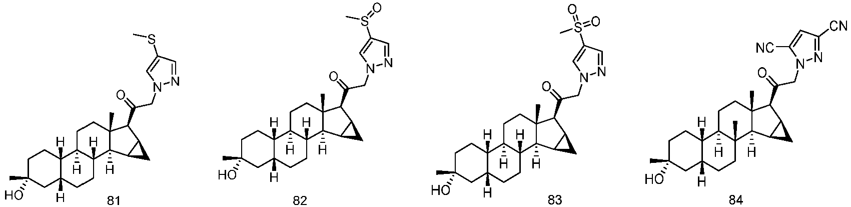

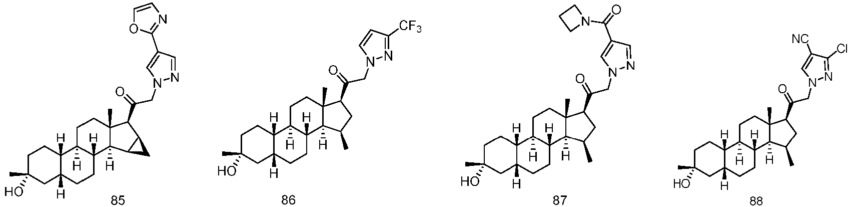

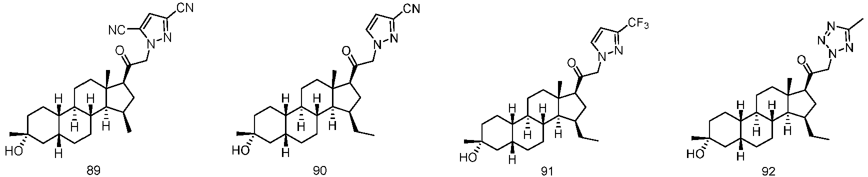

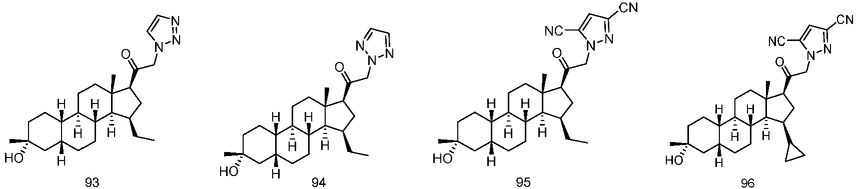

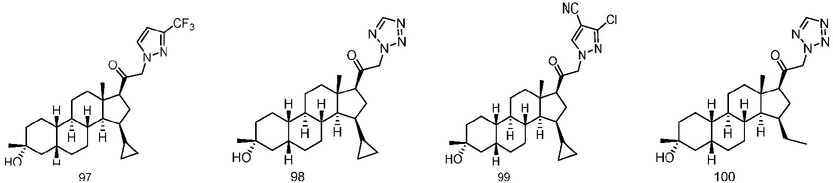

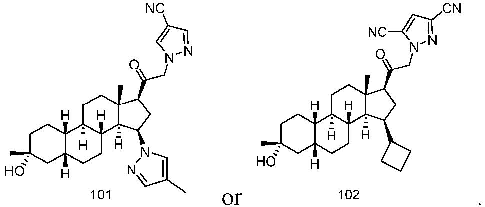

According to the present invention, there is also provided the crystal form having the structure as shown in formula (VI), wherein x and M are as defined in claim 6.

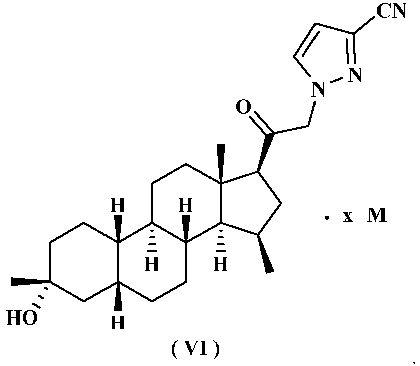

A further preferred embodiment of the present invention is characterized in that x is 0, and the compound of formula (VI) is a crystal form of free base.

A further preferred embodiment of the present invention is characterized in that when x is selected from the group consisting of 1 and 2. the compound of formula (VI) is a crystal form of salt, and preferably x is 1.

A further preferred embodiment of the present invention is a crystal form of free base of the compound of formula (VI), characterized in that the compound of formula (VI) is a hydrate or an anhydrate.

The objective of the present invention is also to provide a method for preparing the the compound of formula (Ia). specifically comprising the following steps of:
1) preparation of stock solution: the free base of the compound of the general formula is dissolved in an organic solvent to obtain a clear stock solution, the concentration of the solution is preferably 50 to 100 mg/mL, and more preferably 100 mg/mL;
2) preparation of counter ion acid solution: a counter ion acid is added to an organic solvent or water to obtain a clear counter ion acid solution; the organic solvent is preferably ethanol, and the concentration is preferably 1.2 to 2.2 mol/L;
3) preparation of salt of the compound: the stock solution is added to the counter ion acid solution to obtain a clear salt solution, the salt solution is stirred overnight to precipitate a solid, and then filtered, and the filter cake is dried in vacuum to obtain the salt of the compound of formula (I); the vacuum temperature is preferably 40°C, and the amount of counter ion acid is preferably 0.6 to 1.2 equivalents;
   wherein:
   the organic solvent is selected from the group consisting of 88% acetone, methanol, ethanol, ethyl acetate, dichloromethane, acetone, toluene, acetonitrile, tetrahydrofuran, heptane, methyl *tert*-butyl ether, isopropyl ether and N,N-dimethylformamide; and preferably ethyl acetate and ethanol;
   the counter ion acid is selected from the group consisting of hydrochloric acid, sulfuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, capric acid, caproic acid, caprylic acid, cinnamic acid, citric acid, cyclamic acid, camphorsulfonic acid, aspartic acid, camphor acid, gluconic acid, glucuronic acid, glutamic acid, erythorbic acid, lactic acid, malic acid, mandelic acid, pyroglutamic acid, D-tartaric acid, pamoic acid, lauryl sulfuric acid, dibenzoyl tartaric acid, ethane-1,2-disulfonic acid, ethanesulfonic acid, formic acid, fumaric acid, galactonic acid, gentisic acid, glutaric acid, 2-ketoglutaric acid, glycolic acid, hippuric acid, isethionic acid, lactobionic acid, ascorbic acid, asparagic acid, lauric acid, camphor acid, maleic acid, malonic acid, methanesulfonic acid, 1,5-naphthalenedisulfonic acid, naphthalene-2-sulfonic acid, nicotinic acid, oleic acid, orotic acid, oxalic acid, palmitic acid, embonic acid, propionic acid, salicylic acid, 4-aminosalicylic acid, sebacic acid, stearic acid, succinic acid, thiocyanic acid, undecylenic acid, trifluoroacetic acid, benzenesulfonic acid, *p*-toluenesulfonic acid and L-malic acid.

The objective of the present invention is also to provide a method for preparing the the compound of formula (Ia), specifically comprising the following steps of:
1) weighing an appropriate amount of the free base or the acid addition salt obtained in claim 20, followed by adding a good solvent and heating the mixture until dissolved;
2) after stirring for several hours, adding dropwise a poor solvent until turbidity appears;
3) stirring and cooling the mixture, followed by precipitating a crystal to obtain the target product;
   wherein:
   the good solvent is selected from the group consisting of 88% acetone, ethyl acetate, methanol, ethanol, dichloromethane, acetone and tetrahydrofuran; and preferably ethyl acetate;
   the organic solvent is selected from the group consisting of 88% acetone, methanol, ethanol, ethyl acetate, dichloromethane, acetone, toluene, acetonitrile, tetrahydrofuran, heptane, methyl *tert*-butyl ether, isopropyl ether and N,N-dimethylformamide; and preferably tetrahydrofuran and ethanol; the good solvent and organic solution are miscible when used;
   the poor solvent is selected from the group consisting of *n*-heptane, methyl *tert-butyl* ether and isopropyl ether; and preferably *n*-heptane and methyl *tert-butyl* ether; the poor solvent, good solvent and organic solution are miscible when used; in a preferred embodiment of the present invention, the crystal form of the compound of formula (VI) is crystal form I of the free base (crystal form I of the free base of Example 40), wherein x is 0, the crystal form is crystal form I of the free base, the X-ray powder diffraction pattern thereof has a diffraction peak at 20 (±0.2°) of 16.7, or a diffraction peak at 2θ (±0.2°) of 12.6, or a diffraction peak at 2θ (±0.2°) of 17.4, or a diffraction peak at 2θ (±0.2°) of 7.3, or a diffraction peak at 2θ (±0.2°) of 20.2, or a diffraction peak at 2θ (±0.2°) of 20.6, or a diffraction peak at 2θ (±0.2°) of 11.9, or a diffraction peak at 2θ (±0.2°) of 11.1, or a diffraction peak at 2θ (±0.2°) of 23.9, or a diffraction peak at 2θ (±0.2°) of 21.9, or a diffraction peak at 2θ (±0.2°) of 38.4; preferably comprises any 3 to 11, or 5 to 8, or 6 to 8 of the above diffraction peaks, and more preferably comprises any 3, 6, 8, 10 or 11 of the above diffraction peaks.

In a preferred embodiment of the present invention, the X-ray powder diffraction pattern of crystal form I of the free base of Example 40 has diffraction peaks at 2θ (±0.2°) of 16.7, 12.6 and 17.4, and optionally further comprises one or more diffraction peaks at 2θ (±0.2°) of 7.3, 20.2, 20.6, 11.9, 11.1, 23.9, 21.9 and 38.4.

Preferably, the X-ray powder diffraction pattern of the crystal form has diffraction peaks at 2θ (±0.2°) of 16.7, 12.6, 17.4, 7.3, 20.2 and 20.6.

Further preferably, the X-ray powder diffraction pattern of the crystal form has diffraction peaks at 2θ (±0.2°) of 16.7, 12.6, 17.4, 7.3, 20.2, 20.6, 11.9 and 11.1.

Still further preferably, the X-ray powder diffraction pattern of the crystal form has diffraction peaks at 2θ (±0.2°) of 16.7, 12.6, 17.4, 7.3, 20.2, 20.6, 11.9, 11.1, 23.9 and 21.9.

More further preferably, the X-ray powder diffraction pattern of the crystal form has diffraction peaks at 2θ (±0.2°) of 16.7, 12.6, 17.4, 7.3, 20.2, 20.6, 11.9, 11.1, 23.9, 21.9 and 38.4.

In a preferred embodiment of the present invention, the crystal form of the compound of formula (VI) is crystal form I of the free base *(i.e.,* crystal form I of the free base of Example 40), the X-ray powder diffraction pattern has diffraction peaks at 2θ (±0.2°) of 16.7, 12.6 and 14.6, preferably has diffraction peaks at 2θ (±0.2°) of 7.3, 13.2, 17.4, 19.4, 20.2 and 20.6, and more preferably has diffraction peaks at 2θ (±0.2°) of 9.2, 11.1, 11.9, 19.6, 22.3 and 25.5.

Using Cu-Kα radiation, the characteristic X-ray diffraction peaks represented by 2θ angle and interplanar spacing d value are shown in Table 1.

**Table 1**

| No. | XRPD diffraction data of crystal form I of the free base of Example 40 | | | | | |
|---|---|---|---|---|---|---|
| | 2θ (±0.2°) | d value | Peak height | Proportion (I%) | Area | Proportion (I%) |
| 1 | 7.288 | 12.1189 | 2551 | 37.8 | 22414 | 34.8 |
| 2 | 9.150 | 9.6575 | 870 | 12.9 | 7007 | 10.9 |
| 3 | 11.116 | 7.9532 | 903 | 13.4 | 8073 | 12.5 |
| 4 | 11.908 | 7.4258 | 1388 | 20.6 | 11394 | 17.7 |
| 5 | 12.596 | 7.0219 | 5007 | 74.1 | 47359 | 73.6 |
| 6 | 13.162 | 6.7210 | 1828 | 27.1 | 17604 | 27.4 |
| 7 | 14.600 | 6.0620 | 3880 | 57.5 | 48117 | 74.8 |
| 8 | 16.711 | 5.3008 | 6753 | 100.0 | 64337 | 100.0 |
| 9 | 17.399 | 5.0927 | 4992 | 73.9 | 44437 | 69.1 |
| 10 | 19.364 | 4.5801 | 2485 | 36.8 | 28292 | 44.0 |
| 11 | 19.606 | 4.5242 | 265 | 3.9 | 12347 | 19.2 |
| 12 | 20.220 | 4.3880 | 2717 | 40.2 | 23559 | 36.6 |
| 13 | 20.584 | 4.3114 | 2486 | 36.8 | 24247 | 37.7 |
| 14 | 21.923 | 4.0509 | 493 | 7.3 | 6503 | 10.1 |
| 15 | 22.266 | 3.9893 | 1498 | 22.2 | 17506 | 27.2 |
| 16 | 23.893 | 3.7212 | 429 | 6.4 | 4438 | 6.9 |
| 17 | 24.253 | 3.6667 | 558 | 8.3 | 5180 | 8.1 |
| 18 | 25.549 | 3.4836 | 880 | 13.0 | 14209 | 22.1 |
| 19 | 26.400 | 3.3733 | 501 | 7.4 | 5772 | 9.0 |
| 20 | 27.738 | 3.2135 | 712 | 10.5 | 6813 | 10.6 |
| 21 | 28.657 | 3.1125 | 382 | 5.7 | 4540 | 7.1 |
| 22 | 29.362 | 3.0393 | 699 | 10.4 | 6699 | 10.4 |
| 23 | 30.354 | 2.9422 | 179 | 2.7 | 3524 | 5.5 |
| 24 | 35.099 | 2.5546 | 303 | 4.5 | 3607 | 5.6 |
| 25 | 38.421 | 2.3410 | 506 | 7.5 | 6863 | 10.7 |
| 26 | 38.970 | 2.3093 | 246 | 3.6 | 3560 | 5.5 |

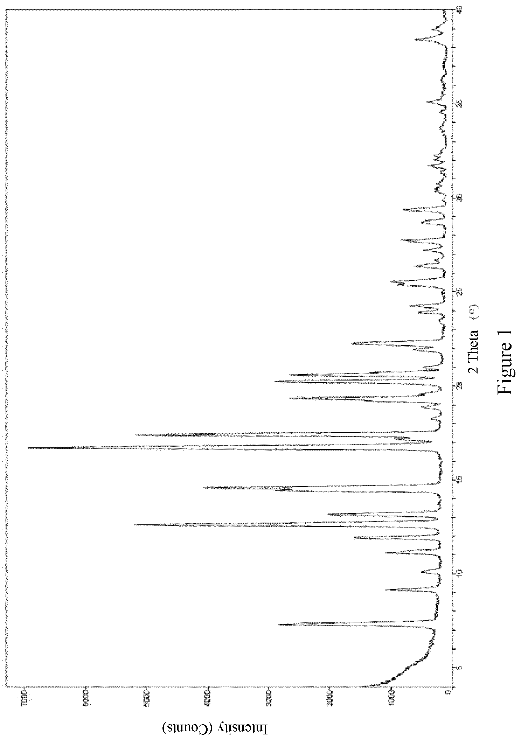

The crystal form of the compound of formula (VI) of the present invention is preferably crystal form I of the free base compound *(i.e.,* crystal form I of the free base of Example 40), the X-ray powder diffraction pattern thereof is substantially as shown in Figure 1.

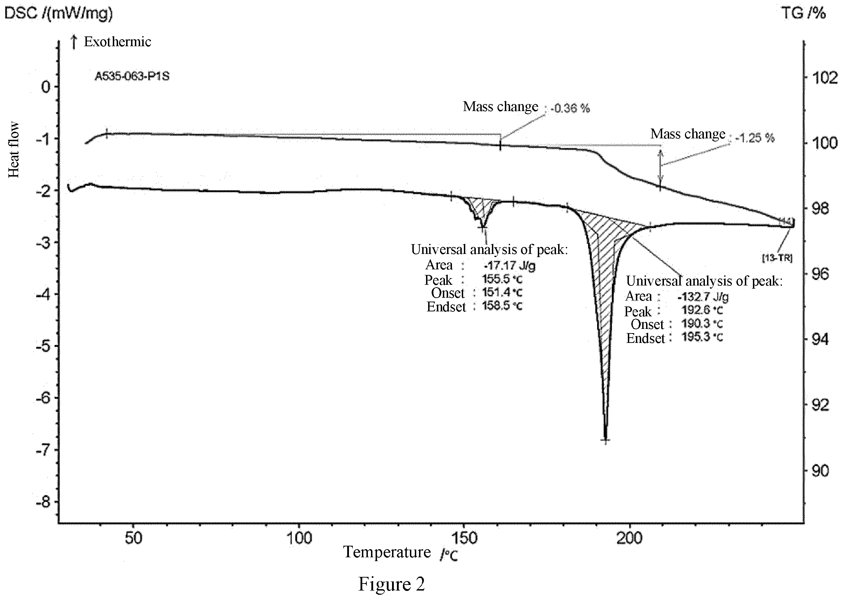

The crystal form of the compound of formula (VI) of the present invention is preferably crystal form I of the free base compound *(i.e.,* crystal form I of the free base of Example 40), the DSC spectrum thereof has an endothermic peak at 151.4±0.5°C, and the TGA spectrum shows a weight loss of 0.36% at 40 to 150°C, indicating that there is almost no residual solvent. The DSC spectrum thereof shows no thermal effect before 120°C, and the TGA spectrum shows no weight loss before 120°C, indicating that there is no crystal water in crystal form I of the free base. Specifically, the TGA-DSC spectrum thereof is substantially as shown in Figure 2.

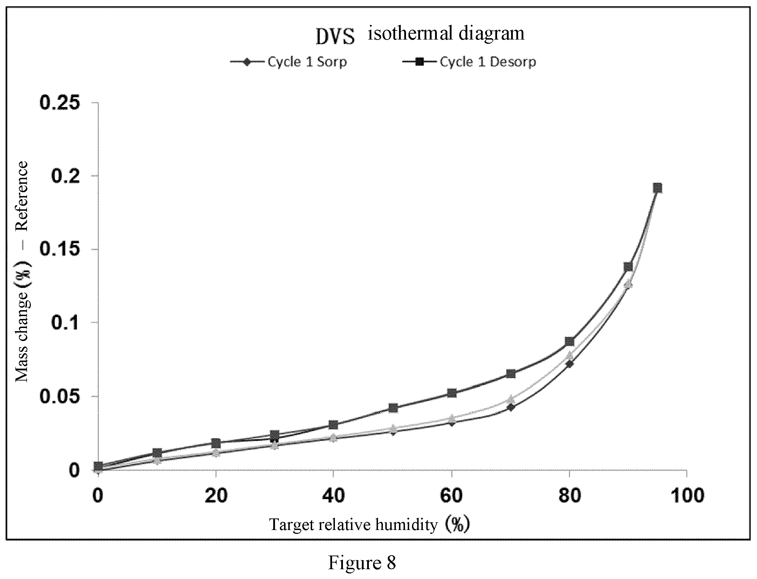

The crystal form of the compound of formula (VI) of the present invention is preferably crystal form I of the free base compound *(i.e.,* crystal form I of the free base of Example 40), the DVS spectrum thereof is substantially as shown in Figure 8.

In a preferred embodiment of the present invention, the acid addition salt of the compound of formula (VI), and a hydrate form, pharmaceutically acceptable salt and crystal form thereof, wherein x is 0, is crystal form II of the free base (*i.e*., crystal form II of the free base of Example 40), the X-ray powder diffraction pattern has diffraction peaks at 2θ (±0.2°) of 11.7, 13.4, 13.6, 16.6 and 18.9, further has diffraction peaks at 2θ (±0.2°) of 9.5, 10.1, 14.7, 19.3, and more further has diffraction peaks at 2θ (±0.2°) of 19.6, 20.6, 20.9, 21.6, 22.1, 22.5, 22.7 and 24.4.

The crystal form of the compound of formula (VI) of the present invention is preferably crystal form II of the free base *(i.e.,* crystal form II of the free base of Example 40), using Cu-Kα radiation, the characteristic X-ray diffraction peaks represented by 2θ angle and interplanar spacing d value are shown in Table 2.

**Table 2**

| No. | XRPD diffraction data of crystal form II of the free base of Example 40 | | | | | |
|---|---|---|---|---|---|---|
| | 2θ (±0.2°) | d value | Peak height | Proportion (1%) | Area | Proportion (1%) |
| 1 | 6.859 | 12.8757 | 247 | 3.2 | 1404 | 2.8 |
| 2 | 9.553 | 9.2509 | 549 | 7.0 | 3649 | 7.2 |
| 3 | 10.064 | 8.7818 | 919 | 11.7 | 6282 | 12.4 |
| 4 | 11.686 | 7.5667 | 7662 | 97.7 | 49042 | 96.9 |
| 5 | 13.447 | 6.5792 | 3825 | 48.8 | 36976 | 73.1 |
| 6 | 13.611 | 6.5004 | 4165 | 53.1 | 50588 | 100.0 |
| 7 | 14.687 | 6.0266 | 1032 | 13.2 | 6283 | 12.4 |
| 8 | 16.587 | 5.3401 | 7055 | 90.0 | 45926 | 90.8 |
| 9 | 18.135 | 4.8877 | 337 | 4.3 | 2153 | 4.3 |
| 10 | 18.904 | 4.6905 | 7841 | 100.0 | 50136 | 99.1 |
| 11 | 19.307 | 4.5936 | 2178 | 27.8 | 21273 | 42.1 |
| 12 | 19.593 | 4.5272 | 998 | 12.7 | 6508 | 12.9 |
| 13 | 20.601 | 4.3079 | 1793 | 22.9 | 12880 | 25.5 |
| 14 | 20.966 | 4.2336 | 1482 | 18.9 | 10103 | 20.0 |
| 15 | 21.555 | 4.1193 | 1425 | 18.2 | 9159 | 18.1 |
| 16 | 22.064 | 4.0254 | 1165 | 14.9 | 7826 | 15.5 |
| 17 | 22.468 | 3.9538 | 1088 | 13.9 | 8815 | 17.4 |
| 18 | 22.676 | 3.9181 | 1160 | 14.8 | 9277 | 18.3 |
| 19 | 23.402 | 3.7981 | 559 | 7.1 | 4389 | 8.7 |
| 20 | 24.437 | 3.6396 | 1187 | 15.1 | 9394 | 18.6 |
| 21 | 25.635 | 3.4721 | 582 | 7.4 | 5209 | 10.3 |

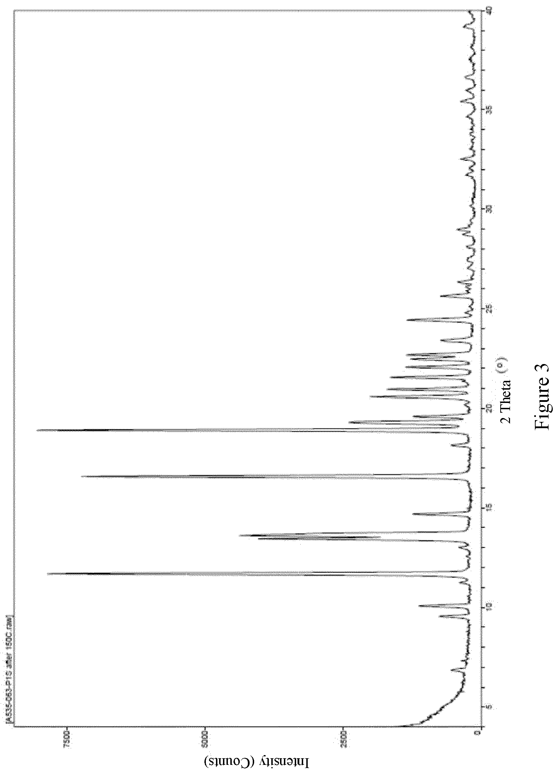

The crystal form of the compound of formula (VI) of the present invention is preferably crystal form II of the free base *(i.e.,* crystal form II of the free base of Example 40), the X-ray powder diffraction pattern thereof is substantially as shown in Figure 3.

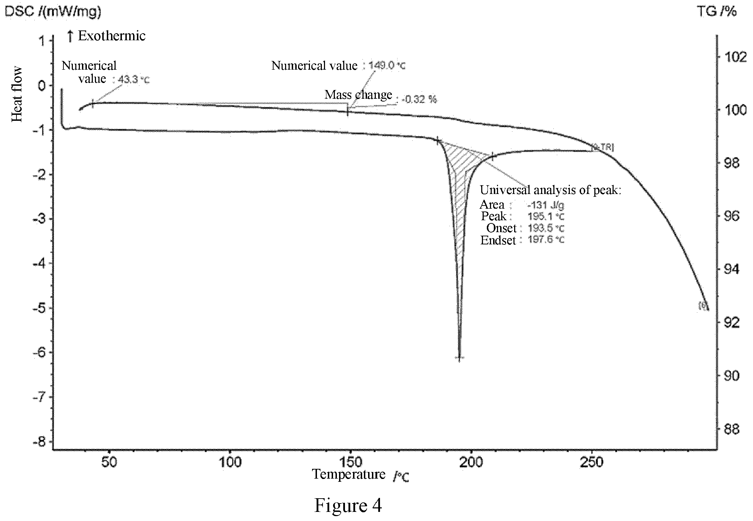

The crystal form of the compound of formula (VI) of the present invention is preferably crystal form II of the free base *(i.e.,* crystal form II of the free base of Example 40), the DSC spectrum thereof has an endothermic peak at 193.5±0.5°C, and the TGA spectrum shows a weight loss of 0.32% at 40 to 150°C, indicating that there is almost no residual solvent. The DSC spectrum thereof shows no thermal effect before 120°C, and the TGA spectrum shows no weight loss before 120°C, indicating that there is no crystal water in crystal form II of the free base. Specifically, the TGA-DSC spectrum thereof is substantially as shown in Figure 4.

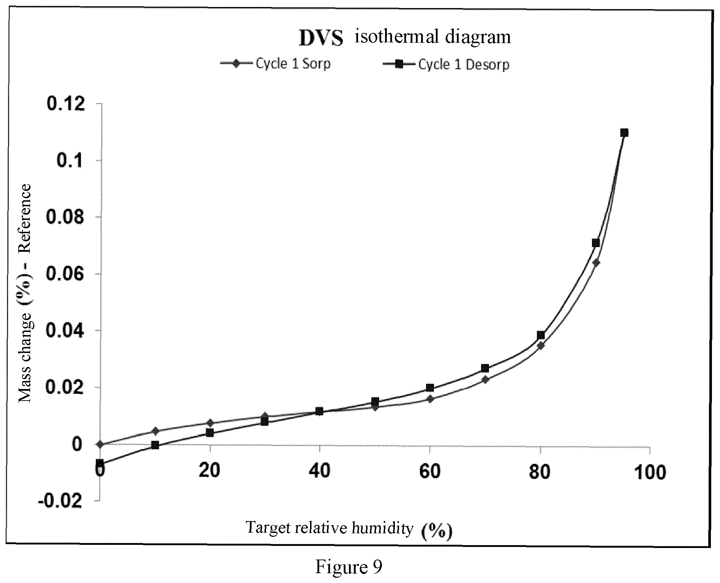

The crystal form of the compound of formula (VI) of the present invention is preferably crystal form II of the free base *(i.e.,* crystal form II of the free base of Example 40), the DVS spectrum thereof is substantially as shown in Figure 9.

In a preferred embodiment of the present invention, the acid addition salt of the compound of formula (VI), and a hydrate form, stereoisomer, pharmaceutically acceptable salt and crystal form thereof, wherein x is 0, is crystal form III of the free base (*i.e*., crystal form III of the free base of Example 40), the X-ray powder diffraction pattern has diffraction peaks at 2θ (±0.2°) of 10.0, 11.7, 13.7, 16.6, 18.9 and 19.2, and further has diffraction peaks at 2θ (±0.2°) of 13.4, 19.6, 20.6, 20.9, 22.0, 22.7, 23.4 and 25.6.

The crystal form of the compound of formula (VI) of the present invention is preferably crystal form III of the free base *(i.e.,* crystal form III of the free base of Example 40), using Cu-Kα radiation, the characteristic X-ray diffraction peaks represented by 2θ angle and interplanar spacing d value are shown in Table 3.

**Table 3**

| No. | XRPD diffraction data of crystal form III of the free base of Example 40 | | | | | |
|---|---|---|---|---|---|---|
| | 2θ (±0.2°) | d value | Peak height | Proportion (I%) | Area | Proportion (I%) |
| 1 | 10.044 | 8.7990 | 2662 | 20.1 | 15303 | 20.3 |
| 2 | 11.663 | 7.5810 | 13229 | 100.0 | 75496 | 100.0 |
| 3 | 13.425 | 6.5899 | 634 | 4.8 | 4077 | 5.4 |
| 4 | 13.686 | 6.4649 | 1660 | 12.5 | 10588 | 14.0 |
| 5 | 16.553 | 5.3512 | 1365 | 10.3 | 9233 | 12.2 |
| 6 | 18.898 | 4.6921 | 1677 | 12.7 | 11100 | 14.7 |
| 7 | 19.242 | 4.6089 | 5126 | 38.7 | 34095 | 45.2 |
| 8 | 19.570 | 4.5324 | 735 | 5.6 | 5086 | 6.7 |
| 9 | 20.563 | 4.3156 | 781 | 5.9 | 5059 | 6.7 |
| 10 | 20.930 | 4.2408 | 922 | 7.0 | 5977 | 7.9 |
| 11 | 22.042 | 4.0294 | 740 | 5.6 | 4722 | 6.3 |
| 12 | 22.668 | 3.9194 | 524 | 4.0 | 3890 | 5.2 |
| 13 | 23.382 | 3.8013 | 579 | 4.4 | 4110 | 5.4 |
| 14 | 25.611 | 3.4754 | 524 | 4.0 | 3860 | 5.1 |

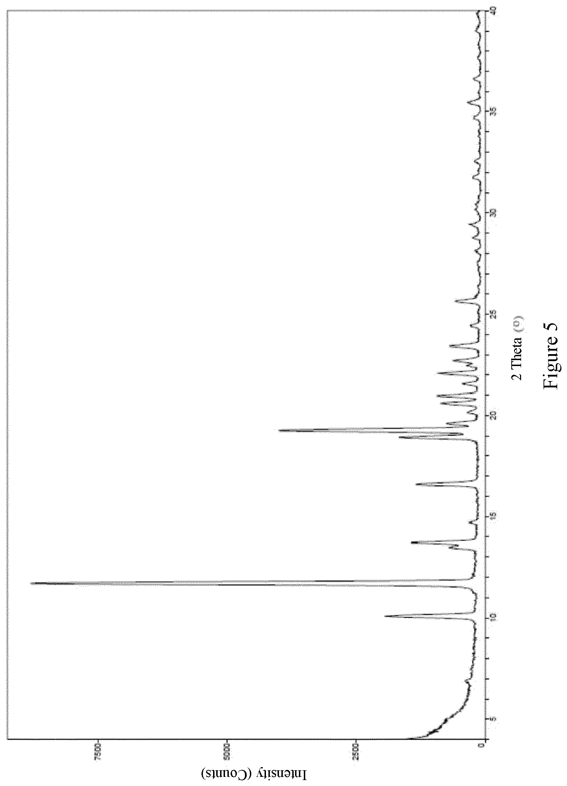

The crystal form of the compound of formula (VI) of the present invention is preferably crystal form III of the free base *(i.e.,* crystal form III of the free base of Example 40), the X-ray powder diffraction pattern thereof is substantially as shown in Figure 5.

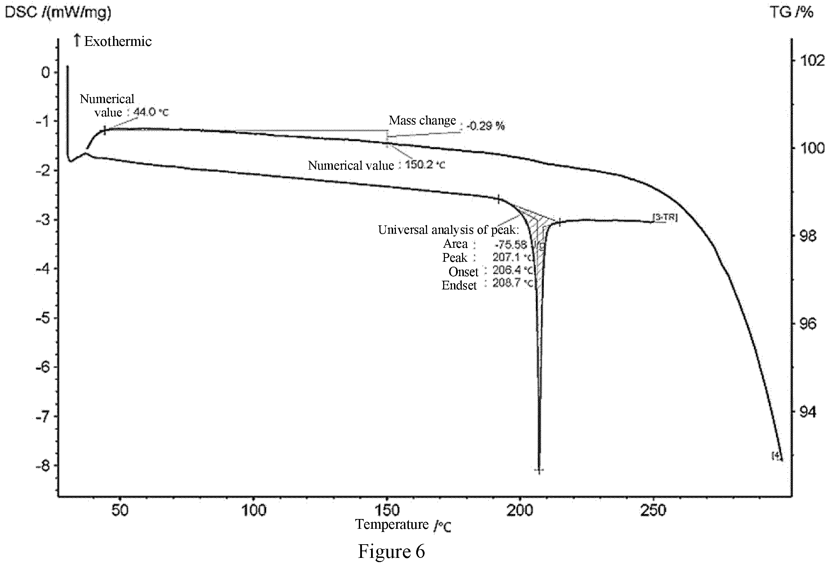

The crystal form of the compound of formula (VI) of the present invention is preferably crystal form III of the free base *(i.e.,* crystal form III of the free base of Example 40), the DSC spectrum thereof has an endothermic peak at 206.4±0.5°C, and the TGA spectrum shows a weight loss of 0.29% at 40 to 150°C, indicating that there is almost no residual solvent. The DSC spectrum thereof shows no thermal effect before 120°C, and the TGA spectrum shows no weight loss before 120°C, indicating that there is no crystal water in crystal form III of the free base. The TGA-DSC spectrum thereof is substantially as shown in Figure 6.

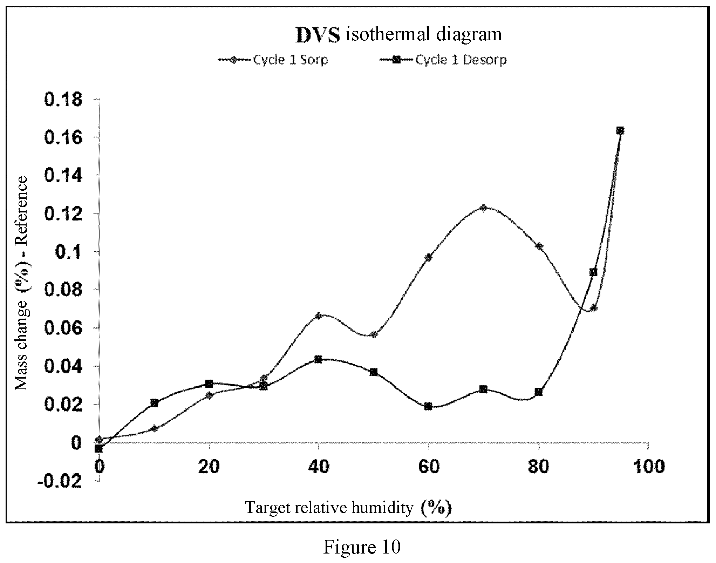

The crystal form of the compound of formula (VI) of the present invention is preferably crystal form III of the free base *(i.e.,* crystal form III of the free base of Example 40), the DVS spectrum thereof is substantially as shown in Figure 10.

In a preferred embodiment of the present invention, the acid addition salt of the compound of formula (VI), and a hydrate form, stereoisomer, pharmaceutically acceptable salt and crystal form thereof, wherein M is methanesulfonic acid, x is 1, is a crystal form of mesylate *(i.e.,* crystal form of the mesylate of Example 40), the X-ray powder diffraction pattern has diffraction peaks at 2θ (±0.2°) of 12.5, 13.5, 19.4 and 19.9, further has diffraction peaks at 2θ (±0.2°) of 15.1, 15.8, 16.5, 17.3, 18.7 and 23.1, and more further has diffraction peaks at 2θ (±0.2°) of 11.1, 11.5, 13.9, 18.5, 21.3, 21.7, 26.5 and 28.9.

The crystal form of the compound of formula (VI) of the present invention is preferably mesylate *(i.e.,* the mesylate of Example 40), using Cu-Kα radiation, the characteristic X-ray diffraction peaks represented by 2θ angle and interplanar spacing d value are shown in Table 4.

**Table 4**

| No. | XRPD diffraction data of the mesylate of formula (VI) (*i.e.,* the mesylate of Example 40) | | | | | |
|---|---|---|---|---|---|---|
| | 2θ (±0.2°) | d value | Peak height | Proportion (I%) | Area | Proportion (I%) |
| 1 | 11.095 | 7.9682 | 80 | 11.4 | 766 | 10.0 |
| 2 | 11.513 | 7.6794 | 71 | 10.1 | 802 | 10.5 |
| 3 | 12.453 | 7.1021 | 404 | 57.5 | 2870 | 37.5 |
| 4 | 13.469 | 6.5687 | 232 | 33.0 | 3530 | 46.1 |
| 5 | 13.936 | 6.3493 | 111 | 15.8 | 1324 | 17.3 |
| 6 | 15.088 | 5.8672 | 257 | 36.6 | 2126 | 27.8 |
| 7 | 15.839 | 5.5907 | 189 | 26.9 | 1459 | 19.0 |
| 8 | 16.477 | 5.3756 | 139 | 19.8 | 1696 | 22.1 |
| 9 | 17.260 | 5.1335 | 360 | 51.3 | 2964 | 38.7 |
| 10 | 18.470 | 4.7997 | 157 | 22.4 | 1178 | 15.4 |
| 11 | 18.720 | 4.7363 | 154 | 21.9 | 1529 | 20.0 |
| 12 | 19.387 | 4.5748 | 702 | 100.0 | 7661 | 100.0 |
| 13 | 19.892 | 4.4596 | 245 | 34.9 | 3840 | 50.1 |
| 14 | 20.904 | 4.2461 | 70 | 10.0 | 268 | 3.5 |
| 15 | 21.252 | 4.1773 | 79 | 11.3 | 1703 | 22.2 |
| 16 | 21.653 | 4.1008 | 67 | 9.5 | 1915 | 25.0 |
| 17 | 21.825 | 4.0690 | 65 | 9.3 | 1119 | 14.6 |
| 18 | 22.211 | 3.9991 | 93 | 13.2 | 506 | 6.6 |
| 19 | 23.136 | 3.8412 | 202 | 28.8 | 3124 | 40.8 |
| 20 | 23.463 | 3.7883 | 84 | 12.0 | 651 | 8.5 |
| 21 | 24.006 | 3.7039 | 121 | 17.2 | 1078 | 14.1 |
| 22 | 25.142 | 3.5391 | 82 | 11.7 | 825 | 10.8 |
| 23 | 26.479 | 3.3634 | 116 | 16.5 | 1526 | 19.9 |
| 24 | 28.939 | 3.0828 | 93 | 13.2 | 1964 | 25.6 |

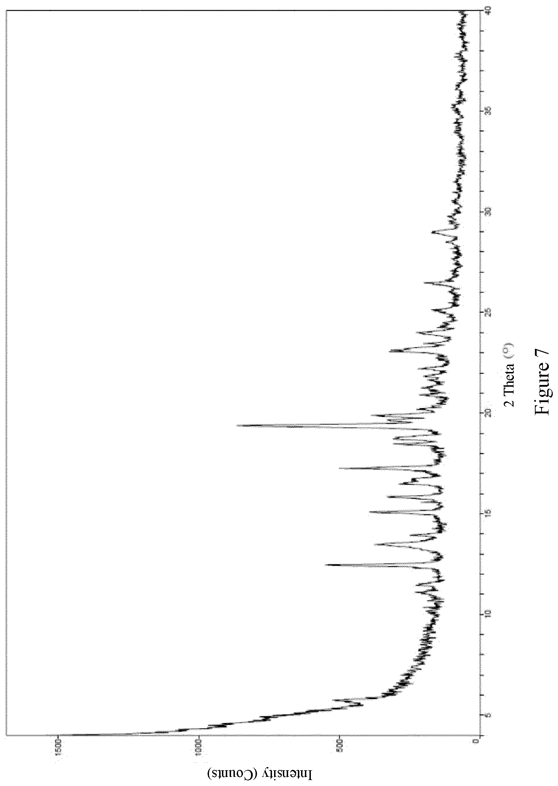

The crystal form of the compound of formula (VI) of the present invention is preferably mesylate *(i.e.,* the mesylate of Example 40), the X-ray powder diffraction pattern thereof is substantially as shown in Figure 7.

The objective of the present invention is also to provide a pharmaceutical composition comprising a therapeutically effective amount of the acid addition salt of formula (Ia), and one or more pharmaceutically acceptable carriers.

The objective of the present invention is also to provide a use of the compound of formula (Ia) or the pharmaceutical composition comprsing the same in the preparation of a GABA_{A} receptor regulator medicament.

The objective of the present invention is also to provide a pharmaceutical composition comprising a therapeutically effective amount of the crystal form of the compound of formula (VI), and one or more pharmaceutically acceptable carriers.

The objective of the present invention is also to provide a pharmaceutical composition comprising the compound formula (Ia) or (VI).

In a preferred embodiment of the present invention, the compound of formula (Ia) is selected from the group consisting of:

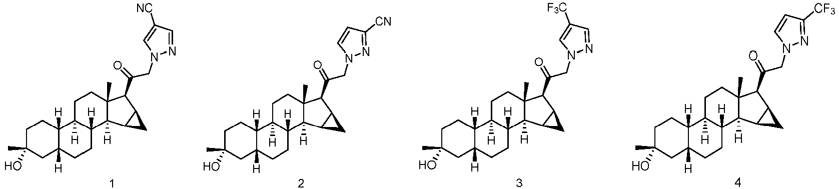

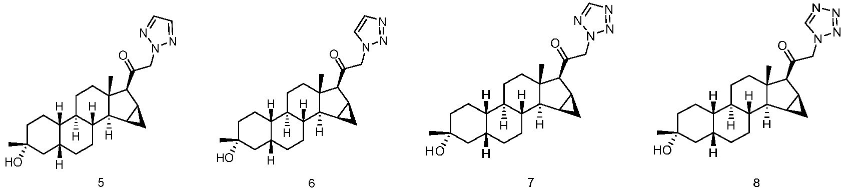

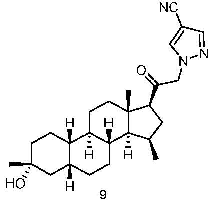

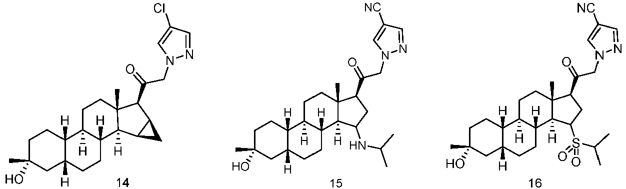

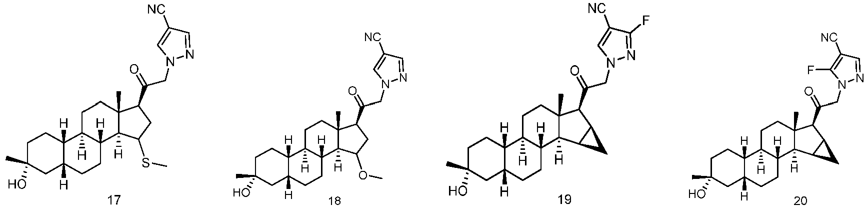

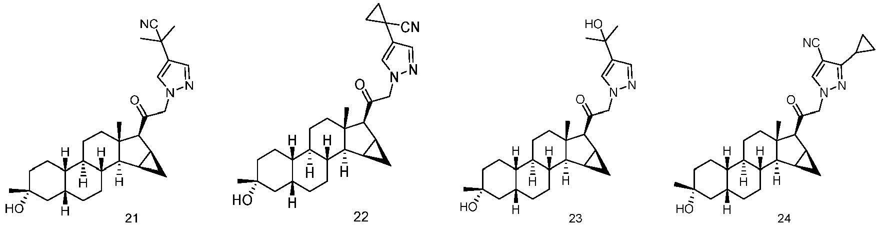

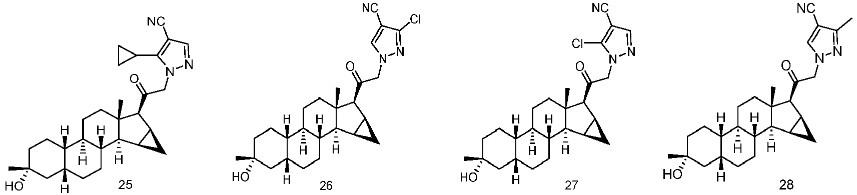

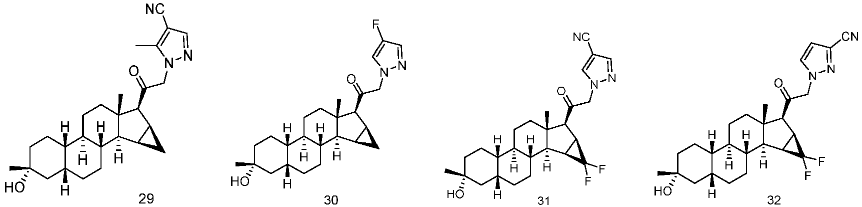

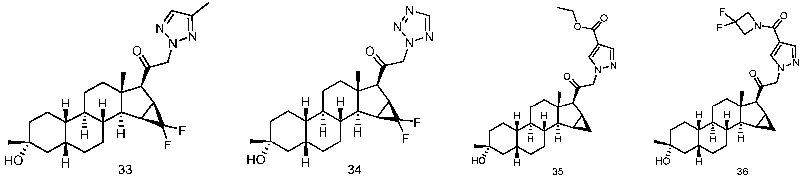

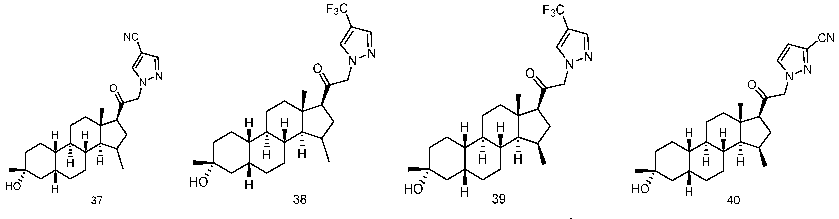

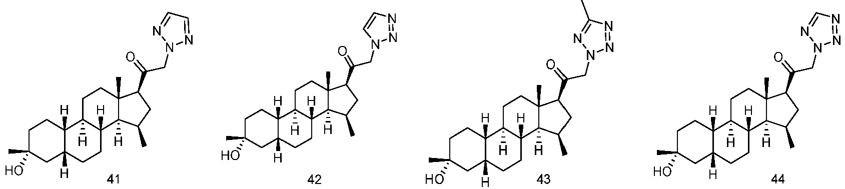

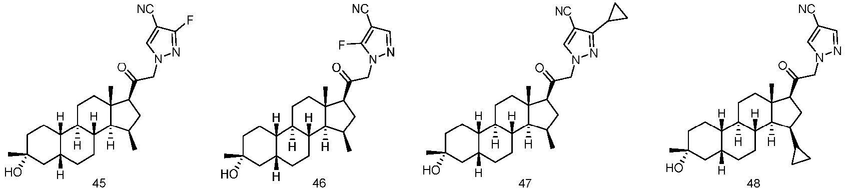

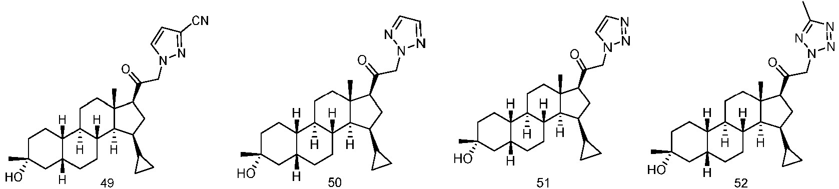

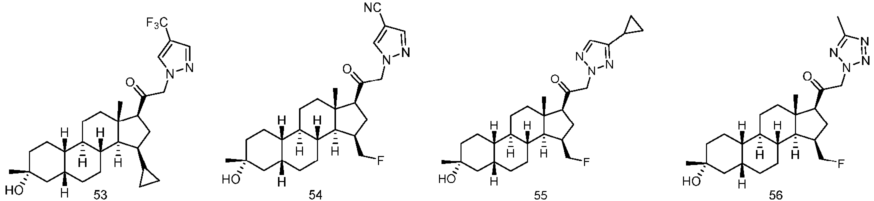

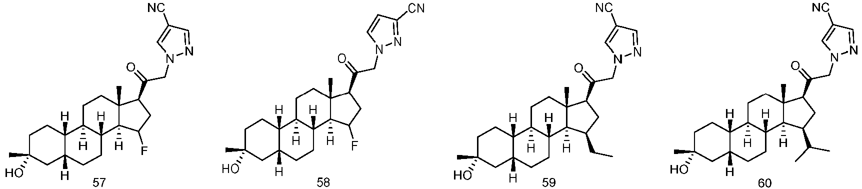

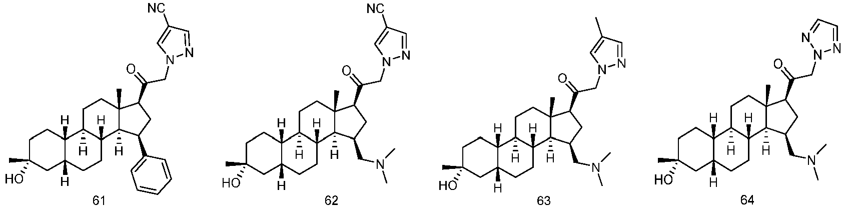

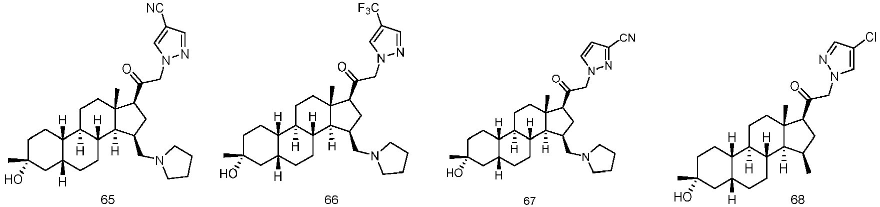

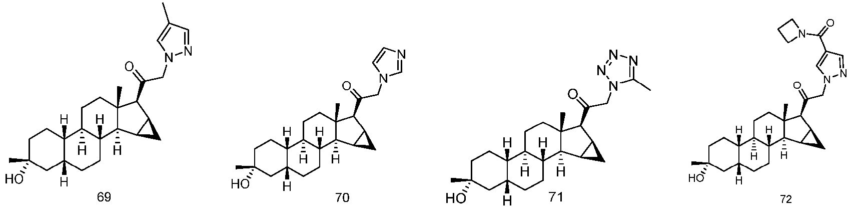

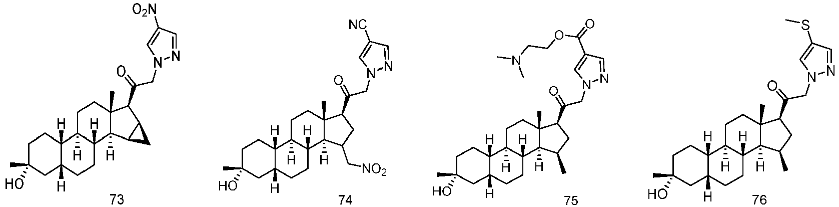

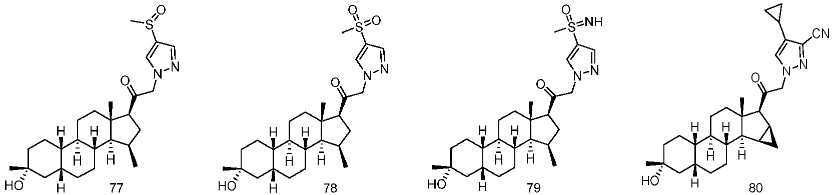

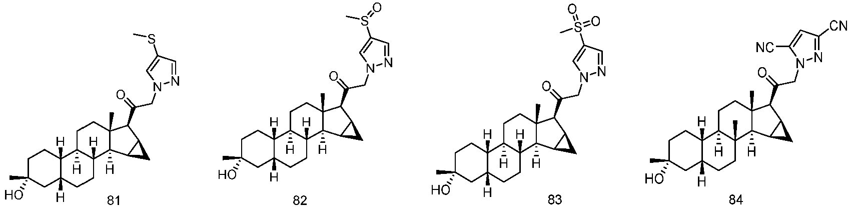

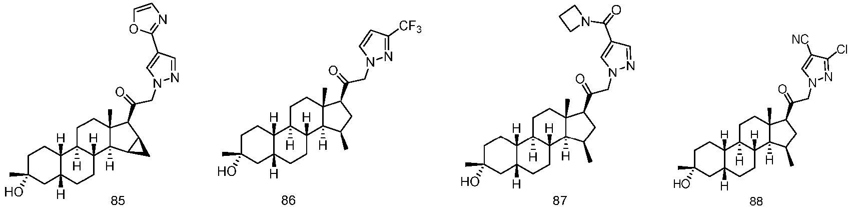

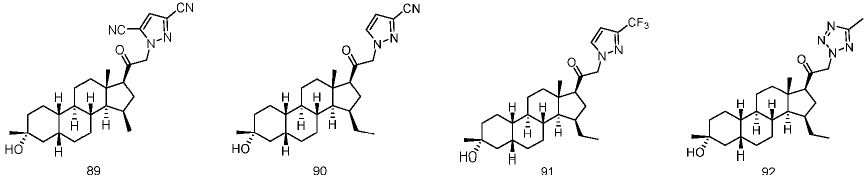

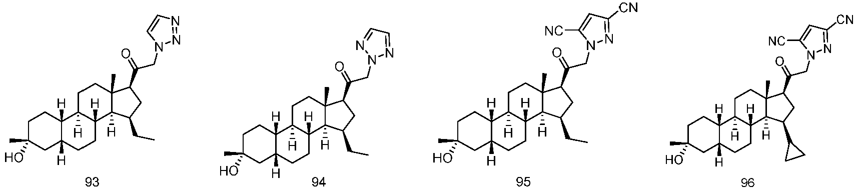

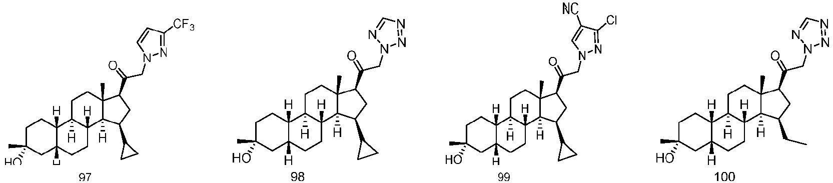

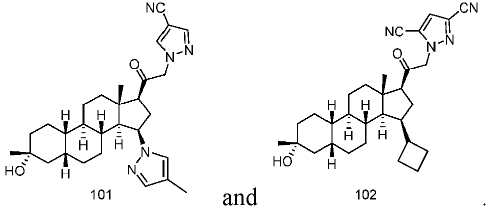

According to the present invention, it is the crystal form of the compound of formula (VI): 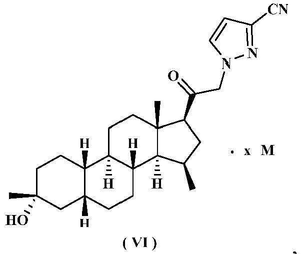 wherein x is selected from the group consisting of 0, 1 and 2 and M is as defined in claim 6.

In a preferred embodiment of the present invention, the pharmaceutical composition is an injection or an oral preparation, and preferably a tablet or a capsule.

In a preferred embodiment of the present invention, the unit dose is 1 to 200 mg, preferably 5 to 200 mg, further preferably 10 to 100 mg, and more preferably 10 to 50 mg.

In a preferred embodiment of the present invention, the unit dose can be 1 mg, 2 mg, 3 mg, 4 mg, 5 mg, 10 mg, 20 mg, 30 mg, 40 mg, 50 mg, 60 mg, 70 mg, 80 mg, 90 mg, 100 mg, 110 mg, 120 mg, 150 mg and the like, preferably 1 to 50 mg, or preferably 1 to 30 mg.

The objective of the present invention is also to provide a use of the crystal form of the compound of formula (Ia) or the pharmaceutical composition according to claim 12 in the preparation of a GABA_{A} receptor regulator medicament.

The objective of the present invention is also to provide the crystal form of the compound of formula (VI), and one or more pharmaceutically acceptable carriers.

The objective of the present invention is also to provide a use of the crystal form of the compound of formula (VI) or the pharmaceutical composition according to claim 31 in the preparation of a GABA_{A} receptor regulator medicament.

In a preferred embodiment of the present invention, the pharmaceutical composition of the GABA_{A} receptor regulator medicament is provided for use in treating a Central Nervous System (CNS)-related disease, wherein the CNS-related disease is selected from the group consisting of sleep disorder, mood disorder, schizophrenia spectrum disorder, spasmodic disorder, memory disorder and/or cognitive disorder, dyskinesia, personality disorder, autism spectrum disorder, pain, traumatic brain injury, vascular disease, substance abuse disorder and/or withdrawal syndrome or tinnitus.

### DESCRIPTION OF THE DRAWINGS

Figure 1 is the XRPD pattern of crystal form I of the free base of formula (VI) *(i.e.,* crystal form I of the free base of Example 40).
Figure 2 is the TGA-DSC spectrum of crystal form I of the free base of formula (VI) *(i.e.,* crystal form I of the free base of Example 40).
Figure 3 is the XRPD pattern of crystal form II of the free base of formula (VI) *(i.e.,* crystal form II of the free base of Example 40).
Figure 4 is the TGA-DSC spectrum of crystal form II of the free base of formula (VI) *(i.e.,* crystal form II of the free base of Example 40).
Figure 5 is the XRPD pattern of crystal form III of the free base of formula (VI) *(i.e.,* crystal form III of the free base of Example 40).
Figure 6 is the TGA-DSC spectrum of crystal form III of the free base of formula (VI) *(i.e.,* crystal form III of the free base of Example 40).
Figure 7 is the XRPD pattern of the mesylate of formula (VI) *(i.e.,* the mesylate of Example 40).
Figure 8 is the DVS spectrum of crystal form I of the free base of formula (VI) *(i.e.,* crystal form I of the free base of Example 40).
Figure 9 is the DVS spectrum of crystal form II of the free base of formula (VI) *(i.e.,* crystal form II of the free base of Example 40).
Figure 10 is the DVS spectrum of crystal form III of the free base of formula (VI) *(i.e.,* crystal form III of the free base of Example 40).
Figure 11 is the schematic diagram of the single crystal structure of crystal form I of the free base of formula (VI) *(i.e.,* crystal form I of the free base of Example 40).

### DETAILED DESCRIPTION OF THE INVENTION

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 8 carbon atoms, more preferably an alkyl having 1 to 6 carbon atoms, and most preferably an alkyl having 1 to 3 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n-*octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. The alkyl group can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl. The alkyl of the present invention is preferably selected from the group consisting of methyl, ethyl, isopropyl, *tert*-butyl, haloalkyl, deuterated alkyl, alkoxy-substituted alkyl and hydroxy-substituted alkyl.

The term "alkylene" refers to an alkyl of which a hydrogen atom is further substituted, for example, "methylene" refers to -CH₂-, "ethylene" refers to -(CH₂)₂-, "propylene" refers to -(CH₂)₃-, "butylene" refers to -(CH₂)₄- and the like. The above substituents can be bonded to different carbon atoms to form a carbon chain, or can be bonded to one carbon atom to form a cycloalkyl. The term "alkenyl" refers to an alkyl as defined above that consists of at least two carbon atoms and at least one carbon-carbon double bond, for example, ethenyl, 1-propenyl, 2-propenyl, 1-, 2- or 3-butenyl and the like. The alkenyl group can be substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyl" refers to a saturated or partially unsaturated monocyclic or polycyclic hydrocarbon substituent group having 3 to 20 carbon atoms, preferably 3 to 8 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring. The cycloalkyl is preferably cyclopropyl, cyclobutyl, cyclohexyl, cyclopentyl and cycloheptyl.

The term "heterocyclyl" refers to a 3 to 20 membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 8 ring atoms; and most preferably 3 to 8 ring atoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably tetrahydrofuranyl, pyrazolidinyl, morpholinyl, piperazinyl and pyranyl. Polycyclic heterocyclyl includes a heterocyclyl having a spiro ring, fused ring or bridged ring. The heterocyclyl having a spiro ring, fused ring or bridged ring is optionally bonded to other group via a single bond, or further bonded to other cycloalkyl, heterocyclyl, aryl and heteroaryl via any two or more atoms on the ring. The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered all-carbon monocyclic ring or polycyclic fused ring *(i.e.,* each ring in the system shares an adjacent pair of carbon atoms with another ring in the system) having a conjugated π-electron system, preferably a 6 to 10 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. Non-limiting examples thereof include:

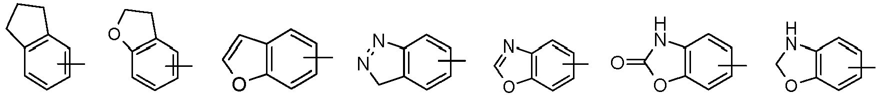

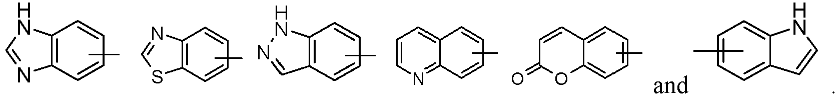

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of O, S and N. The heteroaryl is preferably a 5 to 10 membered heteroaryl, and more preferably a 5 or 6 membered heteroaryl, for example imidazolyl, furyl, thienyl, thiazolyl, pyrazolyl, oxazolyl, pyrrolyl, triazolyl, tetrazolyl, pyridyl, pyrimidinyl, thiadiazolyl, pyrazinyl and the like, preferably triazolyl, thienyl, imidazolyl, pyrazolyl or pyrimidinyl, thiazolyl, and more preferably triazolyl, pyrrolyl, thienyl, thiazolyl and pyrimidinyl. The heteroaryl ring can be fused to the ring of aryl, heterocyclyl or cycloalkyl, wherein the ring bound to the parent structure is heteroaryl ring. Non-limiting examples thereof include:

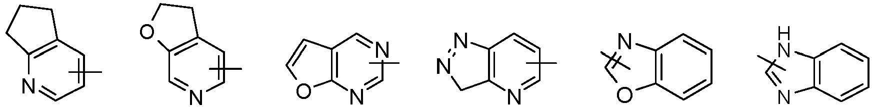

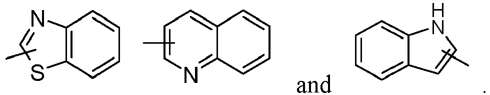

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "alkoxy" refers to an -O-(alkyl) or an -O-(unsubstituted cycloalkyl) group, wherein the alkyl is as defined above. The alkoxy is preferably an alkoxy having 1 to 8 carbon atoms, more preferably an alkoxy having 1 to 6 carbon atoms, and most preferably an alkoxy having 1 to 3 carbon atoms. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

"Haloalkyl" refers to an alkyl group substituted by one or more halogens, wherein the alkyl is as defined above.

"Haloalkoxy" refers to an alkoxy group substituted by one or more halogens, wherein the alkoxy is as defined above.

"Hydroxyalkyl" refers to an alkyl group substituted by hydroxy(s), wherein the alkyl is as defined above.

"Alkenyl" refers to a chain alkenyl, also known as alkene group, which is preferably an alkenyl having 2 to 8 carbon atoms, more preferably an alkenyl having 2 to 6 carbon atoms, and most preferably an alkenyl having 2 to 3 carbon atoms. The alkenyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

"Alkynyl" refers to (CH=C-), which is preferably an alkynyl having 2 to 8 carbon atoms, more preferably an alkynyl having 2 to 6 carbon atoms, and most preferably an alkynyl having 2 to 3 carbon atoms. The alkynyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

"Hydroxy" refers to an -OH group.

"Halogen" refers to fluorine, chlorine, bromine or iodine.

"Amino" refers to a -NH₂ group.

"Cyano" refers to a -CN group.

"Nitro" refers to a -NO₂ group.

"Carboxy" refers to a -C(O)OH group.

Different expressions such as "X is selected from the group consisting of A, B or C", "X is selected from the group consisting of A, B and C", "X is A, B or C", "X is A, B and C" and the like, express the same meaning, that is, X can be any one or more of A, B and C.

"Optional" or "optionally" means that the event or circumstance described subsequently can, but need not, occur, and such a description includes the situation in which the event or circumstance does or does not occur.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive efforts. For example, the combination of amino or hydroxy having free hydrogen and carbon atoms having unsaturated bonds (such as olefinic) may be unstable.

"Stereoisomerism" includes geometric isomerism (cis-trans isomerism), optical isomerism, and conformational isomerism.

The hydrogen atom of the present invention can be substituted by its isotope deuterium. Any of the hydrogen atoms in the compounds of the examples of the present invention can also be substituted by deuterium atom.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present invention, which is safe and effective in mammals and has the desired biological activity.

"TGA" refers to thermogravimetric analysis (TGA) test.

"DSC" refers to differential scanning calorimetry (DSC) test.

"DVS" refers to dynamic vapour sorption (DVS) test.

"XRPD" refers to X-ray powder diffraction (XRPD) test.

"HPLC" refers to high performance liquid chromatography (HPLC) test.

"PK" refers to pharmacokinetic (PK) test.

The present invention will be further described with reference to the following examples, but the examples should not be considered as limiting the scope of the present invention, which is as defined in the claims.

### 1.1 Experimental instruments

### 1.1.1 Some parameters of physical and chemical testing instruments

| | | |
|---|---|---|
| XRPD | Instrument model | Bruker D8 Advance |
| | Diffraction ray | CuK (1.5418) (40 kV, 40 mA), step size 0.02, slit 2 mm |
| | Scan rate | 10°/min (2θ value) |
| | Scan range | 4° ~ 40° (2θ value) |
| DSC | Instrument model | NETZSCH DSC 214 Polyma |
| | Purge gas | Nitrogen |
| | Purge rate | 40 mL/min |
| | Heating rate | 10 °C /min |
| | Temperature range | 25~250 °C |
| | Plate type | Aluminum plate |
| TGA | Instrument model | NETZSCH TG 209 F3 Tarsus |
| | Purge gas | Nitrogen |
| | Purge rate | 40 mL/min |
| | Heating rate | 10 °C /min |
| | Temperature range | Room temperature~300 °C |
| | Plate type | Ceramic pot |
| DVS | Instrument model | SMS Intrinsic |
| | Experiment temperature | 25 °C |
| | Drying time | 0%RH 120 min |
| | Balance dm/dt | 0.02%/min (minimum 10 min, maximum 180 min) |
| | RH(%) measurement step size | 10% |
| | Measuring gradient | 0-95-0 % |
| | Cycles | 2 |
| | Instrument model | Bruker Smart Apex II |
| | light source | Mo target |
| | X-ray | Mo -Kα (*λ*=0.71073Å) |
| | Detector | CCD area detector |
| Single crystal diffraction | Resolution | 0.84 Å |
| | Current and voltage | 40kV, 25 mA |
| | Exposure time | 5 s |
| | Distance from area detector to sample | 50 mm |
| | Test temperature | 173(2) K |

### 1.2 Instruments and liquid phase analysis conditions

### 1.2.1 Instruments and devices

| Instrument name | Model |
|---|---|
| Analytical Balance | Sartorius BSA224S-CW |
| Analytical Balance | Mettler-Toledo XPR2 |
| Pure water machine | Milli-Q Plus, Millipore |
| High performance liquid chromatograph | Agilent1260 |
| Pump | Agilent G1311B |
| Injector | G1329B |
| Column thermostat | G1316A |
| Detector | G1315D |
| Dynamic vapour sorption analyser | SMS Intrinsic |

### 1.2.2 Chromatographic conditions

Chromatographic column: Agilent ZORBAX^{®} Bonus-RP (3.5 µm, 4.6*150 mm)
Flow rate: 1.0 mL/min
Column temperature: 40°C
Detection wavelength: 230 nm
Injection volume: 10.0 µL
Running time: 30 min
Diluent: methanol-water (v/v, 3:1)
Mobile phase: A: water (0.05% trifluoroacetic acid); B: acetonitrile (0.05% trifluoroacetic acid)

| Elution time (min) | Mobile phase A (%) | Mobile phase B (%) |
|---|---|---|
| 0.0 | 60 | 40 |
| 20.0 | 5 | 95 |
| 25.0 | 5 | 95 |
| 25.1 | 60 | 40 |
| 30 | 60 | 40 |

### Preparation of Free Base

### Example 1

### Preparation of 1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carbonitrile

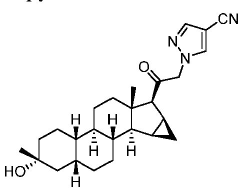

### Step 1: Preparation of 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one

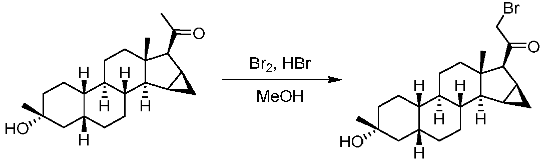

1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)ethan-1-one (70 mg, 0.21 mmol) was dissolved in methanol (3 mL). A drop of hydrogen bromide was added to the solution, followed by the addition of liquid bromine (41 mg, 0.25 mmol), and then the reaction solution was stirred at room temperature for 1 hour. Water (20 mL) was added to the reaction solution, and the aqueous phase was extracted with ethyl acetate (20 mL×2). The organic phases were combined, dried over anhydrous sodium sulfate, filtrated and concentrated to obtain the crude product (87 mg, yield: 100%, crude), which was used directly in the next step.

### Step 2: Preparation of 1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carbonitrile

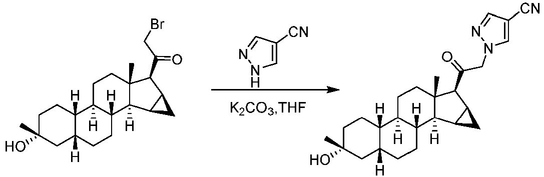

2-Bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one (87 mg, 0.21 mmol), 1H-pyrazole-4-carbonitrile (59 mg, 0.64 mmol) and potassium carbonate (145 mg, 1.05 mmol) were dissolved in tetrahydrofuran (2 mL), and the resulting reaction solution was stirred at room temperature overnight. The reaction solution was filtrated, and the filtrate was concentrated. The resulting crude product was purified by high performance liquid chromatography to obtain 1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carbonitrile (33 mg, yield: 37%).

MS m/z (ESI): 404.2 [M-H₂O+H]⁺.

¹H NMR (400 MHz, CDCl₃) δ 7.88 (s, 1H), 7.82 (s, 1H), 5.24-5.13 (m, 2H), 2.84 (d, J = 2.7 Hz, 1H), 1.98 - 1.92 (m, 1H), 1.87 - 1.77 (m, 4H), 1.76 - 1.66 (m, 3H), 1.57 - 1.51 (m, 1H), 1.46 - 1.24 (m, 15H), 1.12 - 1.02 (m, 1H), 1.00 - 0.96 (m, 1H), 0.78 (s, 3H), 0.54 - 0.46 (m, 1H).

### Example 2

### 1-(2-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-oxoethyl)-1H-pyrazole-3-carbonitrile

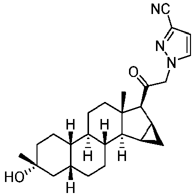

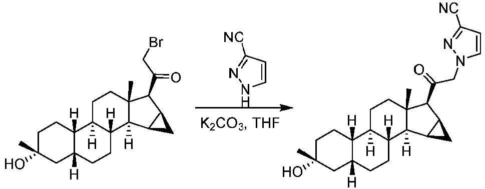

2-Bromo-1-((2R,4aS,4bR,6a8S,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one (80 mg, 0.19 mmol), 1H-pyrazole-3-carbonitrile (55 mg, 0.58 mmol) and potassium carbonate (131 mg, 0.95 mmol) were dissolved in tetrahydrofuran (3 mL), and the resulting reaction solution was stirred at room temperature overnight. The reaction solution was filtrated, and the filtrate was concentrated. The resulting crude product was purified by high performance liquid chromatography to obtain 1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-3-carbonitrile (34.7 mg, yield: 42%).

MS m/z (ESI): 404.2 [M-H₂O+H]⁺

¹H NMR (400 MHz, CDCl₃) δ 7.50 (d, J = 2.4 Hz, 1H), 6.74 (d, J = 2.4 Hz, 1H), 5.29 - 5.13 (m, 2H), 2.83 (d, J = 2.9 Hz, 1H), 1.97 - 1.92 (m, 1H), 1.86 - 1.79 (m, 4H), 1.73 - 1.64 (m, 3H), 1.58 - 1.52 (m, 1H), 1.43 - 1.27 (m, 15H), 1.12 - 0.96 (m, 2H), 0.79 (s, 3H), 0.53 - 0.44 (m, 1H).

### Example 3

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(4-(trifluoromethyl)-1H-pyrazol-1-yl)ethan-1-one

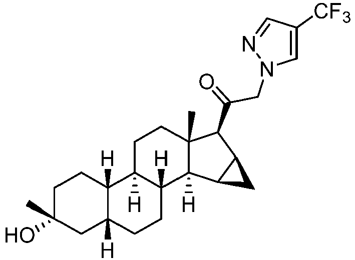

### Step 1: Preparation of 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-(trifluoromethyl)-1H-pyrazol-1-yl)ethan-1-one

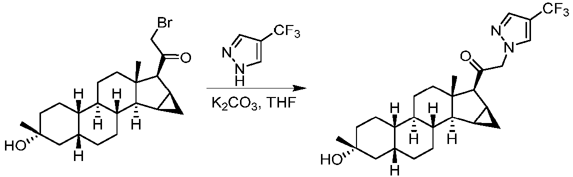

2-Bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one (60 mg, 0.15 mmol), 4-(trifluoromethyl)-1H-pyrazole (60 mg, 0.44 mmol) and potassium carbonate (104 mg, 0.75 mmol) were dissolved in tetrahydrofuran (3 mL), and the resulting reaction solution was stirred at room temperature overnight. The reaction solution was filtrated, and the filtrate was concentrated. The resulting crude product was purified by high performance liquid chromatography to obtain 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-(trifluoromethyl)-1H-pyrazol-1-yl)ethan-1-one (22.2 mg, yield: 33%).

MS m/z (ESI): 465.3 [M+H]⁺.

¹H NMR (400 MHz, CDCl₃) δ 7.74 (s, 1H), 7.73 (s, 1H), 5.25 - 5.09 (m, 2H), 2.83 (d, J = 3.6 Hz, 1H), 2.00 - 1.91 (m, 1H), 1.89 - 1.79 (m, 4H), 1.78 - 1.65 (m, 3H), 1.58 - 1.52 (m, 1H), 1.47 - 1.22 (m, 15H), 1.15 - 0.95 (m, 2H), 0.79 (s, 3H), 0.52 - 0.44 (m, 1H).

### Example 4

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(3-(trifluoromethyl)-1H-pyrazol-1-yl)ethan-1-one

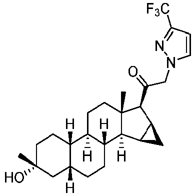

### Step 1: Preparation of 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(3-(trifluoromethyl)-1H-pyrazol-1-yl)ethan-1-one

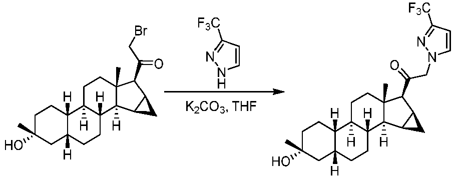

2-Bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one (80 mg, 0.2 mmol), 3-(trifluoromethyl)-1H-pyrazole (80 mg, 0.6 mmol) and potassium carbonate (138 mg, 1.0 mmol) were dissolved in tetrahydrofuran (5 mL), and the resulting reaction solution was stirred at room temperature overnight. The reaction solution was filtrated, and the filtrate was concentrated. The resulting crude product was purified by high performance liquid chromatography to obtain 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(3-(trifluoromethyl)-1H-pyrazol-1-yl)ethan-1-one (17 mg, yield: 18%).

MS m/z (ESI): 447.3 [M-H₂O+H]⁺.

¹H NMR (400 MHz, CDCl₃) δ 7.49 (d, J = 1.9 Hz, 1H), 6.60 (d, J = 1.9 Hz, 1H), 5.27 - 5.13 (m, 2H), 2.83 (d, J = 3.7 Hz, 1H), 1.99 - 1.90 (m, 1H), 1.89 - 1.76 (m, 4H), 1.75 - 1.61 (m, 3H), 1.58 - 1.50 (m, 1H), 1.50 - 1.21 (m, 15H), 1.13 - 0.96 (m, 2H), 0.79 (s, 3H), 0.52 - 0.45 (m, 1H).

### Example 5 and Example 6

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(2H-1,2,3-triazol-2-yl)ethan-1-one (5)

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(1H-1,2,3-triazol-1-yl)ethan-1-one (6)

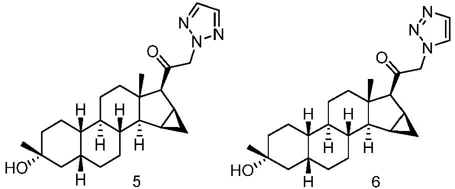

### Step 1: Preparation of 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(2H-1,2,3-triazol-2-yl)ethan-1-one (5) and 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(1H-1,2,3-triazol-1-yl)ethan-1-one (6)

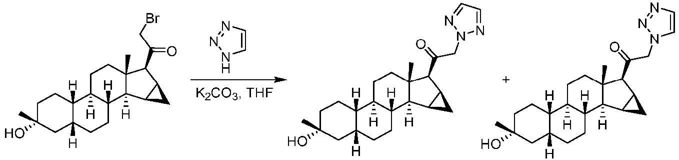

2-Bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one (80 mg, 0.19 mmol), 1H-1,2,3-triazole (40 mg, 0.58 mmol) and potassium carbonate (131 mg, 0.95 mmol) were dissolved in tetrahydrofuran (3 mL), and the resulting reaction solution was stirred at room temperature overnight. The reaction solution was filtrated, and the filtrate was concentrated. The resulting crude product was purified by high performance liquid chromatography to obtain 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(2H-1,2,3-triazol-2-yl)ethan-1-one **(5)** (9.2 mg, yield: 12%) and 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(1H-1,2,3-triazol-1-yl)ethan-1-one **(6)** (17.8 mg, yield: 23%).

### Example 5:

MS m/z (ESI): 380.3 [M-H₂O+H]⁺.

¹H NMR (400 MHz, CDCl₃) δ 7.69 (s, 2H), 5.55 - 5.36 (m, 2H), 2.82 (d, J = 4.1 Hz, 1H), 1.99 - 1.92 (m, 1H), 1.87 - 1.74 (m, 4H), 1.73 - 1.52 (m, 5H), 1.47 - 1.26 (m, 14H), 1.14 - 0.98 (m, 2H), 0.84 (s, 3H), 0.52 - 0.43 (m, 1H).

### Example 6:

MS m/z (ESI): 398.3 [M+H]⁺.

¹H NMR (400 MHz, CDCl₃) δ 7.76 (s, 1H), 7.66 (s, 1H), 5.52 - 5.35 (m, 2H), 2.87 (d, J = 3.9 Hz, 1H), 2.02 - 1.92 (m, 1H), 1.89 - 1.78 (m, 4H), 1.76 - 1.65 (m, 3H), 1.59 - 1.52 (m, 1H), 1.48 - 1.23 (m, 15H), 1.15 - 0.98 (m, 2H), 0.80 (s, 3H), 0.56 - 0.45 (m, 1H).

### Example 7 and Example 8

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(2H-tetrazol-2-yl)ethan-1-one (7)

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(1H-tetrazol-1-yl)ethan-1-one (8)

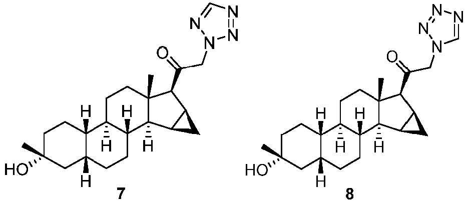

### Step 1: Preparation of 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(2H-tetrazol-2-yl)ethan-1-one (7) and 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(1H-tetrazol-1-yl)ethan-1-one (8)

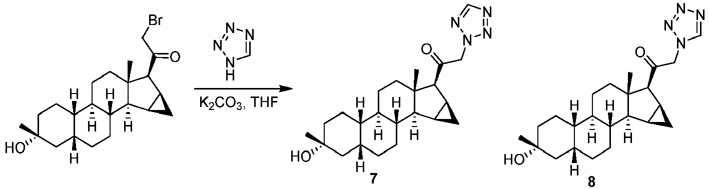

2-Bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one (50 mg, 0.12 mmol), 1H-tetrazole (26 mg, 0.37 mmol) and potassium carbonate (83 mg, 0.6 mmol) were dissolved in tetrahydrofuran (3 mL), and the resulting reaction solution was stirred at room temperature overnight. The reaction solution was filtrated, and the filtrate was concentrated. The resulting crude product was purified by high performance liquid chromatography to obtain 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(2H-tetrazol-2-yl)ethan-1-one (7) (11.6 mg, yield: 24%) and 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(1H-tetrazol-1-yl)ethan-1-one **(8)** (4.4 mg, yield: 9%).

### Example 7:

MS m/z (ESI): 381.2 [M-H₂O+H]⁺

¹H NMR (400 MHz, CDCl₃) δ8.58 (s, 1H), 5.74 - 5.62 (m, 2H), 2.88 (d, *J* = 3.8 Hz, 1H), 1.97 - 1.91 (m, 1H), 1.87 - 1.79 (m, 4H), 1.75 - 1.64 (m, 3H), 1.58 - 1.55 (m, 1H), 1.45 - 1.35 (m, 7H), 1.34 - 1.24 (m, 8H), 1.14 - 1.01 (m, 2H), 0.85 (s, 3H), 0.56 - 0.48 (m, 1H).

### Example 8:

MS m/z (ESI): 399.3 [M+H]⁺

¹H NMR (400 MHz, CDCl₃) δ8.77 (s, 1H), 5.57 - 5.39 (m, 2H), 2.90 (s, 1H), 2.00 - 1.91 (m, 1H), 1.88 - 1.79 (m, 4H), 1.76 - 1.65 (m, 3H), 1.59 - 1.56 (m, 1H), 1.46 - 1.37 (m, 7H), 1.35 - 1.24 (m, 8H), 1.13 - 0.99 (m, 2H), 0.79 (s, 3H), 0.58 - 0.49 (m, 1H).

### Example 9

### 1-(2-((3R,5R,8R,9R,10S, 13S,14S,15R)-3-Hydroxy-3,13,15-trimethylhexadecahydro-1H-cyclopenta[a]phenanthren-17-yl)-2-oxoethyl)-1H-pyrazole-4-carbonitrile

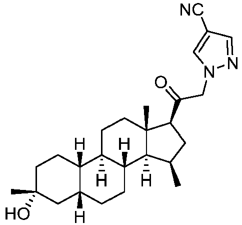

### Step 1: (3R,5R,8R,9R,10S,13S,14S,15R)-3-Hydroxy-3,13,15-trimethylhexadecahydro-17H-cyclopenta[a]phenanthren-17-one

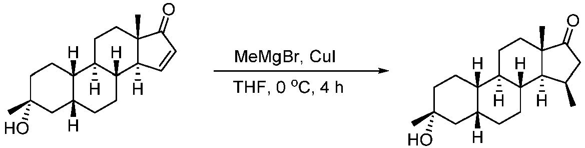

3.0 M methylmagnesium bromide (8.5 mL, 25.5 mmol) and 20 mL of anhydrous tetrahydrofuran were added to a dry 100 mL round bottom flask. The reaction system was purged with nitrogen, and cooled to 0°C. Cuprous iodide (3.94 g, 20.7 mmol) was added, and then the reaction solution was stirred at 0°C for 1 hour. (3R,5R,8R,9R,10S,13S,14S)-3-Hydroxy-3,13-dimethyl-1,2,3,4,5,6,7,8,9,10,11,12,13,14-tetradecahydro-17H-cyclopenta[a]phenanthren-17-one (2 g, 6.9 mmol) was dissolved in 10 ml of anhydrous tetrahydrofuran, and the resulting solution was slowly added dropwise to the reaction system. The reaction solution was stirred for 3 hours, and TLC showed that the reaction was completed. Saturated ammonium chloride solution was added to quench the reaction, and the reaction solution was extracted with ethyl acetate. The organic phase was washed with water and saturated saline, dried over anhydrous sodium sulfate, filtrated and concentrated by rotary evaporation to dryness. The resulting crude product was purified by column chromatography (petroleum ether: ethyl acetate=2:1) to obtain (3R,5R,8R,9R,10S,13S,14S,15R)-3-hydroxy-3,13,15-trimethylhexadecahydro-17H-cyclopenta[a]phenanthren-17-one (1.56 g, yield: 75%).

¹H NMR (400 MHz, CDCl₃) *δ* 2.51-2.42 (m, 2H), 2.24 (d, *J* = 17.6 Hz, 1H), 1.89-1.63 (m, 7H), 1.54-1.18 (m, 16H), 1.10 (d, *J =* 7.6 Hz, 3H), 1.03 (s, 3H).

### Step 2: (3R,5R,8R,9R,10S,13S,14S,15R,E)-17-Ethylidene-3,13,15-trimethylhexadecahydro-1H-cyclopenta[a]phenanthren-3-ol

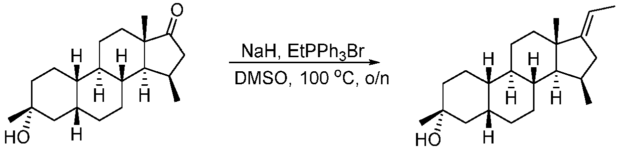

Ethyltriphenylphosphonium bromide (18.5 g, 50 mmol) was dissolved in anhydrous dimethyl sulfoxide (50 mL), and the reaction system was purged with nitrogen. Sodium hydride (2.0 g, 50 mmol) was added, and then the reaction solution was stirred at room temperature for 1 hour. (3R,5R,8R,9R,10S,13S,14S,15R)-3-Hydroxy-3,13,15-trimethylhexadecahydro-17H-cyclopenta[a]phenanthren-17-one (1.52 g, 5 mmol) was added, and then the reaction solution was stirred at 100°C overnight. The reaction solution was cooled to room temperature. Water (200 mL) was added to the reaction solution to quench the reaction, and the aqueous phase was extracted with ethyl acetate (200 mL×2). The organic phases were combined, dried over anhydrous sodium sulfate, filtrated and concentrated. The resulting crude product was purified by column chromatography (petroleum ether/ethyl acetate: 50/1 - 3/1) to obtain (3R,5R,8R,9R,10S,13S,14S,15R,E)-17-ethylidene-3,13,15-trimethylhexadecahydro-1H-cyclopenta[a]phenanthren-3-ol (1.37 g, yield: 86%).

### Step 3: (3R,5R,8R,9R,10S,13S,14S,15R,17S)-17-((R)-1-Hydroxyethyl)-3,13,15-trimethylhexadecahydro-1H-cyclopenta[a]phenanthren-3-ol

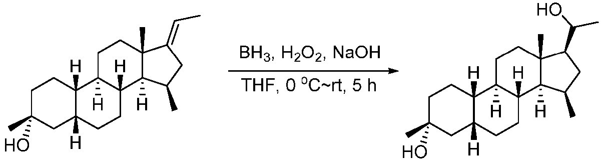

(3R,5R,8R,9R,10S,13S,14S,15R,E)-17-Ethylidene-3,13,15-trimethylhexadecahydro-1H-cyclopenta[a]phenanthren-3-ol (1.37 g, 4.33 mmol) was dissolved in anhydrous tetrahydrofuran (50 mL). The solution was cooled to 0°C, and then BH₃/THF (43 mL, 43 mmol) was added dropwise. The reaction solution was stirred at room temperature for 3 hours, and TLC showed that the reaction was completed. The reaction solution was cooled to 0°C, and then 3 M aqueous NaOH solution (40 mL) was slowly added, followed by the addition of H₂O₂ (30 mL). The reaction solution was stirred at room temperature for 2 hours, and TLC showed that the reaction was completed. Ethyl acetate (50 mL) was added, and then the reaction solution was washed with saturated aqueous Na₂S₂O₃ solution (30 mL) and water (30 mL) successively. The organic phase was dried over anhydrous sodium sulfate, filtrated and concentrated to obtain the crude product (1.37 g), which was used directly in the next step.

### Step 4: 1-((3R,5R,8R,9R,10S,13S,14S,15R,17S)-3-Hydroxy-3,13,15-trimethylhexadecahydro-1H-cyclopenta[a]phenanthren-17-yl)ethan-1-one

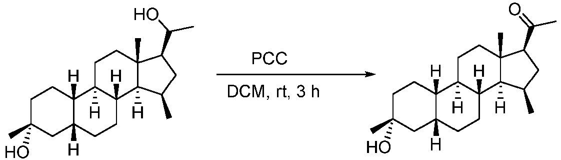

(3R,5R,8R,9R,10S,13S,14S,15R,17S)-17-((R)-1-Hydroxyethyl)-3,13,15-trimethylhexadecahydro-1H-cyclopenta[a]phenanthren-3-ol (1.37 g, crude) was dissolved in dichloromethane (30 mL). PCC (1.8 g, 8.66 mmol) was added, and then the reaction solution was stirred at room temperature for 2 hours. The reaction solution was filtrated, and the organic phase was concentrated. The resulting crude product was purified by column chromatography (petroleum ether/ethyl acetate: 1/1) to obtain 1-((3R,5R,8R,9R,10S,13S,14S,15R,17S)-3-hydroxy-3,13,15-trimethylhexadecahydro-1H-cyclopenta[a]phenanthren-17-yl)ethan-1-one (780 mg, yield of two steps: 54.3%).

¹H NMR (400 MHz, CDCl₃) δ 2.49 (dd, *J* = 8.8, 10.4 Hz, 1H), 2.14-2.03 (m, 5H), 1.95-1.79 (m, 5H), 1.69-1.06 (m, 18H), 0.96 (d, *J =* 7.2 Hz, 3H), 0.78 (s, 3H).

### Step 5: 2-Bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one

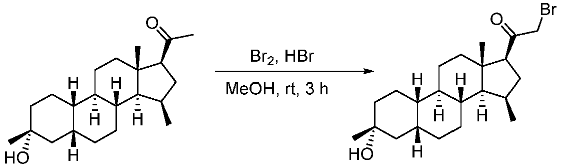

1-((3R,5R,8R,9R,10S,13S,14S,15R,17S)-3-Hydroxy-3,13,15-trimethylhexadecahydro-1H-cyclopenta[a]phenanthren-17-yl)ethan-1-one (333 mg, 1 mmol) was dissolved in methanol (10 mL). A drop of hydrogen bromide was added to the solution, followed by the addition of liquid bromine (176 mg, 1.1 mmol) was added, and then the reaction solution was stirred at room temperature for 1 hour. Water (20 mL) was added to the reaction solution, and the aqueous phase was extracted with ethyl acetate (20 mL×2). The organic phases were combined, dried over anhydrous sodium sulfate, filtrated and concentrated to obtain the crude product (413 mg, crude), which was used directly in the next step.

### Step 6: 1-(2-((3R,5R,8R,9R,10S,13S,14S,15R)-3-Hydroxy-3,13,15-trimethylhexadecahydro-1H-cyclopenta[a]phenanthren-17-yl)-2-oxoethyl)-1H-pyrazole-4-carbonitrile

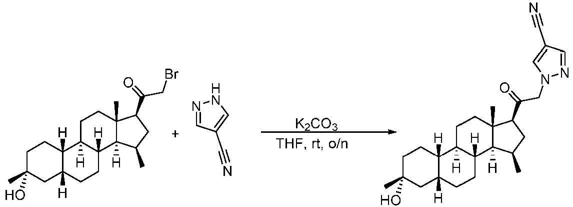

2-Bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one (82 mg, 0.2 mmol), 1H-pyrazole-4-carbonitrile (28 mg, 0.3 mmol) and potassium carbonate (54 mg, 0.3 mmol) were dissolved in anhydrous tetrahydrofuran (5 mL), and the resulting reaction solution was stirred at room temperature overnight. The reaction solution was filtrated, and the filtrate was concentrated. The resulting crude product was purified by high performance liquid chromatography to obtain 1-(2-((3R,5R,8R,9R,10S,13S,14S,15R)-3-hydroxy-3,13,15-trimethylhexadecahydro-1H-cyclopenta[a]phenanthren-17-yl)-2-oxoethyl)-1H-pyrazole-4-carbonitrile (41 mg, yield: 48%).

¹H NMR (400 MHz, CDCl₃) δ 7.86 (s, 1H), 7.81 (s, 1H), 5.01 (d, *J =* 17.9 Hz, 1H), 4.90 (d, *J* = 17.9 Hz, 1H), 2.62-2.48 (m, 1H), 2.30-2.06 (m, 3H), 2.04-1.75 (m, 7H), 1.75-1.04 (m, 15H), 0.99 (d, *J =* 7.1 Hz, 3H), 0.84 (s, 3H).

MS m/z (ESI): 424.6 [M+H]⁺.

### Example 14

### 2-(4-Chloro-1H-pyrazol-1-yl)-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one

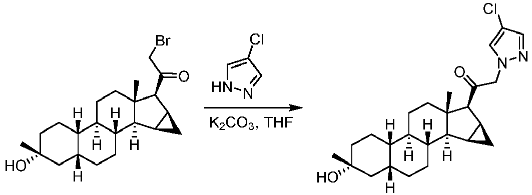

In accordance with Example 5, 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one and 4-chloro-1H-pyrazole were used as the starting materials, accordingly, 2-(4-chloro-1H-pyrazol-1-yl)-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one (21.9 mg, yield: 26%) was obtained.

MS m/z (ESI): 431.2 [M+H]⁺

¹H NMR (400 MHz, CDCl₃) δ7.45 (d, *J* = 11.8 Hz, 2H), 5.17 - 5.01 (m, 2H), 2.81 (d, *J* = 3.5 Hz, 1H), 1.99 - 1.91 (m, 1H), 1.86 - 1.79 (m, 3H), 1.77 - 1.62 (m, 3H), 1.57 - 1.49 (m, 3H), 1.44 - 1.20 (m, 14H), 1.12 - 0.96 (m, 2H), 0.78 (s, 3H), 0.51 - 0.44 (m, 1H).

### Example 24 and Example 25

### 3-Cyclopropyl-1-(2-((2R,4aS,4bR,6a8S,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carbonitrile (24)

### 5-Cyclopropyl-1-(2-((2R,4aS,4bR,6a8S,7S,7aS,8aR,8bR 8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carbonitrile (25)

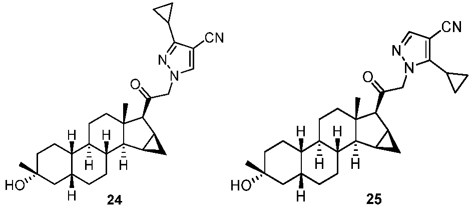

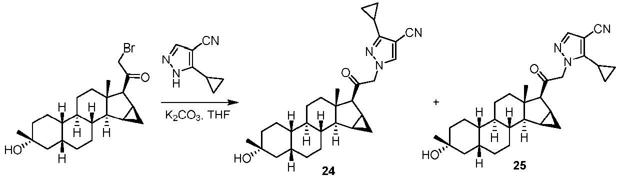

In accordance with Example 5, 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one and 5-cyclopropyl-1H-pyrazole-4-carbonitrile were used as the starting materials, accordingly, 3-cyclopropyl-1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carbonitrile **(24)** (19.2 mg, yield: 21%) and 5-cyclopropyl-1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carbonitrile **(25)** (3.0 mg, yield: 3.3%) were obtained.

### Example 24:

MS m/z (ESI): 462.3 [M+H]⁺

¹H NMR (400 MHz, CDCl₃) δ7.73 (s, 1H), 5.14 - 4.98 (m, 2H), 2.80 (d, *J* = 2.2 Hz, 1H), 2.05 - 1.89 (m, 2H), 1.86 - 1.78 (m, 4H), 1.75 - 1.64 (m, 3H), 1.58 - 1.52 (m, 2H), 1.46 - 1.33 (m, 7H), 1.33 - 1.20 (m, 7H), 1.14 - 0.92 (m, 6H), 0.76 (s, 3H), 0.53 - 0.43 (m, 1H).

### Example 25:

MS m/z (ESI): 462.3 [M+H]⁺

¹H NMR (400 MHz, CDCl₃) δ7.69 (s, 1H), 5.33 - 5.13 (m, 2H), 2.85 (d, *J* = 3.8 Hz, 1H), 2.00 - 1.92 (m, 1H), 1.86 - 1.79 (m, 3H), 1.76 - 1.62 (m, 4H), 1.60 - 1.51 (m, 2H), 1.45 - 1.34 (m, 7H), 1.34 - 1.22 (m, 7H), 1.17 - 0.94 (m, 7H), 0.81 (s, 3H), 0.54 - 0.45 (m, 1H).

### Example 28 and Example 29

### 1-(2-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-oxoethyl)-3-methyl-1H-pyrazole-4-carbonitrile (28)

### 1-(2-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-oxoethyl)-5-methyl-1H-pyrazole-4-carbonitrile (29)

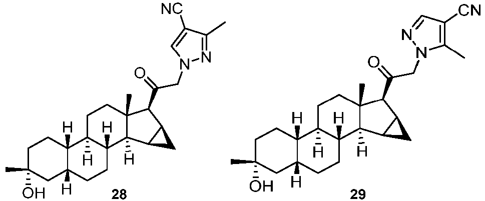

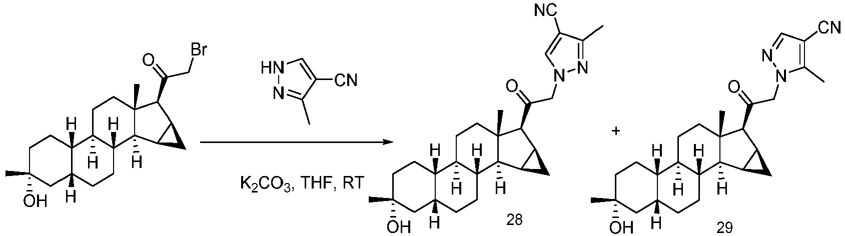

In accordance with Example 5, 2-bromo-1-((2R,4aS,4bR,6aS,7 S,7aS, 8aR, 8bR, 8cR,10aR)-2-hydroxy-2,6a-dimethyloctadecahydrocyclopropa[4,5]cyclopenta[1,2-a]phenanthren-7-yl)ethan-1-one and 3-methyl-1H-pyrazole-4-carbonitrile were used as the starting materials, accordingly, a mixture of Example 28 and Example 29 (approximately 3:1) (25.9 mg, white solid, yield: 39.3%) was obtained. The mixture was further separated by preparative chromatography to obtain 1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-3-methyl-1H-pyrazole-4-carbonitrile (28) and 1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-5-methyl-1H-pyrazole-4-carbonitrile (29).
Example 37A: MS m/z (ESI): 436.3[M+H]⁺
Example 37B: MS m/z (ESI): 436.3[M+H]⁺

### Example 30

### 2-(4-Fluoro-1H-pyrazol-1-yl)-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one

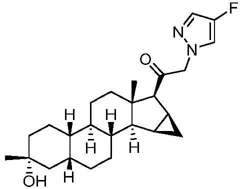

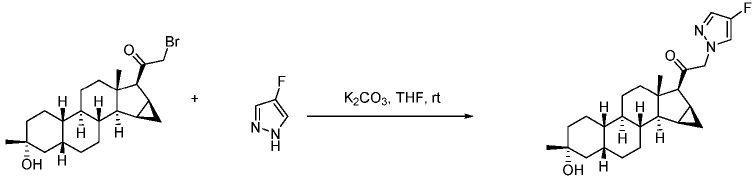

In accordance with Example 5, 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one and 4-fluoropyrazole were used as the starting materials, accordingly, 2-(4-fluoro-1H-pyrazol-1-yl)-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one (6.2 mg, white solid, yield: 7.7%) was obtained .

MS m/z (ESI): 415.2[M+H]⁺
¹H NMR (400 MHz, CDCl₃) δ 7.37 (d, *J* = 4.0 Hz, 1H), 7.32 (d, *J* = 4.0 Hz, 1H), 5.03 (d, *J* = 3.6 Hz, 2H), 2.80 (d, *J* = 3.6 Hz, 1H), 2.01-1.93 (m, 2H), 1.85-1.80 (m, 3H), 1.73-1.61 (m, 6H), 1.41-1.32 (m, 9H), 1.27 (s, 3H), 1.12-1.02 (m, 2H), 0.99-0.96 (m, 1H), 0.78 (s, 3H), 0.50-0.44 (m, 1H).

### Example 35

### Ethyl 1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carboxylate

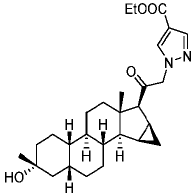

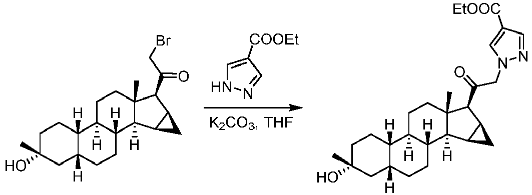

In accordance with Example 5, 2-bromo-1-((2R,4aS,4bR,6aS,7 S,7aS, 8aR, 8bR, 8cR, 10aR)-2-hydroxy-2,6a-dimethyloctadecahydrocyclopropa[4,5]cyclopenta[1,2-a]phenanthren-7-yl)ethan-1-one and ethyl 1H-pyrazole-4-carboxylate were used as the starting materials, accordingly, ethyl 1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carboxylate (29.6 mg, white solid, yield: 43%) was obtained.

MS m/z (ESI): 469.3 [M+H]⁺
¹H NMR (400 MHz, CDCl₃) δ 7.95 (s, 2H), 5.32 - 4.99 (m, 2H), 4.36 - 4.24 (m, 2H), 2.83 (s, 1H), 1.99 - 1.92 (m, 1H), 1.87 - 1.79 (m, 3H), 1.72 - 1.52 (m, 6H), 1.49 - 1.16 (m, 17H), 1.14 - 0.96 (m, 2H), 0.83 (s, 3H), 0.53 - 0.44 (m, 1H).

### Example 39

### 1-((3R,5R,8R,9R,10S,13S,14S,15R,17S)-3-Hydroxy-3,13,15-trimethylhexadecahydro-1H-cyclopenta[a]phenanthren-17-yl)-2-(4-(trifluoromethyl)-1H-pyrazol-1-yl)ethan-1-one

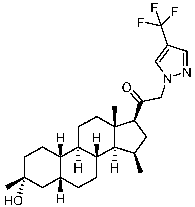

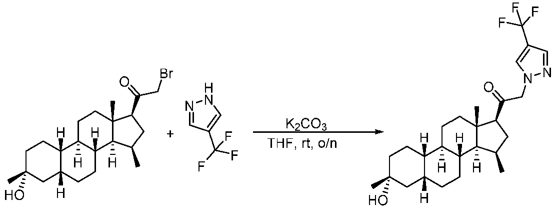

2-Bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one (82 mg, 0.2 mmol), 4-(trifluoromethyl)-1H-pyrazole (41 mg, 0.3 mmol) and potassium carbonate (54 mg, 0.3 mmol) were dissolved in anhydrous tetrahydrofuran (5 mL), and the resulting reaction solution was stirred at room temperature overnight. The reaction solution was filtrated, and the filtrate was concentrated. The resulting crude product was purified by high performance liquid chromatography to obtain 1-((3R,5R,8R,9R,10S,13S,14S,15R,17S)-3-hydroxy-3,13,15-trimethylhexadecahydro-1H-cyclopenta[a]phenanthren-17-yl)-2-(4-(trifluoromethyl)-1H-pyrazol-1-yl)ethan-1-one (24.6 mg, yield: 26%).

¹H NMR (400 MHz, CDCl₃) δ 7.72 (s, 2H), 5.00 (d, *J* = 16 Hz, 1H), 4.90 (d, *J* = 16 Hz, 1H), 2.58-2.54 (m, 1H), 1.88-1.83 (m, 2H), 1.69-1.58 (m, 5H), 1.50-1.25 (m, 19H), 0.98 (d, *J* = 8.0 Hz, 3H), 0.85 (s, 3H).

MS m/z (ESI): 467.3 [M+H]⁺

### Example 40

### 1-(2-((3R,5R,8R,9R,10S,13S,14S,15R,17S)-3-Hydroxy-3,13,15-trimethylhexadecahydro-1H-cyclopenta[a]phenanthren-17-yl)-2-oxoethyl)-1H-pyrazole-3-carbonitrile

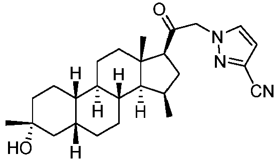

### Step 1: Preparation of 1-(2-((3R,5R,8R,9R,10S,13S,14S,15R,17S)-3-hydroxy-3,13,15-trimethylhexadecahydro-1H-cyclopenta[a]phenanthren-17-yl)-2-oxoethyl)-1H-pyrazole-3-carbonitrile

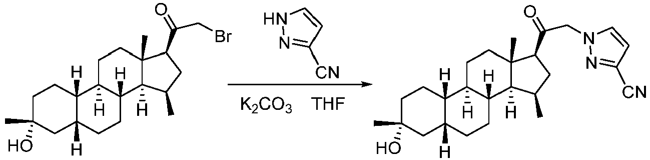

In accordance with Example 5, 2-bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one was used as the starting material, accordingly, 1-(2-((3R,5R,8R,9R,10S,13S,14S,15R,17S)-3-hydroxy-3,13,15-trimethylhexadecahydro-1H-cyclopenta[a]phenanthren-17-yl)-2-oxoethyl)-1H-pyrazole-3-carbonitrile (12.5 mg, yield: 20.2%) was obtained.

MS m/z (ESI): 424.1[M+H]⁺.

¹H NMR (400 MHz, CDCl₃) δ 7.48 (d, *J =* 2.4 Hz, 1H), 6.73 (d, *J =* 2.4 Hz, 1H), 5.07 - 4.84 (m, 2H), 2.55 (t, *J* = 8.1 Hz, 1H), 2.33 - 1.06 (m, 25H), 0.98 (d, *J* = 7.0 Hz, 3H), 0.84 (s, 3H).

### Example 41 and Example 42

### 1-((3R,5R,8R,9R,10S,13S,14S,15R,17S)-3-Hydroxy-3,13,15-trimethylhexadecahydro-1H-cyclopenta[a]phenanthren-17-yl)-2-(2H-1,2,3-triazol-2-yl)ethan-1-one (41)

### 1-((3R,5R,8R,9R,10S,13S,14S,15R,17S)-3-Hydroxy-3,13,15-trimethylhexadecahydro-1H-cyclopenta[a]phenanthren-17-yl)-2-(1H-1,2,3-triazol-1-yl)ethan-1-one (42)

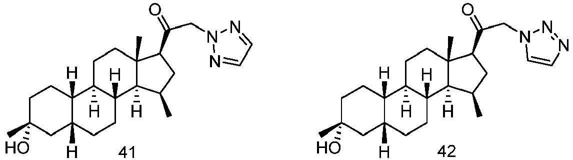

### Step 1: Preparation of 1-((3R,5R,8R,9R,10S,13S,14S,15R,17S)-3-hydroxy-3,13,15-trimethylhexadecahydro-1H-cyclopenta[a]phenanthren-17-yl)-2-(2H-1,2,3-triazol-2-yl)ethan-1-one and 1-((3R,5R,8R,9R,10S,13S,14S,15R,17S)-3-hydroxy-3,13,15-trimethylhexadecahydro-1H-cyclopenta[a]phenanthren-17-yl)-2-(1H-1,2,3-triazol-1-yl)ethan-1-one

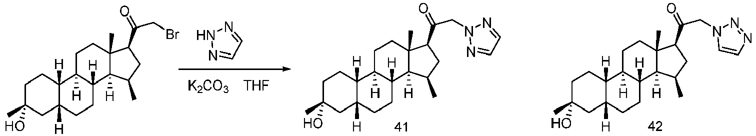

In accordance with Example 5, 2-bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one was used as the starting material, accordingly, 1-((3R,5R,8R,9R,10S,13S,14S,15R,17S)-3-hydroxy-3,13,15-trimethylhexadecahydro-1H-cyclopenta[a]phenanthren-17-yl)-2-(2H-1,2,3-triazol-2-yl)ethan-1-one (9.5 mg, yield: 16.3%) and 1-((3R,5R,8R,9R,10S,13S,14S,15R,17S)-3-hydroxy-3,13,15-trimethylhexadecahydro-1H-cyclopenta[a]phenanthren-17-yl)-2-(1H-1,2,3-triazol-1-yl)ethan-1-one (13 mg, yield: 22.3%) were obtained .

### Example 41:

MS m/z (ESI): 400.2[M+H]⁺.

1H NMR (400 MHz, CDCl₃) δ 7.68 (s, 2H), 5.29 - 5.15 (m, 2H), 2.52 (t, *J=* 8.0 Hz, 1H), 2.17 - 1.29 (m, 24H), 1.15 - 1.05 (m, 1H), 0.97 (d, *J* = 7.0 Hz, 3H), 0.87 (s, 3H).

### Example 42:

MS m/z (ESI): 400.2[M+H]⁺.

1H NMR (400 MHz, CDCl₃) δ7.77 (s, 1H), 7.65 (s, 1H), 5.32 - 5.02 (m, 2H), 2.60 (t, *J* = 9.1 Hz, 1H), 2.30 - 2.12 (m, 2H), 1.99 - 1.27 (m, 22H), 1.17 - 1.06 (m, 1H), 0.99 (d, *J* = 6.9 Hz, 3H), 0.84 (s, 3H)

### Example 43

### 1-((3R,5R,8R,9R,10S,13S,14S,15R,17S)-3-Hydroxy-3,13,15-trimethylhexadecahydro-1H-cyclopenta[a]phenanthren-17-yl)-2-(5-methyl-2H-tetrazol-2-yl)ethan-1-one

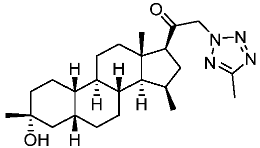

### Step 1: Preparation of 1-((3R,5R,8R,9R,10S,13S,14S,15R,17S)-3-hydroxy-3,13,15-trimethylhexadecahydro-1H-cyclopenta[a]phenanthren-17-yl)-2-(5-methyl-2H-tetrazol-2-yl)ethan-1-one

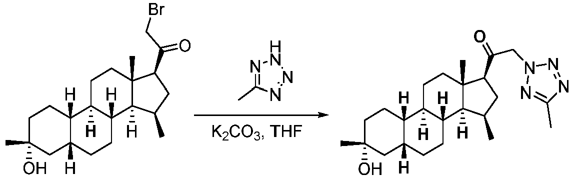

In accordance with Example 5, 2-bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one was used as the starting material, accordingly, 1-((3R,5R,8R,9R,10S,13S,14S,15R,17S)-3-hydroxy-3,13,15-trimethylhexadecahydro-1H-cyclopenta[a]phenanthren-17-yl)-2-(5-methyl-2H-tetrazol-2-yl)ethan-1-one (4.7 mg, yield: 7%) was obtained.

MS m/z (ESI): 415.3[M+H]⁺

¹H NMR (400 MHz, CDCl₃) δ 5.36 (s, 2H), 2.57 (s, 3H), 2.27 - 2.11 (m, 2H), 2.00 -198 (m, 1H), 1.87 - 1.83 (m, 5H), 1.77 - 1.57 (m, 6H), 1.52 - 1.30 (m, 12H), 1.11 (s, 2H), 0.98 (d, *J* = 7.0 Hz, 3H), 0.88 (s, 3H).

### Example 44

### 1-((3R,5R,8R,9R,10S,13S,14S,15R,17S)-3-Hydroxy-3,13,15-trimethylhexadecahydro-1H-cyclopenta[a]phenanthren-17-yl)-2-(2H-tetrazol-2-yl)ethan-1-one

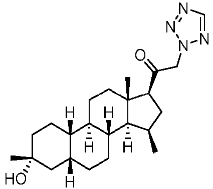

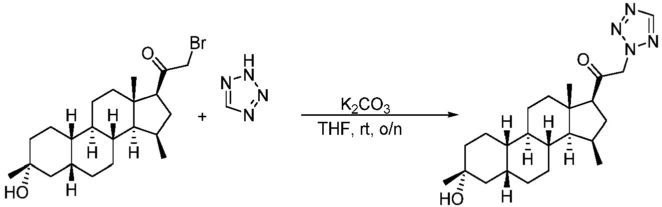

2-Bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one (82 mg, 0.2 mmol), 2H-tetrazole (21 mg, 0.3 mmol) and potassium carbonate (54 mg, 0.3 mmol) were dissolved in anhydrous tetrahydrofuran (5 mL), and the resulting reaction solution was stirred at room temperature overnight. The reaction solution was filtrated, and the filtrate was concentrated. The resulting crude product was purified by high performance liquid chromatography to obtain 1-((3R,5R,8R,9R,10S,13S,14S,15R,17S)-3-hydroxy-3,13,15-trimethylhexadecahydro-1H-cyclopenta[a]phenanthren-17-yl)-2-(2H-tetrazol-2-yl)ethan-1-one (15.7 mg, yield: 20%).

MS m/z (ESI): 401.3 [M+H]⁺
¹H NMR (400 MHz, CDCl₃) δ 8.58 (s, 1H), 4.46 (s, 2H), 2.62-2.58 (m, 1H), 1.89-1.84 (m, 2H), 1.70-1.26 (m, 23H), 0.99 (d, *J* = 8.0 Hz, 3H), 0.88 (s, 3H).

### Example 47

### 3-Cyclopropyl-1-(2-((3R,5R,8R,9R,10S,13S,14S,15R,17S)-3-hydroxy-3,13,15-trimethylhexadecahydro-1H-cyclopenta[a]phenanthren-17-yl)-2-oxoethyl)-1H-pyrazole-4-carbonitrile

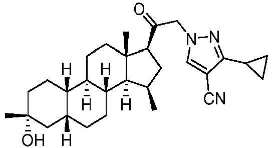

### Step 1: Preparation of 3-cyclopropyl-1-(2-((3R,5R,8R,9R,10S,13S,14S,15R,17S)-3-hydroxy-3,13,15-trimethylhexadecahydro-1H-cyclopenta[a]phenanthren-17-yl)-2-oxoethyl)-1H-pyrazole-4-carbonitrile

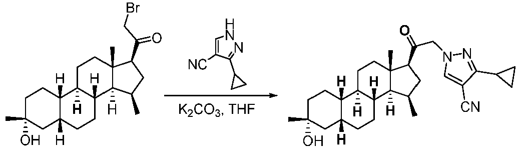

In accordance with Example 5, 2-bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one was used as the starting material, accordingly, 3-cyclopropyl-1-(2-((3R,5R,8R,9R, 10S,13S,14S,15R,17S)-3-hydroxy-3,13,15-trimethylhexadecahydro-1H-cyclopenta[a]phenanthren-17-yl)-2-oxoethyl)-1H-pyrazole-4-carbonitrile (30 mg, yield: 41%) was obtained .

MS m/z (ESI): 464.3[M+H]⁺
¹H NMR (400 MHz, CDCl₃) δ 7.71 (s, 1H), 4.82 (q, *J* = 17.9 Hz, 2H), 2.51 - 2.46 (m, 1H), 2.27 - 2.18 (m, 1H), 2.19 - 2.08 (m, 1H), 2.04 - 1.91 (m, 2H), 1.90 - 1.80 (m, 4H), 1.68 - 1.60 (m, 4H), 1.57 (s, 3H), 1.51 - 1.29 (m, 8H), 1.18 - 1.03 (m, 3H), 1.02 - 0.95 (m, 7H), 0.82 (s, 3H).

### Example 48

### 1-(2-((3R,5R,8R,9R,10S,13S,14S,15S)-15-Cyclopropyl-3-hydroxy-3,13-dimethylhexadecahydro-1H-cyclopenta[a]phenanthren-17-yl)-2-oxoethyl)-1H-pyrazole-4-carbonitrile

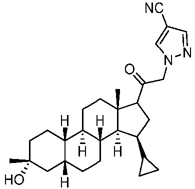

### Step 1: (3R,5R,8R,9R,10S,13S,14S,15S)-15-Cyclopropyl-3-hydroxy-3,13-dimethylhexadecahydro-17H-cyclopenta[a]phenanthren-17-one

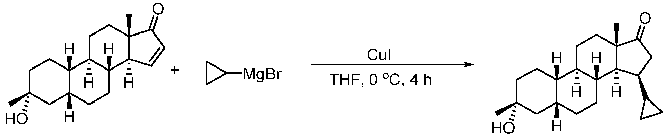

1.0 M cyclopropylmagnesium bromide (12.7 mL, 12.7 mmol) and 20 mL of anhydrous tetrahydrofuran were added to a dry 100 mL round bottom flask. The reaction system was purged with nitrogen, and cooled to 0°C. Cuprous iodide (1.97 g, 10.4 mmol) was added, and then the reaction solution was stirred at 0°C for 1 hour. (3R,SR,8R,9R, 10S, 13S, 14S)-3-Hydroxy-3, 13-dimethyl-1,2,3,4,5,6,7,8,9,10,11,12,13,14-tetradecahydro-17H-cyclopenta[a]phenanthren-17-one (1 g, 3.5 mmol) was dissolved in 10 ml of anhydrous tetrahydrofuran, and the resulting solution was slowly added dropwise to the reaction system. The reaction solution was stirred for 3 hours, and TLC showed that the reaction was completed. Saturated ammonium chloride solution was added to quench the reaction, and the reaction solution was extracted with ethyl acetate. The organic phase was washed with water and saturated saline, dried over anhydrous sodium sulfate, filtrated and concentrated by rotary evaporation to dryness. The resulting crude product was purified by column chromatography (eluent: petroleum ether/ethyl acetate=2/1) to obtain (3R,5R,8R,9R,10S,13S,14S,15S)-15-cyclopropyl-3-hydroxy-3,13-dimethylhexadecahydro-17H-cyclopenta[a]phenanthren-17-one (1.11 g, 96%).

¹H NMR (400 MHz, CDCl₃) *δ* 2.45-2.30 (m, 2H), 1.85-1.76 (m, 9H), 1.59-1.52 (m, 5H), 1.59-1.27 (m, 12H), 1.11-0.98 (m, 1H), 0.67-0.64 (m, 1H), 0.47-0.43 (m, 1H), 0.22-0.18 (m, 1H), 0.09-0.07 (m, 1H).

### Step 2: (3R,5R,8R,9R,10S,13S,14S,15S,E)-15-Cyclopropyl-17-ethylidene-3,13-dimethylhexadecahydro-1H-cyclopenta[a]phenanthren-3-ol

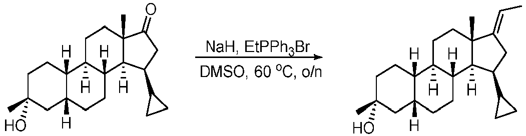

Ethyltriphenylphosphonium bromide (12.5 g, 33 mmol) was dissolved in anhydrous dimethyl sulfoxide (50 mL), and the reaction system was purged with nitrogen. Sodium hydride (1.32 g, 33 mmol) was added, and then the reaction solution was stirred at room temperature for 1 hour. (3R,5R,8R,9R,10S,13S,14S,15S)-15-Cyclopropyl-3-hydroxy-3,13-dimethylhexadecahydro-17H-cyclopenta[a]phenanthren-17-one (1.1 g, 3.3 mmol) was added, and then the reaction solution was stirred at 60°C overnight. The reaction solution was cooled to room temperature. Water (200 mL) was added to the reaction solution to quench the reaction, and the aqueous phase was extracted with ethyl acetate (200 mL×2). The organic phases were combined, dried over anhydrous sodium sulfate, filtrated and concentrated. The resulting crude product was purified by column chromatography (petroleum ether/ethyl acetate: 50/1 ~ 3/1) to obtain (3R,5R,8R,9R,10S,13S,14S,15S,E)-15-cyclopropyl-17-ethylidene-3,13-dimethylhexadecahydro-1H-cyclopenta[a]phenanthren-3-ol (0.67 g, yield: 59%).

¹H NMR (400 MHz, CDCl₃) *δ* 5.15-5.09 (m, 1H), 2.45-2.38 (m, 1H), 2.38-2.18 (m, 2H), 1.90-1.07 (m, 28H), 0.86-0.78 (m, 1H), 0.56-0.50 (m, 1H), 0.38-0.32 (m, 1H), 0.12-0.02 (m, 2H).

Step 3: (3R,5R,8R,9R,10S,13S,14S,15S,17S)-15-Cyclopropyl-17-((R)-1-hydroxyethyl)-3,13-dimethylhexadecahydro-1H-cyclopenta[a]phenanthren-3-ol

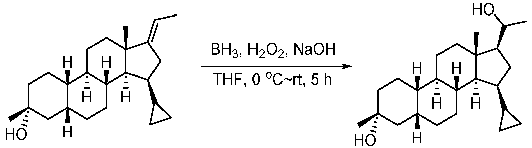

(3R,5R,8R,9R,10S,13S,14S,15S,E)-15-Cyclopropyl-17-ethylidene-3,13-dimethylhexadecahydro-1H-cyclopenta[a]phenanthren-3-ol (0.67 g, 1.96 mmol) was dissolved in anhydrous tetrahydrofuran (20 mL). The solution was cooled to 0°C, and then BH₃/THF (9.8 mL, 9.8 mmol) was added dropwise. The reaction solution was stirred at room temperature for 3 hours, and TLC showed that the reaction was completed. The reaction solution was cooled to 0°C, and then 3 M aqueous NaOH solution (10 mL) was slowly added, followed by the addition of 30% hydrogen peroxide (8 mL). The reaction solution was stirred at room temperature for 2 hours, and TLC showed that the reaction was completed. Ethyl acetate (50 mL) was added, and then the reaction solution was washed with saturated aqueous Na₂S₂O₃ solution (30 mL) and water (30 mL) successively. The organic phase was dried over anhydrous sodium sulfate, filtrated and concentrated to obtain the crude product (0.71 g), which was used directly in the next step.

### Step 4: 1-((3R,5R,8R,9R,10S,13S,14S,15S,17S)-15-Cyclopropyl-3-hydroxy-3,13-dimethylhexadecahydro-1H-cyclopenta[a]phenanthren-17-yl)ethan-1-one

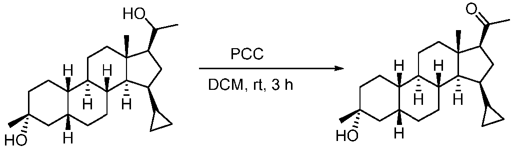

(3R,5R,8R,9R,10S,13S,14S,15S,17S)-15-Cyclopropyl-17-((R)-1-hydroxyethyl)-3,13-dimethylhexadecahydro-1H-cyclopenta[a]phenanthren-3-ol (0.71 g, crude) was dissolved in dichloromethane (20 mL). Pyridinium chlorochromate (1.27 g, 5.88 mmol) was added, and then the reaction solution was stirred at room temperature for 2 hours. The reaction solution was filtrated, and the organic phase was concentrated. The resulting crude product was purified by column chromatography (petroleum ether/ethyl acetate: 1/1) to obtain 1-((3R,5R,8R,9R,10S,13S,14S,15S,17S)-15-cyclopropyl-3-hydroxy-3,13-dimethylhexadecahydro-1H-cyclopenta[a]phenanthren-17-yl)ethan-1-one (480 mg, yield of two steps: 68.3%).

¹H NMR (400 MHz, CDCl₃) δ 2.45-2.40 (m, 1H), 2.18-2.10 (m, 4H), 1.99-1.03 (m, 24H), 0.84-0.79 (m, 4H), 0.60-0.53 (m, 1H), 0.43-0.38 (m, 1H), 0.14-0.02 (m, 2H).

### Step 5: 2-Bromo-1-((3R,5R,8R,9R,10S,13S,14S,15S,17S)-15-cyclopropyl-3-hydroxy-3,13-dimethylhexadecahydro-1H-cyclopenta[a]phenanthren-17-yl)ethan-1-one

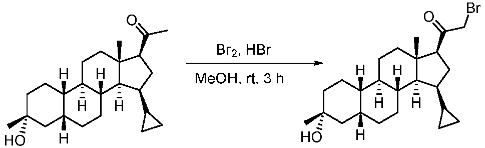

1-((3R,5R,8R,9R,10S,13S,14S,15S,17S)-15-Cyclopropyl-3-hydroxy-3,13-dimethylhexadecahydro-1H-cyclopenta[a]phenanthren-17-yl)ethan-1-one (107 mg, 0.3 mmol) was dissolved in methanol (5 mL). A drop of hydrogen bromide was added to the solution, followed by the addition of liquid bromine (56 mg, 0.35 mmol), and then the reaction solution was stirred at room temperature for 1 hour. Water (20 mL) was added to the reaction solution, and the aqueous phase was extracted with ethyl acetate (20 mL×2). The organic phases were combined, dried over anhydrous sodium sulfate, filtrated and concentrated to obtain the crude product (120 mg, crude), which was used directly in the next step.

### Step 6: 1-(2-((3R,5R,8R,9R,10S,13S,14S,15S)-15-Cyclopropyl-3-hydroxy-3,13-dimethylhexadecahydro-1H-cyclopenta[a]phenanthren-17-yl)-2-oxoethyl)-1H-pyrazole-4-carbonitrile

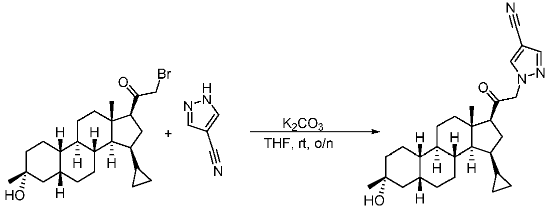

1-((3R,5R,8R,9R,10S,13S,14S,15S,17S)-15-Cyclopropyl-3-hydroxy-3,13-dimethylhexadecahydro-1H-cyclopenta[a]phenanthren-17-yl)ethan-1-one (60 mg, 0.14 mmol), 1H-pyrazole-4-carbonitrile (28 mg, 0.3 mmol) and potassium carbonate (54 mg, 0.3 mmol) were dissolved in anhydrous tetrahydrofuran (5 mL), and the resulting reaction solution was stirred at room temperature overnight. The reaction solution was filtrated, and the filtrate was concentrated. The resulting crude product was purified by high performance liquid chromatography to obtain 1-(2-((3R,5R,8R,9R,10S,13S,14S,15S)-15-cyclopropyl-3-hydroxy-3,13-dimethylhexadecahydro-1H-cyclopenta[a]phenanthren-17-yl)-2-oxoethyl)-1H-pyrazole-4-carbonitrile (31 mg, yield: 49%).

MS m/z (ESI): 450.3 [M+H]⁺
¹H NMR (400 MHz, CDCl₃) δ 7.86 (s, 1H), 7.81 (s, 1H), 5.02 (d, *J* = 16.0 Hz, 1H), 4.92 (d, *J=* 16.0 Hz, 1H), 2.51-2.47 (m, 1H), 2.09-1.71 (m, 9H), 1.48-1.10 (m, 16H), 0.90 (s, 3H), 0.83-0.79 (m, 1H), 0.62-0.58 (m, 1H), 0.45-0.40 (m, 1H), 0.14-0.11 (m, 1H), 0.05-0.02 (m, 1H).

### Example 49

### 1-(2-((3R,5R,8R,9R,10S,13S,14S,15S,17S)-15-Cyclopropyl-3-hydroxy-3,13-dimethylhexadecahydro-1H-cyclopenta[a]phenanthren-17-yl)-2-oxoethyl)-1H-pyrazole-3-carbonitrile

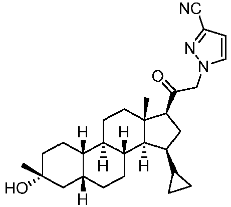

### Step 1: Preparation of 1-(2-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-oxoethyl)-1H-pyrazole-3-carbonitrile

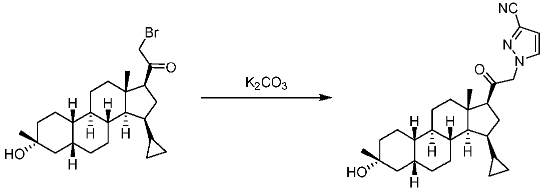

In accordance with Example 3, 1-((3R,5R,8R,9R,10S,13S,14S,15S,17S)-15-cyclopropyl-3-hydroxy-3,13-dimethylhexadecahydro-1H-cyclopenta[a]phenanthren-17-yl)ethan-1-one was used as the starting material, accordingly, 1-(2-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-oxoethyl)-1H-pyrazole-3-carbonitrile (20 mg, yield 20%) was obtained .

MS m/z (ESI): 432.2[M-H₂O +H]⁺
¹H NMR (400 MHz, CDCl₃) δ 7.49 (d, *J* = 2.4 Hz, 1H), 6.73 (d, *J* = 2.4 Hz, 1H), 4.98 (dd, *J* = 40.0, 17.8 Hz, 2H), 2.52 - 2.45 (m, 1H), 2.24 - 2.13 (m, 1H), 2.11 - 2.03 (m, 1H), 2.04 - 1.95 (m, 2H), 1.90 - 1.82 (m, 2H), 1.75 - 1.66 (m, 2H), 1.54 - 1.23 (m, 16H), 1.18 - 1.05 (m, 2H), 0.93 - 0.79 (m, 4H), 0.63 - 0.55 (m, 1H), 0.45 - 0.37 (m, 1H), 0.18 - 0.09 (m, 1H), 0.08 - 0.01 (m, 1H).

### Example 50 and Example 51

### 1-((3R,5R,8R,9R,10S,13S,14S,15S,17S)-15-Cyclopropyl-3-hydroxy-3,13-dimethylhexadecahydro-1H-cyclopenta[a]phenanthren-17-yl)-2-(2H-1,2,3-triazol-2-yl)ethan-1-one (50)

### 1-((3R,5R,8R,9R,10S,13S,14S,15S,17S)-15-Cyclopropyl-3-hydroxy-3,13-dimethylhexadecahydro-1H-cyclopenta[a]phenanthren-17-yl)-2-(1H-1,2,3-triazol-1-yl)ethan-1-one (51)

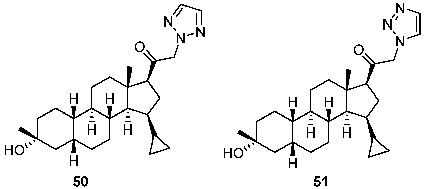

### Step 1: Preparation of 1-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-(2H-1,2,3-triazol-2-yl)ethan-1-one and 1-((3R,5R,8R,9R,10S,13S,14S,15S,17S)-15-Cyclopropyl-3-hydroxy-3,13-dimethylhexadecahydro-1H-cyclopenta[a]phenanthren-17-yl)-2-(1H-1,2,3-triazol-1-yl)ethan-1-one

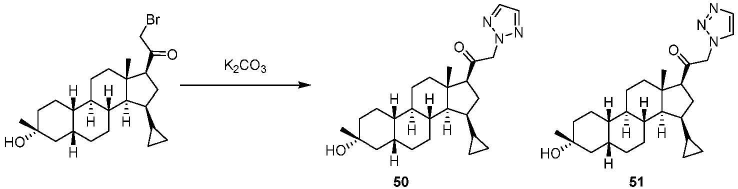

In accordance with Example 3, 1-((3R,5R,8R,9R,10S,13S,14S,15S,17S)-15-cyclopropyl-3-hydroxy-3,13-dimethylhexadecahydro-1H-cyclopenta[a]phenanthren-17-yl)ethan-1-one was used as the starting material, accordingly, 1-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-(2H-1,2,3-triazol-2-yl)ethan-1-one (7.6 mg, yield: 7.8%) and 1-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-(1H-1,2,3-triazol-1-yl)ethan-1-one (11.6 mg, yield: 11.9%) were obtained.

### Example 50:

MS m/z (ESI): 408.3[M-H₂O +H]⁺
¹H NMR (400 MHz, CDCl₃) δ 7.68 (s, 2H), 5.24 (s, 2H), 2.49 - 2.42 (m, 1H), 2.23 - 2.14 (m, 1H), 2.10 - 1.94 (m, 3H), 1.91 - 1.81 (m, 3H), 1.75 - 1.66 (m, 2H), 1.48 - 1.24 (m, 15H), 1.17 - 1.04 (m, 2H), 0.94 (s, 3H), 0.86 - 0.76 (m, 1H), 0.63 - 0.54 (m, 1H), 0.45 - 0.38 (m, 1H), 0.14 - 0.01 (m, 2H).

### Example 51:

MS m/z (ESI): 426.3[M +H]⁺
¹H NMR (400 MHz, CDCl₃) δ 7.78 (s, 1H), 7.67 (s, 1H), 5.22 (dd, *J* = 47.1, 17.8 Hz, 2H), 2.59 - 2.50 (m, 1H), 2.25 - 2.15 (m, 1H), 2.14 - 1.98 (m, 2H), 1.96 - 1.79 (m, 3H), 1.76 - 1.58 (m, 6H), 1.56 - 1.25 (m, 12H), 1.22 - 1.04 (m, 2H), 0.91 (s, 3H), 0.87 - 0.77 (m, 1H), 0.62 - 0.55 (m, 1H), 0.47 - 0.36 (m, 1H), 0.18 - 0.02 (m, 2H).

### Example 52

### 1-((3R,5R,8R,9R,10S,13S,14S,15S,17S)-15-Cyclopropyl-3-hydroxy-3,13-dimethylhexadecahydro-1H-cyclopenta[a]phenanthren-17-yl)-2-(5-methyl-2H-tetrazol-2-yl)ethan-1-one

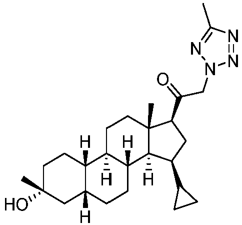

### Step 1: Preparation of 1-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-(5-methyl-2H-tetrazol-2-yl)ethan-1-one

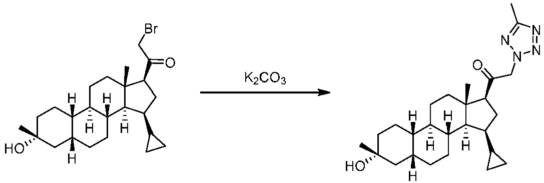

In accordance with Example 3, 1-((3R,5R,8R,9R,10S,13S,14S,15S,17S)-15-cyclopropyl-3-hydroxy-3,13-dimethylhexadecahydro-1H-cyclopenta[a]phenanthren-17-yl)ethan-1-one was used as the starting material, accordingly, 1-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-(5-methyl-2H-tetrazol-2-yl)ethan-1-one (8.5 mg, yield: 9%) was obtained.

MS m/z (ESI): 441.3[M +H]⁺
¹H NMR (400 MHz, CDCl₃) δ 5.36 (s, 2H), 2.57 (s, 3H), 2.54 - 2.47 (m, 1H), 2.25 - 2.15 (m, 1H), 2.13 - 1.95 (m, 3H), 1.92 - 1.79 (m, 3H), 1.77 - 1.66 (m, 2H), 1.50 - 1.24 (m, 15H), 1.18 - 1.05 (m, 2H), 0.94 (s, 3H), 0.87 - 0.78 (m, 1H), 0.63 - 0.55 (m, 1H), 0.45 - 0.37 (m, 1H), 0.17 - 0.02 (m, 2H).

### Example 59

### 1-(2-(3R,5R,8R,9R,10S,13S,14S,15R,17S)-15-Ethyl-3-hydroxy-3,13-dimethylhexadecahydro-1H-cyclopenta[a]phenanthren-17-yl)-2-oxoethyl)-1H-pyrazole-4-carbonitrile

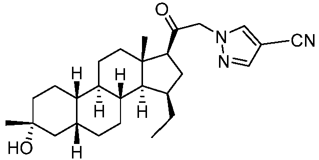

### Step 1: (3R,5R,8R,9R,10S,13S,14S,15R)-15-Ethyl-3-hydroxy-3,13-dimethylhexadecahydro-17H-cyclopenta[a]phenanthren-17-one

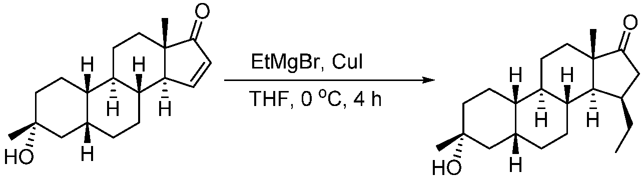

Tetrahydrofuran (15 mL) was added to a 100 mL three-neck flask, and ethyl magnesium bromide (10 mL, 1M, 10 mmol) was then added at 0°C under a nitrogen atmosphere, followed by the addition of cuprous iodide (1.6 g, 8.4 mmol). The reaction solution was stirred at 0°C for 1 hour. (3R,5R,8R,9R,10S,13S,14S)-3-Hydroxy-3,13-dimethyl-1,2,3,4,5,6,7,8,9,10,11,12,13,14-tetradecahydro-17H-cyclopenta[a]phenanthren-17-one (800 mg, 2.8 mmol) was dissolved in tetrahydrofuran (5 mL), and the resulting solution was slowly added dropwise to the reaction solution, which was then stirred at 0°C for 4 hours. Saturated aqueous ammonium chloride solution was added to quench the reaction, and the reaction solution was extracted with ethyl acetate (20 mL). The organic phase was washed with saline (10 mL×3), dried over anhydrous sodium sulfate, filtrated and concentrated by rotary evaporation to dryness. The resulting crude product was purified by column chromatography (petroleum ether/ethyl acetate: 3/1) to obtain (3R,5R,8R,9R,10S,13S,14S,15R)-15-ethyl-3-hydroxy-3,13-dimethylhexadecahydro-17H-cyclopenta[a]phenanthren-17-one (750 mg, yield: 84.9%).

¹H NMR (400 MHz, CDCl₃) δ 2.37 - 2.30 (m, 1H), 2.15 - 2.02 (m, 2H), 1.91 - 1.31 (m, 17H), 1.27 (s, 3H), 1.24 - 1.03 (m, 4H), 0.98 (s, 3H), 0.90 (t, *J* = 7.3 Hz, 3H).

### Step 2: (3R,5R,8R,9R,10S,13S,14S,15R)-15-Ethyl-17-ethylidene-3,13-dimethylhexadecahydro-1H-cyclopenta[a]phenanthren-3-ol

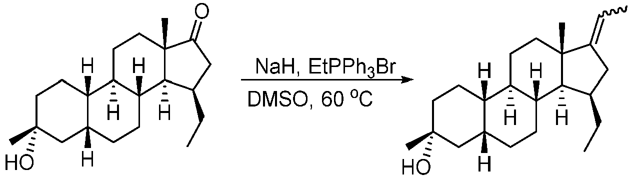

Dimethyl sulfoxide (20 mL) was added to a 100 mL three-neck flask, and ethyltriphenylphosphonium bromide (8.7 g, 23.5 mmol) was then added under a nitrogen atmosphere. Sodium hydride (60%) (940 mg, 23.5 mmol) was added in batches, and the reaction solution was stirred at room temperature for 1 hour. (3R,5R,8R,9R,10S,13S,14S,15R)-15-Ethyl-3-hydroxy-3,13-dimethylhexadecahydro-17H-cyclopenta[a]phenanthren-17-one (750 mg, 2.35 mmol) was dissolved in dimethyl sulfoxide (5 mL), and the resulting solution was slowly added dropwise to the reaction solution, which was then stirred under a nitrogen atmosphere at 80°C for 5 hours. The reaction solution was cooled to room temperature. Saturated aqueous ammonium chloride solution was added to quench the reaction, and the reaction solution was extracted with ethyl acetate (50 mL). The organic phase was washed with saline (20 mL×3), dried over anhydrous sodium sulfate, filtrated and concentrated by rotary evaporation to dryness. The resulting crude product was purified by column chromatography (petroleum ether/ethyl acetate: 3/1) to obtain (3R,5R,8R,9R,10S,13S,14S,15R)-15-ethyl-17-ethylidene-3,13-dimethylhexadecahydro-1H-cyclopenta[a]phenanthren-3-ol (580 mg, yield: 71.7%).

¹H NMR (400 MHz, CDCl₃) δ 5.22 - 5.06 (m, 1H), 2.54 - 2.39 (m, 1H), 2.28 - 2.09 (m, 2H), 1.96 - 1.80 (m, 4H), 1.78 - 1.59 (m, 6H), 1.55 - 1.33 (m, 11H), 1.26 (s, 3H), 1.20 - 1.08 (m, 3H), 1.05 (s, 3H), 0.82 (t, *J* = 7.3 Hz, 3H).

### Step 3: (3R,5R,8R,9R,10S,13S,14S,15R,17R)-15-Ethyl-17-(1-hydroxyethyl)-3,13-dimethylhexadecahydro-1H-cyclopenta[a]phenanthren-3-ol

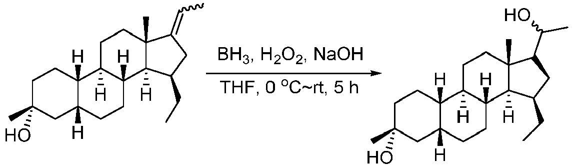

In accordance with Step 3 of Example 71, (3R,5R,8R,9R,10S,13S,14S,15R)-15-ethyl-17-ethylidene-3,13-dimethylhexadecahydro-1H-cyclopenta[a]phenanthren-3-ol was used as the starting material, accordingly, (3R,5R,8R,9R,10S,13S,14S,15R,17R)-15-ethyl-17-(1-hydroxyethyl)-3,13-dimethylhexadecahydro-1H-cyclopenta[a]phenanthren-3-ol (600 mg, yield: 98.1%) was obtained.

### Step 4: 1-((3R,5R,8R,9R,10S,13S,14S,15R,17S)-15-Ethyl-3-hydroxy-3,13-dimethylhexadecahydro-1H-cyclopenta[a]phenanthren-17-yl)ethan-1-one

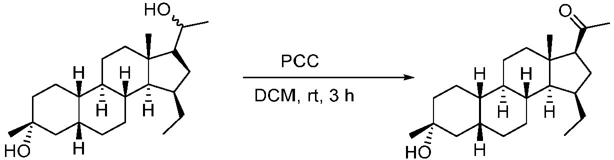

In accordance with Step 4 of Example 71, (3R,5R,8R,9R,10S,13S,14S,15R,17R)-15-ethyl-17-(1-hydroxyethyl)-3,13-dimethylhexadecahydro-1H-cyclopenta[a]phenanthren-3-ol was used as the starting material, accordingly, 1-((3R,5R,8R,9R,10S,13S,14S,15R,17S)-15-ethyl-3-hydroxy-3,13-dimethylhexadecahydro-1H-cyclopenta[a]phenanthren-17-yl)ethan-1-one (500 mg, yield: 83.8%) was obtained.

1H NMR (400 MHz, CDCl₃) δ 2.51 (t, *J* = 8.0 Hz, 1H), 2.12 (s, 3H), 2.00 - 1.30 (m, 20H), 1.28 (s, 3H), 1.25 - 1.00 (m, 4H), 0.84 (t, *J* = 7.2 Hz, 3H), 0.73 (s, 3H).

### Step 5: 2-Bromo-1-((3R,5R,8R,9R,10S,13S,14S,15R,17S)-15-ethyl-3-hydroxy-3,13-dimethylhexadecahydro-1H-cyclopenta[a]phenanthren-17-yl)ethan-1-one

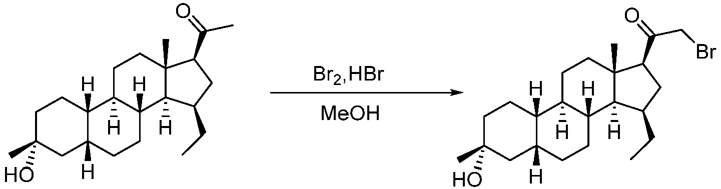

In accordance with Step 5 of Example 71, 1-((3R,5R,8R,9R,10S,13S,14S,15R,17S)-15-ethyl-3-hydroxy-3,13-dimethylhexadecahydro-1H-cyclopenta[a]phenanthren-17-yl)ethan-1-one was used as the starting material, accordingly, 2-bromo-1-((3R,5R,8R,9R,10S,13S,14S,15R,17S)-15-ethyl-3-hydroxy-3,13-dimethylhexadecahydro-1H-cyclopenta[a]phenanthren-17-yl)ethan-1-one (500 mg, yield: 81.4%) was obtained.

### Step 6: 1-(2-((3R,5R,8R,9R,10S,13S,14S,15R,17S)-15-Ethyl-3-hydroxy-3,13-dimethylhexadecahydro-1H-cyclopenta[a]phenanthren-17-yl)-2-oxoethyl)-1H-pyrazole-4-carbonitrile

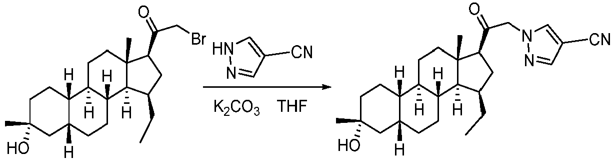

In accordance with Step 6 of Example 71, 2-bromo-1-((3R,5R,8R,9R,10S,13S,14S,15R,17S)-15-ethyl-3-hydroxy-3,13-dimethylhexadecahydro-1H-cyclopenta[a]phenanthren-17-yl)ethan-1-one was used as the starting material, accordingly, 1-(2-((3R,5R,8R,9R,10S,13S,14S,15R,17S)-15-ethyl-3-hydroxy-3,13-dimethylhexadecahydro-1H-cyclopenta[a]phenanthren-17-yl)-2-oxoethyl)-1H-pyrazole-4-carbonitrile (24 mg, yield: 38.8%) was obtained.

MS m/z (ESI): 438.2[M+H]+.

¹H NMR (400 MHz, CDCl₃) δ 7.86 (s, 1H), 7.81 (s, 1H), 5.08 - 4.78 (m, 2H), 2.58 (t, *J* = 8.0 Hz, 1H), 2.07 - 1.29 (m, 20H), 1.28 (s, 3H), 1.27 - 1.06 (m, 4H), 0.85 (t, *J* = 7.3 Hz, 3H), 0.79 (s, 3H).

### Example 68

### 2-(4-Chloro-1H-pyrazol-1-yl)-1-((3R,5R,8R,9R,10S,13S,14S,15R,17S)-3-hydroxy-3,13,15-trimethylhexadecahydro-1H-cyclopenta[a]phenanthren-17-yl)ethan-1-one

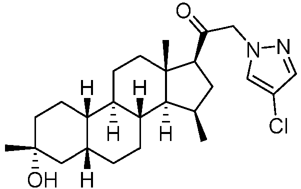

### Step 1: Preparation of 2-(4-chloro-1H-pyrazol-1-yl)-1-((3R,5R,8R,9R,10S,13S,14S,15R,17S)-3-hydroxy-3,13,15-trimethylhexadecahydro-1H-cyclopenta[a]phenanthren-17-yl)ethan-1-one

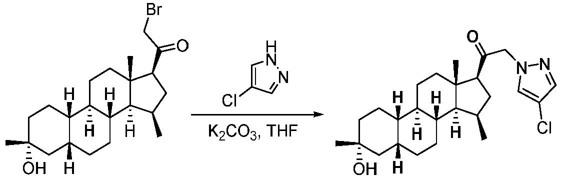

In accordance with Example 5, 2-bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one was used as the starting material, accordingly, 2-(4-chloro-1H-pyrazol-1-yl)-1-((3R,5R,8R,9R,10S,13S,145,15R,17S)-3-hydroxy-3,13,15-trimethylhexadecahydro-1H-cyclopenta[a]phenanthren-17-yl)ethan-1-one (10 mg, yield: 14%) was obtained.

MS m/z (ESI): 433.3[M+H]⁺
¹H NMR (400 MHz, CDCl₃) δ 7.45 (s, 1H), 7.41 (s, 1H), 4.86 (q, *J* = 17.9 Hz, 2H), 2.61 - 2.44 (m, 1H), 2.30 - 2.07 (m, 2H), 2.02 - 1.91 (m, 1H), 1.86 - 1.80 (m, 4H), 1.76 - 1.57 (m, 4H), 1.51 - 1.38 (m, 6H), 1.37 - 1.20 (m, 6H), 1.20 - 1.03 (m, 2H), 0.98 (d, *J* = 7.1 Hz, 3H), 0.83 (s, 3H).

### Example 69

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(4-methyl-1H-pyrazol-1-yl)ethan-1-one

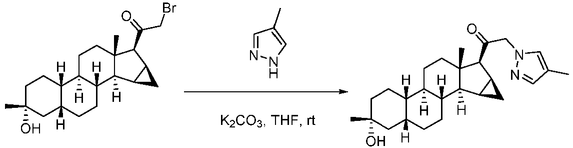

In accordance with Example 5, 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one and 4-methylpyrazole were used as the starting materials, accordingly, 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-methyl-1H-pyrazol-1-yl)ethan-1-one (12.6 mg, white solid, yield: 15.7%) was obtained.

MS m/z (ESI): 411.3[M+H]⁺
¹H NMR (400 MHz, CDCl₃) δ 7.44 (s, 1H), 7.26 (s, 1H), 5.23( m, 2H), 2.83 (d, *J* = 4.4 Hz, 1H), 2.13 (s, 3H), 1.98-1.85 (m, 2H), 1.83-1.69 (m, 9H), 1.60-1.56 (m, 1H), 1.40-1.27 (m, 12H), 1.09-1.01 (m, 2H), 0.80 (s, 3H), 0.48-0.45 (m , 1H).

### Example 70

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(1H-imidazol-1-yl)ethan-1-one

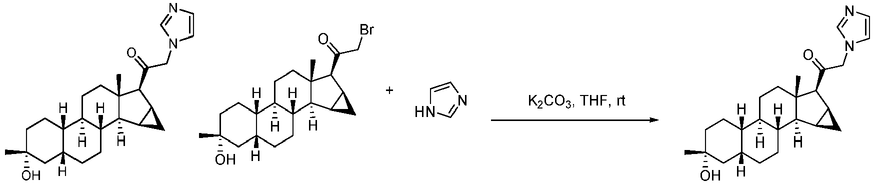

In accordance with Example 63, 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one and imidazole were used as the starting materials, accordingly, 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(1H-imidazol-1-yl)ethan-1-one (23.3 mg, white solid, yield: 34.4%) was obtained.

MS m/z (ESI): 397.2[M+H]⁺
¹H NMR (400 MHz, CDCl₃) δ 7.48 (s, 1H), 7.12 (s, 1H), 6.88 (s, 1H), 4.98-4.87 (m, 2H), 2.82 (d, *J* = 2.8 Hz, 1H), 1.94-1.91(m, 1H), 1.85-1.81( m, 4H), 1.70-1.53 (m, 10H), 1.41-1.28 (m, 9H), 1.13-0.95 (m, 2H), 0.78 (s, 3H), 0.52-0.46 (m, 1H).

### Example 71

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(5-methyl-1H-tetrazol-1-yl)ethan-1-one

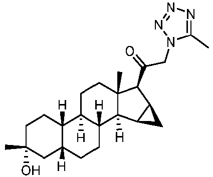

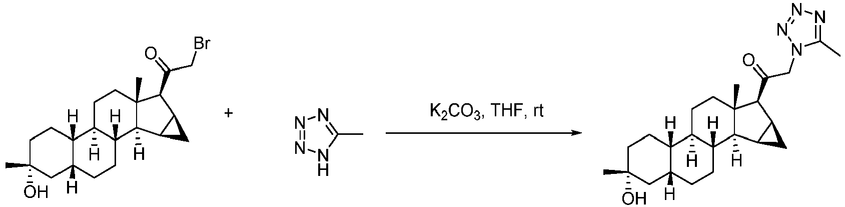

In accordance with Example 5, 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one and 5-methyl-1H-4-tetrazole were used as the starting materials, accordingly, 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(5-methyl-1H-tetrazol-1-yl)ethan-1-one (19 mg, white solid, yield: 23.5%) was obtained.

MS m/z (ESI): 413.2[M+H]⁺
¹H NMR (400 MHz, CDCl₃) δ 5.58 (dd, *J₁ =* 17.6 Hz, *J₂* = 7.6 Hz, 2H), 2.86 (d, *J* = 4.0 Hz, 1H), 2.57 (s, 3H), 1.96-1.93 (m, 1H), 1.89-1.61 (m, 8H), 1.55-1.22 (m, 14H), 1.14-0.97 (m, 3H), 0.84 (s, 3H), 0.47-0.53 (m, 1H).

### Example 72

### 2-(4-(Azetidine-1-carbonyl)-1H-pyrazol-1-yl)-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one

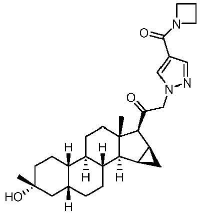

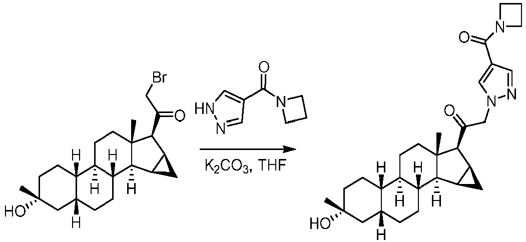

In accordance with Example 5, 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one and azetidin-1-yl(1H-pyrazol-4-yl)methanone were used as the starting materials, accordingly, 2-(4-(azetidine-1-carbonyl)-1H-pyrazol-1-yl)-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one (10.0 mg, yield: 14.3%) was obtained.

MS m/z (ESI): 480.3 [M+H]⁺
¹H NMR (400 MHz, CDCl₃) δ 7.85 - 7.80 (m, 1H), 7.80 - 7.74 (m, 1H), 5.20 - 5.05 (m, 2H), 4.53 - 4.10 (m, 4H), 2.82 (d, *J* = 3.3 Hz, 1H), 2.44 - 2.31 (m, 2H), 1.98 - 1.90 (m, 1H), 1.86 - 1.79 (m, 3H), 1.74 - 1.64 (m, 3H), 1.57 - 1.53 (m, 2H), 1.44 - 1.24 (m, 15H), 1.13 - 0.97 (m, 2H), 0.79 (s, 3H), 0.51 - 0.44 (m, 1H).

### Example 73

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(4-nitro-1H-pyrazol-1-yl)ethan-1-one

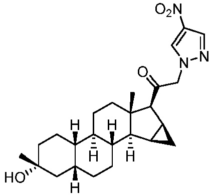

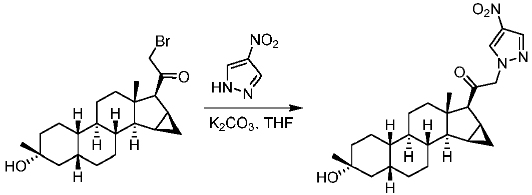

In accordance with Example 5, 2-bromo-1-((2R,4aS,4bR,6aS,7 S,7aS, 8aR, 8bR, 8cR, 10aR)-2-hydroxy-2,6a-dimethyloctadecahydrocyclopropa[4,5]cyclopenta[1,2-a]phenanthren-7-yl)ethan-1-one and 4-nitro-1H-pyrazole were used as the starting materials, accordingly, 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-nitro-1H-pyrazol-1-yl)ethan-1-one (32.5 mg, yield: 60%) was obtained.

MS m/z (ESI): 424.2 [M+H-H₂O]⁺
¹H NMR (400 MHz, CDCl₃) δ8.20 (s, 1H), 8.09 (s, 1H), 5.26 - 5.09 (m, 2H), 2.85 (s, 1H), 1.98 - 1.91 (m, 1H), 1.87 - 1.79 (m, 4H), 1.74 - 1.65 (m, 3H), 1.56 - 1.52 (m, 2H), 1.47 - 1.35 (m, 7H), 1.34 - 1.24 (m, 7H), 1.13 - 0.97 (m, 2H), 0.79 (s, 3H), 0.55 - 0.47 (m, 1H).

### Example 80

### 3-Cyclopropyl-1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carbonitrile

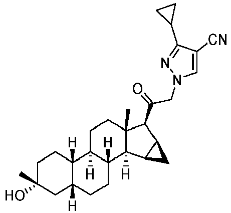

### Step 1: Preparation of 3-cyclopropyl-1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carbonitrile

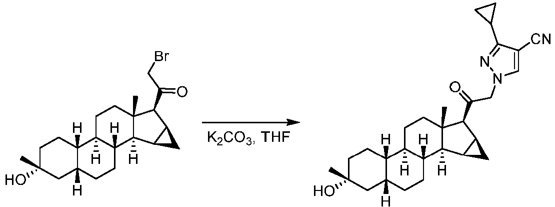

In accordance with Example 5, 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one was used as the starting material, accordingly, 3-cyclopropyl-1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-4-carbonitrile (19.2 mg, yield: 21%) was obtained.

MS m/z (ESI): 462.3 [M+H]⁺
¹H NMR (400 MHz, CDCl₃) δ 7.73 (s, 1H), 5.13 - 4.95 (m, 2H), 2.80 (d, *J* = 2.2 Hz, 1H), 2.04 - 1.89 (m, 2H), 1.86 - 1.79 (m, 3H), 1.76 - 1.63 (m, 3H), 1.58 - 1.49 (m, 3H), 1.46 - 1.21 (m, 14H), 1.12 - 0.93 (m, 6H), 0.76 (s, 3H), 0.51 - 0.43 (m, 1H).

### Example 81

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(4-(methylthio)-1H-pyrazol-1-yl)ethan-1-one

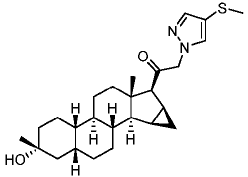

### Step 1: Preparation of 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-(methylthio)-1H-pyrazol-1-yl)ethan-1-one

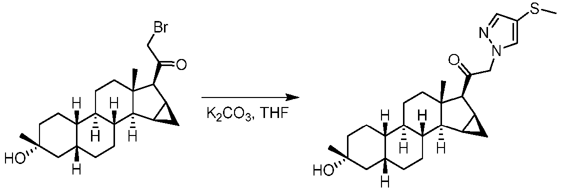

In accordance with Example 5, 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one was used as the starting material, accordingly, 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-(methylthio)-1H-pyrazol-1-yl)ethan-1-one (8.7 mg, yield: 16%) was obtained.

MS m/z (ESI): 443.2 [M+H]⁺
¹H NMR (400 MHz, CDCl₃) δ 7.54 (s, 1H), 7.45 (s, 1H), 5.17 - 5.00 (m, 2H), 2.81 (d, *J* = 3.8 Hz, 1H), 2.35 (s, 3H), 1.99 - 1.91 (m, 1H), 1.88 - 1.76 (m, 4H), 1.73 - 1.63 (m, 3H), 1.55 - 1.50 (m, 1H), 1.43 - 1.18 (m, 15H), 1.12 - 0.97 (m, 2H), 0.79 (s, 3H), 0.50 - 0.41 (m, 1H).

### Example 82 and Example 83

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(4-(methylsulfinyl)-1H-pyrazol-1-yl)ethan-1-one (82)

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(4-(methylsulfonyl)-1H-pyrazol-1-yl)ethan-1-one (83)

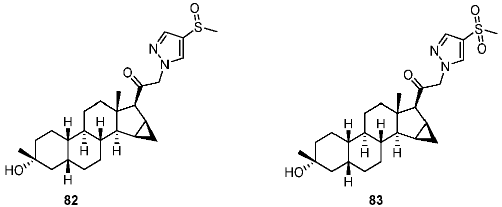

### Step 1: Preparation of 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-(methylsulfinyl)-1H-pyrazol-1-yl)ethan-1-one and 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-(methylsulfonyl)-1H-pyrazol-1-yl)ethan-1-one

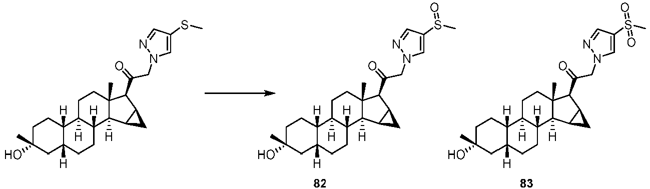

1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(4-(methylthio)-1H-pyrazol-1-yl)ethan-1-one (80 mg, 0.18 mmol) was dissolved in dichloromethane (10 mL), and the solution was cooled to -78°C. m-Chloroperoxybenzoic acid (55 mg, 0.27 mmol) was added, and the reaction solution was stirred for 1 hour. Water (10 mL) was added, and then the reaction solution was washed with saturated aqueous sodium bicarbonate solution (10 mL). The organic phase was separated, dried over anhydrous sodium sulfate, filtrated and concentrated by rotary evaporation to dryness. The resulting crude product was purified by high performance liquid chromatography to obtain 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-(methylsulfinyl)-1H-pyrazol-1-yl)ethan-1-one (17.2 mg, yield: 20%) and 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-(methylsulfonyl)-1H-pyrazol-1-yl)ethan-1-one (20.4 mg, yield: 25%).

### Example 82:

MS m/z (ESI): 441.3 [M-H₂O+H]⁺
¹H NMR (400 MHz, CDCl₃) δ 7.85 (s, 1H), 7.83 (s, 1H), 5.30 - 5.09 (m, 2H), 2.91 (s, 3H), 2.85 - 2.82 (m, 1H), 1.98 - 1.90 (m, 1H), 1.87 - 1.78 (m, 3H), 1.75 - 1.50 (m, 8H), 1.47 - 1.21 (m, 12H), 1.13 - 0.96 (m, 2H), 0.79 (s, 3H), 0.54 - 0.45 (m, 1H).

### Example 83:

MS m/z (ESI): 457.2 [M-H₂O+H]⁺
¹H NMR (400 MHz, CDCl₃) δ 7.94 (s, 1H), 7.87 (s, 1H), 5.26 - 5.10 (m, 2H), 3.14 (s, 3H), 2.85 (d, *J* = 2.7 Hz, 1H), 1.98 - 1.91 (m, 1H), 1.87 - 1.79 (m, 3H), 1.76 - 1.64 (m, 3H), 1.59 - 1.52 (m, 1H), 1.50 - 1.18 (m, 16H), 1.15 - 0.96 (m, 2H), 0.79 (s, 3H), 0.55 - 0.46 (m, 1H).

### Example 84

### 1-(2-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-oxoethyl)-1H-pyrazole-3,5-dicarbonitrile

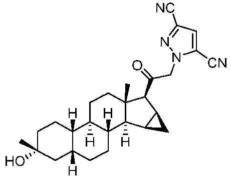

### Step 1: Preparation of 1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-3,5-dicarbonitrile

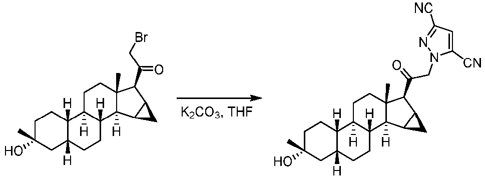

In accordance with Example 5, 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one was used as the starting material, accordingly, 1-(2-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-oxoethyl)-1H-pyrazole-3,5-dicarbonitrile (18.0 mg, yield: 31.8%) was obtained.

MS m/z (ESI): 429.2 [M-H₂O+H]⁺
¹H NMR (400 MHz, CDCl₃) δ 7.21 (s, 1H), 5.38 (q, *J* = 18.0 Hz, 2H), 2.87 (d, *J* = 3.0 Hz, 1H), 1.97 - 1.91 (m, 1H), 1.88 - 1.75 (m, 4H), 1.76 - 1.64 (m, 3H), 1.58 - 1.51 (m, 1H), 1.48 - 1.26 (m, 15H), 1.15 - 0.99 (m, 2H), 0.83 (s, 3H), 0.58 - 0.48 (m, 1H).

### Example 85

### 1-((2R,4aS,4bR,6aS,7S,7aS,8aR,8bR,8cR,10aR)-2-Hydroxy-2,6a-dimethyloctadecahydrocyclopropa[4,5]cyclopenta[1,2-a]phenanthren-7-yl)-2-(4-(oxazol-2-yl)-1H-pyrazol-1-yl)ethan-1-one

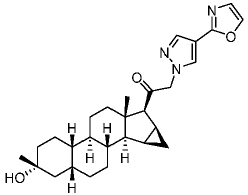

### Step 1: Preparation of 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-(oxazol-2-yl)-1H-pyrazol-1-yl)ethan-1-one

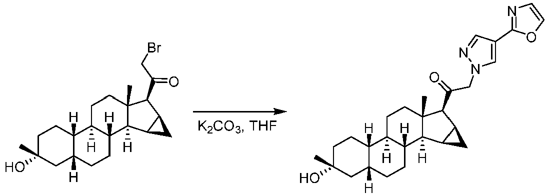

In accordance with Example 5, 2-bromo-1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)ethan-1-one was used as the starting material, accordingly, 1-((2R,4aS,4bR,6aS,7S,7aS,8aR,8bR,8cR,10aR)-2-hydroxy-2,6a-dimethyloctadecahydrocyclopropa[4,5]cyclopenta[1,2-a]phenanthren-7-yl)-2-(4-(oxazol-2-yl)-1H-pyrazol-1-yl)ethan-1-one (16.0 mg, yield: 14%) was obtained.

MS m/z (ESI): 464.3 [M +H]⁺
¹H NMR (400 MHz, CDCl₃) δ 7.89 (s, 1H), 7.79 (s, 1H), 7.73 (s, 1H), 7.13 (s, 1H), 5.27 - 5.13 (m, 2H), 2.85 (d, *J* = 4.0 Hz, 1H), 2.01 - 1.93 (m, 1H), 1.87 - 1.65 (m, 11H), 1.58 - 1.50 (m, 1H), 1.44 - 1.22 (m, 11H), 1.14 - 0.98 (m, 2H), 0.81 (s, 3H), 0.53 - 0.44 (m, 1H).

### Example 86

### 1-((3R,5R,8R,9R,10S,13S,14S,15R,17S)-3-Hydroxy-3,13,15-trimethylhexadecahydro-1H-cyclopenta[a]phenanthren-17-yl)-2-(3 -(trifluoromethyl)-1H-pyrazol-1-yl)ethan-1-one

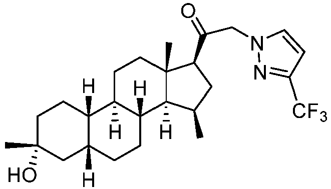

### Step 1: 1-((3R,5R,8R,9R,10S,13S,14S,15R,17S)-3-hydroxy-3,13,15-trimethylhexadecahydro-1H-cyclopenta[a]phenanthren-17-yl)-2-(3-(trifluoromethyl)-1H-pyrazol-1-yl)ethan-1-one

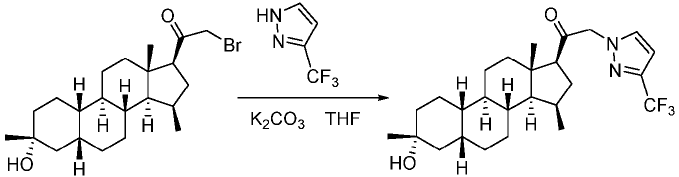

In accordance with Example 5, 2-bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one was used as the starting material, accordingly, 1-((3R,5R,8R,9R,10S,13S,14S,15R,17S)-3-hydroxy-3,13,15-trimethylhexadecahydro-1H-cyclopenta[a]phenanthren-17-yl)-2-(3-(trifluoromethyl)-1H-pyrazol-1-yl)ethan-1-one (20.5 mg, yield: 30.1%) was obtained.

MS m/z (ESI): 467.2[M+H]⁺.

¹H NMR (400 MHz, CDCl₃) δ 7.49 - 7.43 (m, 1H), 6.59 (d, *J* = 2.4 Hz, 1H), 5.14 - 4.85 (m, 2H), 2.54 (t, *J* = 8.1 Hz, 1H), 2.26 - 2.08 (m, 2H), 2.02 - 1.93 (m, 1H), 1.90 - 1.79 (m, 4H), 1.74 -1.59 (m, 3H), 1.57 - 1.19 (m, 14H), 1.21 - 1.04 (m, 1H), 0.98 (d, *J* = 7.0 Hz, 3H), 0.84 (s, 3H).

### Example 87

### 2-(4-(Azetidine-1-carbonyl)-1H-pyrazol-1-yl)-1-((3R,SR,8R,9R,10S,13S,14S,15R,17S)-3-hydroxy-3,13,15-trimethylhexadecahydro-1H-cyclopenta[a]phenanthren-17-yl)ethan-1-one

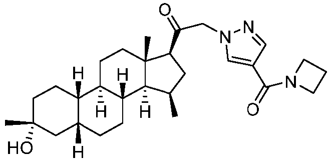

### Step 1: Preparation of 2-(4-(azetidine-1-carbonyl)-1H-pyrazol-1-yl)-1-((3R,5R,8R,9R,10S,13S,14S,15R,17S)-3-hydroxy-3,13,15-trimethylhexadecahydro-1H-cyclopenta[a]phenanthren-17-yl)ethan-1-one

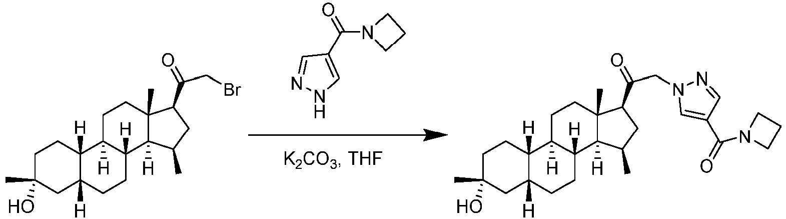

In accordance with Example 5, 2-bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one was used as the starting material, accordingly, 2-(4-(azetidine-1-carbonyl)-1H-pyrazol-1-yl)-1-((3R,5R,8R,9R,10S,13S,14S,15R,17S)-3-hydroxy-3,13,15-trimethylhexadecahydro-1H-cyclopenta[a]phenanthren-17-yl)ethan-1-one (12.0 mg, yield: 21%) was obtained.

MS m/z (ESI): 482.3[M+H]⁺.

¹H NMR (400 MHz, CDCl₃) δ 7.78 (s, 2H), 5.00 - 4.77 (m, 2H), 4.38-4.25 (m, 4H), 2.54 (t, *J =* 7.6 Hz, 1H), 2.43 - 2.32 (m, 2H), 2.26 - 2.08 (m, 2H), 1.98-1.96 (m, 1H), 1.88-1.83 (m, 3H), 1.69-1.62(m, 5H), 1.48-1.42 (m, 4H), 1.38-1.26 (m, 9H), 1.16-1.07 (m, 2H), 0.98 (d, *J* = 7.0 Hz, 3H), 0.84 (s, 3H).

### Example 88

### 3-Chloro-1-(2-((3R,5R,8R,9R,10S,13S,14S,15R,17S)-3-hydroxy-3,13,15-trimethylhexadecahydro-1H-cyclopenta[a]phenanthren-17-yl)-2-oxoethyl)-1H-pyrazole-4-carbonitrile

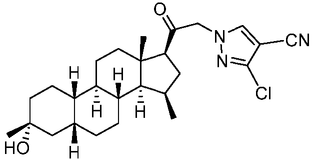

### Step 1: Preparation of 3-chloro-1-(2-((3R,5R,8R,9R,10S,13S,14S,15R,17S)-3-hydroxy-3,13,15-trimethylhexadecahydro-1H-cyclopenta[a]phenanthren-17-yl)-2-oxoethyl)-1H-pyrazole-4-carbonitrile

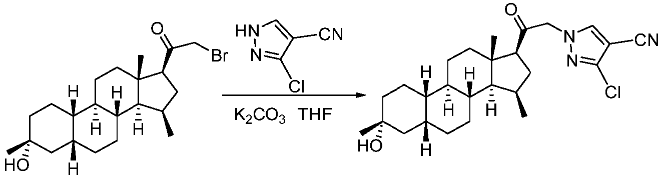

In accordance with Example 5, 2-bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one was used as the starting material, accordingly, the product 3-chloro-1-(2-((3R,5R,8R,9R,10S,13S,14S,15R,17S)-3-hydroxy-3,13,15-trimethylhexadecahydro-1H-cyclopenta[a]phenanthren-17-yl)-2-oxoethyl)-1H-pyrazole-4-carbonitrile (37 mg, yield: 55.3%) was obtained.

MS m/z (ESI): 456.2[M-H]-.

¹H NMR (400 MHz, CDCl₃) δ 7.81 (s, 1H), 5.03 - 4.75 (m, 2H), 2.54 (t, *J* = 8.0 Hz, 1H), 2.31 - 2.13 (m, 2H), 1.93 - 1.06 (m, 23H), 0.99 (d, *J* = 7.1 Hz, 3H), 0.83 (s, 3H).

### Example 89

### 1-(2-((3R,5R,8R,9R,10S,13S,14S,15R,17S)-3-Hydroxy-3,13,15-trimethylhexadecahydro-1H-cyclopenta[a]phenanthren-17-yl)-2-oxoethyl)-1H-pyrazole-3,5-dicarbonitrile

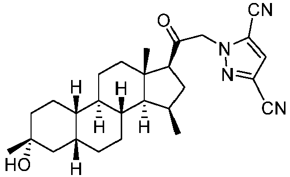

### Step 1: Preparation of 1-(2-((3R,5R,8R,9R,10S,13S,14S,15R,17S)-3-hydroxy-3,13,15-trimethylhexadecahydro-1H-cyclopenta[a]phenanthren-17-yl)-2-oxoethyl)-1H-pyrazole-3,5-dicarbonitrile

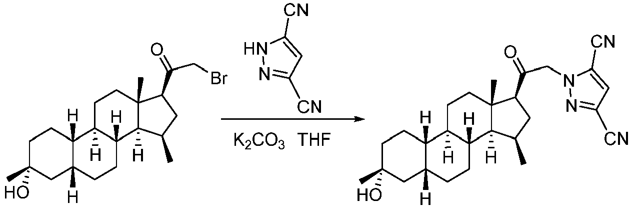

In accordance with Example 5, 2-bromo-1-((3R,5R,8R,9R,10S,13S,14S,15R,17S)-3-hydroxy-3,13,15-trimethylhexadecahydro-1H-cyclopenta[a]phenanthren-17-yl)ethan-1-one was used as the starting material, accordingly, the product 1-(2-((3R,5R,8R,9R,10S,13S,14S,15R,17S)-3-hydroxy-3,13,15-trimethylhexadecahydro-1H-cyclopenta[a]phenanthren-17-yl)-2-oxoethyl)-1H-pyrazole-3,5-dicarbonitrile (18 mg, yield 27.5%) was obtained.

MS m/z (ESI): 447.2[M-H]⁻.

¹H NMR (400 MHz, CDCl₃) δ7.20 (s, 1H), 5.23 - 5.07 (m, 2H), 2.68 - 2.52 (m, 1H), 2.28 - 1.36 (m, 21H), 1.28 (s, 3H), 1.18 - 1.08 (m, 1H), 1.00 (d, *J=* 6.9 Hz, 3H), 0.88 (s, 3H).

### Example 90

### 1-(2-(3R,5R,8R,9R,10S,13S,14S,15R,17S)-15-Ethyl-3-hydroxy-3,13-dimethylhexadecahydro-1H-cyclopenta[a]phenanthren-17-yl)-2-oxoethyl)-1H-pyrazole-3-carbonitrile

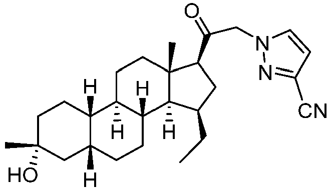

### Step 1: Preparation of 1-(2-((3R,5R,8R,9R,10S,13S,14S,15R,17S)-15-ethyl-3-hydroxy-3,13-dimethylhexadecahydro-1H-cyclopenta[a]phenanthren-17-yl)-2-oxoethyl)-1H-pyrazole-3-carbonitrile

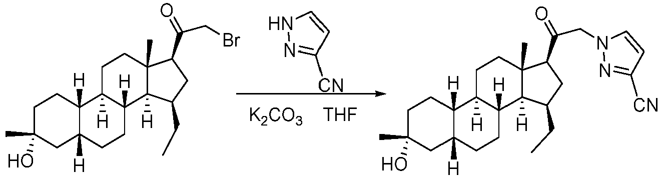

In accordance with Example 5, 2-bromo-1-((3R,5R,8R,9R,10S,13S,14S,15R,17S)-15-ethyl-3-hydroxy-3,13-dimethylhexadecahydro-1H-cyclopenta[a]phenanthren-17-yl)ethan-1-one was used as the starting material, accordingly, the product 1-(2-((3R,5R,8R,9R,10S,13S,14S,15R,17S)-15-ethyl-3-hydroxy-3,13-dimethylhexadecahydro-1H-cyclopenta[a]phenanthren-17-yl)-2-oxoethyl)-1H-pyrazole-3-carbonitrile (16 mg, yield: 25.9%) was obtained.

MS m/z (ESI): 438.2[M+H]⁺.

¹H NMR (400 MHz, CDCl₃) δ 7.49 (d, *J* = 2.5 Hz, 1H), 6.73 (d, *J* = 2.4 Hz, 1H), 5.22 - 4.78 (m, 2H), 2.57 (t, *J* = 8.0 Hz, 1H), 2.15 - 1.30 (m, 20H), 1.28 (s, 3H), 1.16 - 1.03 (m, 4H), 0.84 (t, *J=* 7.3 Hz, 3H), 0.79 (s, 3H).

### Example 91

### 1-((3R,5R,8R,9R,10S,13S,14S,15R,17S)-15-Ethyl-3-hydroxy-3,13-dimethylhexadecahydro-1H-cyclopenta[a]phenanthren-17-yl)-2-(3-(trifluoromethyl)-1H-pyrazol-1-yl)ethan-1-one

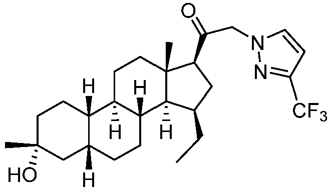

### Step 1: Preparation of 1-((3R,5R,8R,9R,10S,13S,14S,15R,17S)-15-Ethyl-3-hydroxy-3,13-dimethylhexadecahydro-1H-cyclopenta[a]phenanthren-17-yl)-2-(3-(trifluoromethyl)-1H-pyrazol-1-yl)ethan-1-one

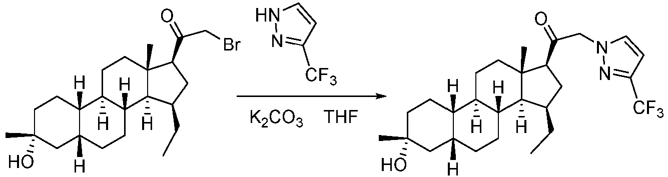

In accordance with Example 5, 2-bromo-1-((3R,5R,8R,9R,10S,13S,14S,15R,17S)-15-ethyl-3-hydroxy-3,13-dimethylhexadecahydro-1H-cyclopenta[a]phenanthren-17-yl)ethan-1-one was used as the starting material, accordingly, the product 1-((3R,5R,8R,9R,10S,13S,14S,15R,17S)-15-ethyl-3-hydroxy-3,13-dimethylhexadecahydro-1H-cyclopenta[a]phenanthren-17-yl)-2-(3-(trifluoromethyl)-1H-pyrazol-1-yl)ethan-1-one (23 mg, yield: 33.9%) was obtained.

MS m/z (ESI): 481.3[M+H]⁺.

¹H NMR (400 MHz, CDCl₃) δ 7.47 (d, *J* =4.0Hz, 1H), 6.59 (d, *J* = 4.0 Hz 1H), 5.07 - 4.89 (m, 2H), 2.56 (t, *J* = 9.3 Hz, 1H), 2.20 - 1.29 (m, 20H),1.28 (s, 3H), 1.27 - 1.03 (m, 4H),0.84 (t, *J* = 7.3 Hz, 3H), 0.80 (s, 3H).

### Example 92

### 1-((3R,5R,8R,9R,10S,13S,14S,15R,17S)-15-Ethyl-3-hydroxy-3,13-dimethylhexadecahydro-1H-cyclopenta[a]phenanthren-17-yl)-2-(5-methyl-2H-tetrazol-2-yl)ethan-1-one

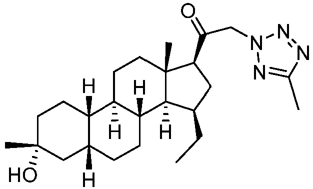

### Step 1: Preparation of 1-((3R,5R,8R,9R,10S,13S,14S,15R,17S)-15-ethyl-3-hydroxy-3,13-dimethylhexadecahydro-1H-cyclopenta[a]phenanthren-17-yl)-2-(5-methyl-2H-tetrazol-2-yl)ethan-1-one

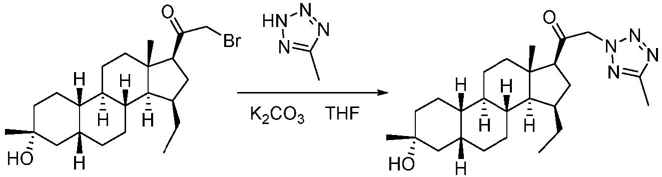

In accordance with Example 5, 2-bromo-1-((3R,5R,8R,9R,10S,13S,14S,15R,17S)-15-ethyl-3-hydroxy-3,13-dimethylhexadecahydro-1H-cyclopenta[a]phenanthren-17-yl)ethan-1-one was used as the starting material, accordingly, the product 1-((3R,5R,8R,9R,10S,13S,14S,15R,17S)-15-ethyl-3-hydroxy-3,13-dimethylhexadecahydro-1H-cyclopenta[a]phenanthren-17-yl)-2-(5-methyl-2H-tetrazol-2-yl)ethan-1-one (13.5 mg, yield: 22.3%) was obtained.

MS m/z (ESI): 429.3[M+H]⁺.

¹H NMR (400 MHz, CDCl₃) δ 5.39 - 5.29 (m, 2H), 2.63 - 2.57 (m, 1H), 2.56 (s, 3H), 2.10 - 1.30 (m, 20H), 1.28 (s, 3H), 1.27 - 1.05 (m, 4H), 0.87 - 0.81 (m, 6H).

### Example 93 and Example 94

### 1-((3R,5R,8R,9R,10S,13S,14S,15R,17S)-15-Ethyl-3-hydroxy-3,13-dimethylhexadecahydro-1H-cyclopenta[a]phenanthren-17-yl)-2-(1H-1,2,3-triazol-1-yl)ethan-1-one (93)

### 1-((3R,5R,8R,9R,10S,13S,14S,15R,17S)-15-Ethyl-3-hydroxy-3,13-dimethylhexadecahydro-1H-cyclopenta[a]phenanthren-17-yl)-2-(2H-1,2,3-triazol-2-yl)ethan-1-one (94)

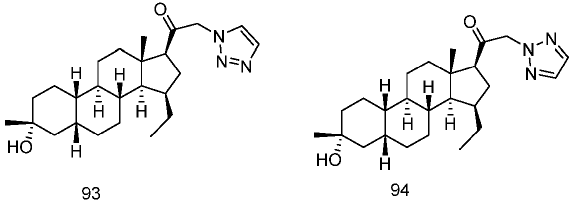

### Step 1: Preparation of 1-((3R,5R,8R,9R,10S,13S,14S,15R,17S)-15-ethyl-3-hydroxy-3,13-dimethylhexadecahydro-1H-cyclopenta[a]phenanthren-17-yl)-2-(1H-1,2,3-triazol-1-yl)ethan-1-one and 1-((3R,5R,8R,9R,10S,13S,14S,15R,17S)-15-ethyl-3-hydroxy-3,13-dimethylhexadecahydro-1H-cyclopenta[a]phenanthren-17-yl)-2-(2H-1,2,3-triazol-2-yl)ethan-1-one

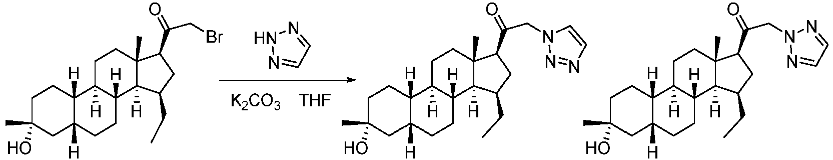

In accordance with Example 5, 2-bromo-1-((3R,5R,8R,9R,10S,13S,14S,15R,17S)-15-ethyl-3-hydroxy-3,13-dimethylhexadecahydro-1H-cyclopenta [a]phenanthren-17-yl)ethan-1-one was used as the starting material, accordingly, the products 1-((3R,5R,8R,9R,10S,13S,14S,15R,17S)-15-ethyl-3-hydroxy-3,13-dimethylhexadecahydro-1H-cyclopenta[a]phenanthren-17-yl)-2-(1H-1,2,3-triazol-1-yl)ethan-1-one (17 mg, yield: 29.1%) and 1-((3R,5R,8R,9R,10S,13S,14S,15R,17S)-15-ethyl-3-hydroxy-3,13-dimethylhexadecahydro-1H-cyclopenta[a]phenanthren-17-yl)-2-(2H-1,2,3-triazol-2-yl)ethan-1-one (9.2 mg, yield: 15.7%) were obtained.

### Example 93:

MS m/z (ESI): 414.3[M+H]⁺.

¹H NMR (400 MHz, CDCl₃) δ 7.77 (s, 1H), 7.67 (s, 1H), 5.37 - 5.09 (m, 2H), 2.62 (t, *J* = 9.2 Hz, 1H), 2.28 - 1.20 (m, 25H), 1.16 - 1.05 (m, 2H), 0.85 (t, *J* = 7.3 Hz, 3H), 0.80 (s, 3H).

### Example 94:

MS m/z (ESI): 414.3[M+H]⁺.

¹H NMR (400 MHz, CDCl₃) δ 7.68 (s, 2H), 5.28 - 5.20 (m, 2H), 2.54 (t, *J* = 8.0 Hz, 1H), 1.99 - 1.23 (m, 25H), 1.13 - 1.06 (m, 2H), 0.88 - 0.78 (m, 6H).

### Example 95

### 1-(2-(3R,5R,8R,9R,10S,13S,14S,15R,17S)-15-Ethyl-3-hydroxy-3,13-dimethylhexadecahydro-1H-cyclopenta[a]phenanthren-17-yl)-2-oxoethyl)-1H-pyrazole-3,5-dicarbonitrile

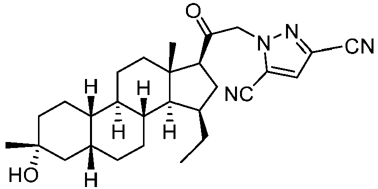

### Step 1: Preparation of 1-(2-((3R,5R,8R,9R,10S,13S,14S,15R,17S)-15-ethyl-3-hydroxy-3,13-dimethylhexadecahydro-1H-cyclopenta[a]phenanthren-17-yl)-2-oxoethyl)-1H-pyrazole-3,5-dicarbonitrile

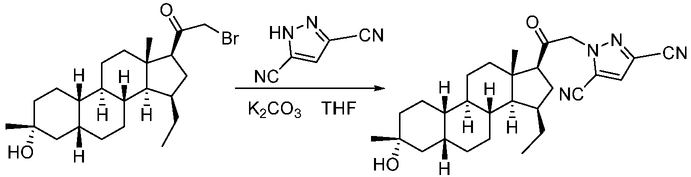

In accordance with Example 5, 2-bromo-1-((3R,5R,8R,9R,10S,13S,14S,15R,17S)-15-ethyl-3-hydroxy-3,13-dimethylhexadecahydro-1H-cyclopenta[a]phenanthren-17-yl)ethan-1-one was used as the starting material, accordingly, the product 1-(2-((3R,5R,8R,9R,10S,13S,14S,15R,17S)-15-ethyl-3-hydroxy-3,13-dimethylhexadecahydro-1H-cyclopenta[a]phenanthren-17-yl)-2-oxoethyl)-1H-pyrazole-3,5-dicarbonitrile (5 mg, yield: 9.2%) was obtained.

MS m/z (ESI): 463.2[M+H]⁺.

¹H NMR (400 MHz, CDCl₃) δ 7.21 (s, 1H), 5.29 - 5.02 (m, 2H), 2.61 (t, *J* = 9.3 Hz, 1H), 2.24 - 1.34 (m, 19H), 1.28 (s, 3H), 1.26 - 1.02 (m, 5H), 0.92 - 0.76 (m, 6H).

### Example 96

### 1-(2-((3R,5R,8R,9R,10S,13S,14S,15S,17S)-15-Cyclopropyl-3-hydroxy-3,13-dimethylhexadecahydro-1H-cyclopenta[a]phenanthren-17-yl)-2-oxoethyl)-1H-pyrazole-3,5-dicarbonitrile

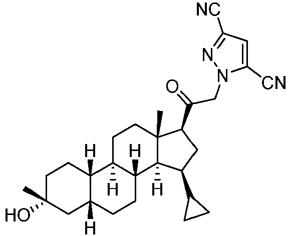

### Step 1: Preparation of 1-(2-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-oxoethyl)-1H-pyrazole-3,5-dicarbonitrile

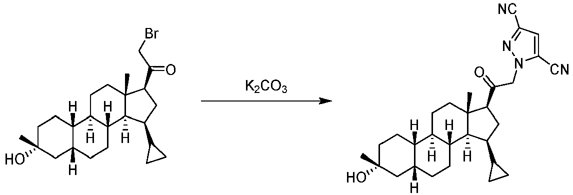

In accordance with Example 3, 1-((3R,5R,8R,9R,10S,13S,14S,15S,17S)-15-cyclopropyl-3-hydroxy-3,13-dimethylhexadecahydro-1H-cyclopenta[a]phenanthren-17-yl)ethan-1-one was used as the starting material, accordingly, 1-(2-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-oxoethyl)-1H-pyrazole-3,5-dicarbonitrile (19.6 mg, yield: 18%) was obtained.

MS m/z (ESI): 473.2[M- H]⁻
¹H NMR (400 MHz, CDCl₃) δ 7.20 (s, 1H), 5.24 - 5.09 (m, 2H), 2.58 - 2.50 (m, 1H), 2.26 - 2.07 (m, 2H), 2.06 - 1.95 (m, 2H), 1.90 - 1.80 (m, 2H), 1.78 - 1.66 (m, 2H), 1.58 - 1.22 (m, 16H), 1.19 - 1.06 (m, 2H), 0.94 (s, 3H), 0.87 - 0.78 (m, 1H), 0.64 - 0.56 (m, 1H), 0.45 - 0.39 (m, 1H), 0.18 - 0.02 (m, 2H).

### Example 97

### 1-((3R,5R,8R,9R,10S,13S,14S,15S,17S)-15-Cyclopropyl-3-hydroxy-3,13-dimethylhexadecahydro-1H-cyclopenta[a]phenanthren-17-yl)-2-(3-(trifluoromethyl)-1H-pyrazol-1-yl)ethan-1-one

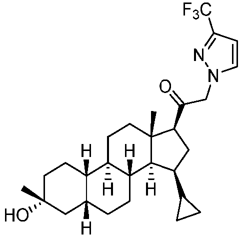

### Step 1: Preparation of 1-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-(3-(trifluoromethyl)-1H-pyrazol-1-yl)ethan-1-one

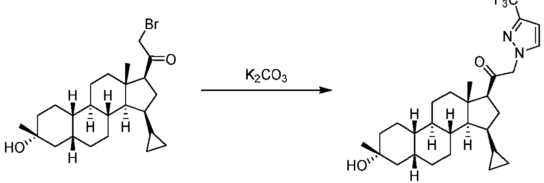

In accordance with Example 3, 1-((3R,5R,8R,9R,10S,13S,14S,15S,17S)-15-cyclopropyl-3-hydroxy-3,13-dimethylhexadecahydro-1H-cyclopenta[a]phenanthren-17-yl)ethan-1-one was used as the starting material, accordingly, 1-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-(3-(trifluoromethyl)-1H-pyrazol-1-yl)ethan-1-one (18.0 mg, yield: 23%) was obtained.

MS m/z (ESI): 491.3[M- H]⁻
¹H NMR (400 MHz, CDCl₃) δ 7.47 (d, *J* = 2.0 Hz, 1H), 6.59 (d, *J* = 2.0 Hz, 1H), 5.08 - 4.90 (m, 2H), 2.53 - 2.42 (m, 1H), 2.24 - 1.94 (m, 4H), 1.91 - 1.79 (m, 3H), 1.78 - 1.66 (m, 2H), 1.52 - 1.22 (m, 15H), 1.18 - 1.02 (m, 2H), 0.91 (s, 3H), 0.88 - 0.76 (m, 1H), 0.65 - 0.54 (m, 1H), 0.48 - 0.36 (m, 1H), 0.18 - 0.00 (m, 2H).

### Example 98

### 1-((3R,5R,8R,9R,10S,13S,14S,15S,17S)-15-Cyclopropyl-3-hydroxy-3,13-dimethylhexadecahydro-1H-cyclopenta[a]phenanthren-17-yl)-2-(2H-tetrazol-2-yl)ethan-1-one

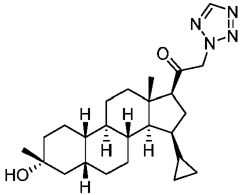

### Step 1: Preparation of 1-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-(2H-tetrazol-2-yl)ethan-1-one

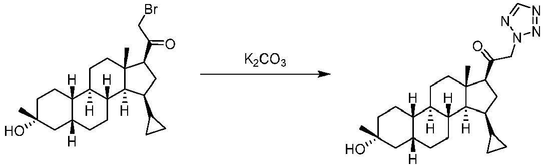

In accordance with Example 3, 1-((3R,5R,8R,9R,10S,13S,14S,15S,17S)-15-cyclopropyl-3-hydroxy-3,13-dimethylhexadecahydro-1H-cyclopenta[a]phenanthren-17-yl)ethan-1-one was used as the starting material, accordingly, 1-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-(2H-tetrazol-2-yl)ethan-1-one (12.9 mg, yield: 13.2%) was obtained.

MS m/z (ESI): 409.3[M-H₂O+ H]⁺
¹H NMR (400 MHz, CDCl₃) δ 8.57 (s, 1H), 5.46 (s, 2H), 2.58 - 2.47 (m, 1H), 2.24 - 1.94 (m, 4H), 1.93 - 1.80 (m, 3H), 1.77 - 1.66 (m, 2H), 1.50 - 1.21 (m, 15H), 1.18 - 1.06 (m, 2H), 0.95 (s, 3H), 0.87 - 0.78 (m, 1H), 0.64 - 0.55 (m, 1H), 0.46 - 0.38 (m, 1H), 0.17 - 0.01 (m, 2H).

### Example 99

### 3-Chloro-1-(2-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-oxoethyl)-1H-pyrazole-4-carbonitrile

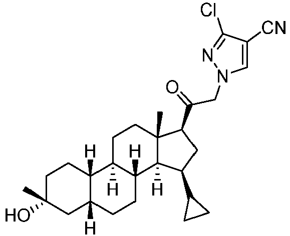

### Step 1: Preparation of 3-chloro-1-(2-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-oxoethyl)-1H-pyrazole-4-carbonitrile

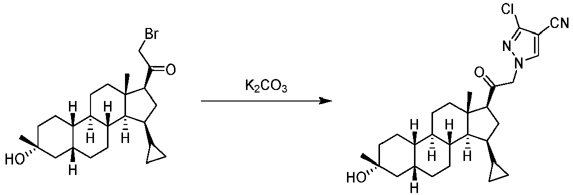

In accordance with Example 5, 1-((3R,5R,8R,9R,10S,13S,14S,15S,17S)-15-cyclopropyl-3-hydroxy-3,13-dimethylhexadecahydro-1H-cyclopenta[a]phenanthren-17-yl)ethan-1-one was used as the starting material, accordingly, 3-chloro-1-(2-((3R,5R,8R,9R,10S,13S,14S,15S,17S)-15-cyclopropyl-3-hydroxy-3,13-dimethylhexadecahydro-1H-cyclopenta[a]phenanthren-17-yl)-2-oxoethyl)-1H-pyrazole-4-carbonitrile (13.5 mg, yield: 17.4%) was obtained.

MS m/z (ESI): 482.2[M- H]⁻
¹H NMR (400 MHz, CDCl₃) δ 7.82 (s, 1H), 5.01 - 4.76 (m, 2H), 2.55 - 2.41 (m, 1H), 2.22 - 1.94 (m, 4H), 1.91 - 1.77 (m, 3H), 1.76 - 1.66 (m, 2H), 1.56 - 1.24 (m, 15H), 1.18 - 1.04 (m, 2H), 0.90 (s, 3H), 0.87 - 0.75 (m, 1H), 0.64 - 0.53 (m, 1H), 0.46 - 0.38 (m, 1H), 0.17 - 0.02 (m, 2H).

### Example 100

### 1-((3R,5R,8R,9R,10S,13S,14S,15R,17S)-15-Ethyl-3-hydroxy-3,13-dimethylhexadecahydro-1H-cyclopenta[a]phenanthren-17-yl)-2-(2H-tetrazol-2-yl)ethan-1-one

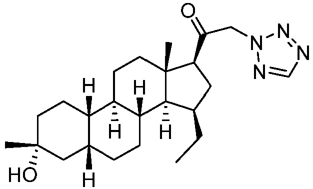

### Step 1: Preparation of 1-((3R,5R,8R,9R,10S,13S,14S,15R,17S)-15-ethyl-3-hydroxy-3,13-dimethylhexadecahydro-1H-cyclopenta[a]phenanthren-17-yl)-2-(2H-tetrazol-2-yl)ethan-1-one

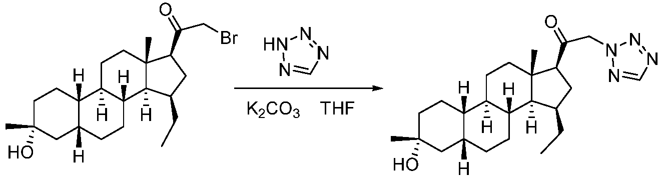

In accordance with Example 3, 2-bromo-1-((3R,5R,8R,9R,10S,13S,14S,15R,17S)-15-ethyl-3-hydroxy-3,13-dimethylhexadecahydro-1H-cyclopenta [a]phenanthren-17-yl)ethan-1-one was used as the starting material, accordingly, the product 1-((3R,5R,8R,9R,10S,13S,14S,15R,17S)-15-ethyl-3-hydroxy-3,13-dimethylhexadecahydro-1H-cyclopenta[a]phenanthren-17-yl)-2-(2H-tetrazol-2-yl)ethan-1-one (11 mg, white solid, yield: 18.8%) was obtained.

MS m/z (ESI): 397.2[M-H₂O+H]⁺
¹H NMR (400 MHz, CDCl₃) δ 8.57 (s, 1H), 5.50 - 5.41 (m, 2H), 2.62 (t, *J* = 8.5 Hz, 1H), 2.29 - 1.30 (m, 20H), 1.28 (s, 3H), 1.27 - 1.05 (m, 4H), 0.85 - 0.82 (m, 6H).

### Biological Assay and Evaluation of the Compounds

The present invention is further described below in combination with the following test examples, which are not intended to limit the scope of the present invention, which is as defined in the claims.

### 1. GABA_{A} receptor binding ability assay of the compounds of the present invention

1.1 Experimental objective: The objective of this test example is to measure the ability of the compounds to allosterically inhibit the binding of the ion channel blocker (tert-butylbicyclophosphorothionate (TBPS)) to the GABA-A receptor.

Experimental instruments:

| Instruments/Consumables | Supplier | Model |
|---|---|---|
| Vortex mixer | IKA | MS3 basic |
| Electric thermostat incubator | Shanghai Yiheng Instrument Co., Ltd. | DHP-9032 |
| TopCount | PerkinElmer | NTX |
| Universal Harvester | Perkin Elmer | UNIFILTER-96 |
| High-speed floor-standing centrifuge | Thermo | LYNX 4000 |
| Glass tissue homogenizer | Nanjing Luanyu Glass Instrument Co., Ltd. | 50 mL |
| Sprague-Dawley Rat | Pharmaron | |
| Protease inhibitor | roche | 11836170001 |
| 1.1 mL deep 96-well plate, round bottom | Axygen | P-DW-11-C |
| ULTIMA GOLD | Perkin Elmer | 77-16061 |
| UNIFILTER-96 GF/B filter plate | Perkin Elmer | 6005177 |
| Polyethylenimine (PEI), branched | Sigma | 408727 |

### 1.2 Experimental procedures

1.2.1 Extraction of cerebral cortex cell membrane:
1. The cerebral cortex of male Sprague-Dawley rat was isolated.
2. A pre-chilled 0.32 M sucrose solution (one tablet of protease inhibitor was added per 100 mL) was added to the cerebral cortex (the volume of sucrose solution was 10 times the volume of the cerebral cortex). The mixture was crushed with a 50 mL glass tissue homogenizer in batches and mixed well.
3. The mixture was centrifuged at 1,500 g, 4°C for 10 minutes, and the supernatant was collected.
4. The mixture was centrifuged at 20,000 g, 4°C for 30 minutes, and the supernatant was discarded.
5. The precipitate was resuspended with the pre-chilled phosphate buffer saline (PBS) (one tablet of protease inhibitor was added per 100 mL). An average of 4 mL of PBS was added per rat, and the mixture was mixed well with a glass tissue homogenizer.
6. The mixture was centrifuged at 10,000 g, 4°C for 10 minutes, and the supernatant was discarded.
7. Steps 5 and 6 were repeated three times.
8. Finally, the precipitate was resuspended with 4 volumes of PBS. The resulting solution was dispensed, frozen in liquid nitrogen, and stored at -80°C.
9. The protein concentration was measured by the bicinchoninic acid (BCA) method.

### 1.2.2 35S-TBPS binding assay

1. 230 µL of PBS was added to each well of a well plate with 1.1 mL volume.
2. 60 µL of the cerebral cortex cell membrane (5 µg/µL) solution was added to each well, and the mixture was mixed well.
3. The test compound (3 µL per well) was added, and the plate was incubated at 25°C for 5 minutes. The DMSO concentration was 1%. The initial compound concentration was 1 µM, and a 3-fold dilution in gradient was carried out to obtain a total of 8 gradients and 2 replicates. 1% DMSO was used as a negative control, and 10 µM P026-2 was used as a positive control.
4. GABA was added at a final concentration of 5 µM, and incubated at 25°C for 5 minutes. 1 mM GABA solution was formulated, and 1.5 µL of the solution was added to each well.
5. 35S-TBPS was added at a final concentration of 2 nM. The concentration of isotope mother solution was 9.7 µM. After dilution with PBS for 100 times, 6 µL of the diluted isotope solution was added to each well.
6. The plate was incubated at 4°C for 20 hours.
7. The FilterMate GF/C plate was pre-treated with 0.5% PEI, and incubated at 4°C for 1 hour.
8. The FilterMate GF/C plate was washed with Universal Harvester twice, 50 mL PBS each time.
9. The reaction solution was transferred to the GF/C plate, and each well was washed 4 times with 900 µL of PBS.
10. The washed GF/C plate was placed at 55°C and dried for 10 minutes.
11. 40 µL of scintillation solution was added to each well, and the CPM value was read with TopCount.

### 1.2.3 Experimental data processing method:

In the experiment, the CPM (counts per minute) value was read with TopCount. According to the readings of the High control (DMSO) and the Low control (10 µM of the positive compound) experimental groups, the % inhibition was calculated based on the following formula: $% Inhibition = 100 \times \left({CPM}_{High control}-{CPM}_{Sample}\right) / \left({CPM}_{High control}-{CPM}_{Low control}\right)$

The IC₅₀ of the compound was calculated according to the following 4-parameter nonlinear logic formula: $Y = Bottom + \left(Top-Bottom\right) / \left(1+{10}^{∧}\left(\left(LogIC50-X\right)*HillSlope\right)\right) ,$ wherein:
X represents the log of compound concentration,
Y represents the % Inhibition.

The effect of the compound of the present invention on the TBPS binding activity was determined by the above test, and the measured IC₅₀ values are shown in Table 5.

**Table 5: IC₅₀ of the compounds of the present invention on inhibiting the TBPS binding activity**

| Example No. | **35S-TBPS bindng assay (nM)** | Example No. | **35S-TBPS bindng assay (nM)** |
|---|---|---|---|
| 1 | 24.3 | 71 | 11.4 |
| 2 | 14.9 | 73 | 10.2 |
| 3 | 13.5 | 80 | 18.3 |
| 4 | 3.1 | 84 | 6.1 |
| 5 | 14.7 | 85 | 15.8 |
| 7 | 21.3 | 86 | 8.0 |
| 9 | 4.6 | 87 | 17.8 |
| 14 | 6.4 | 88 | 6.7 |
| 24 | 18.3 | 89 | 4.3 |
| 25 | 28.1 | 90 | 16.2 |
| 28 | 15.4 | 91 | 25.2 |
| 29 | 15.4 | 92 | 27.0 |
| 30 | 28.7 | 94 | 23.9 |
| 35 | 5.0 | 95 | 16.2 |
| 39 | 6.2 | 96 | 28.5 |
| 40 | 8.2 | 97 | 18.6 |
| 42 | 24.6 | 98 | 21.4 |
| 43 | 5.4 | 99 | 24.4 |
| 44 | 6.1 | 100 | 25.6 |
| 47 | 7.6 | | |
| 48 | 7.5 | | |
| 68 | 9.6 | | |

Conclusion: The compounds of the examples of the present invention have a significant inhibitory effect on the TBPS binding activity.

### 2. Pharmacokinetic assay in Balb/C mice

### 2.1 Test objective:

Balb/C mice were used as test animals. The pharmacokinetic behavior in mice (plasma and brain tissue) of the compounds of Examples 1 to 4, 7, 9, 39 to 41, 44, 48 to 52, 59, 73, 88 to 91, 93 to 94, 96 and 98 orally administered at a dose of 5 mg/kg was studied.

### 2.2 Test scheme

### 2.2.1 Test compounds:

Compounds of Examples 1 to 4, 7, 9, 39 to 41, 44, 48 to 52, 59, 73, 88 to 90, 93 to 94, 96 and 98 of the present invention, prepared by the applicant.

### 2.2.2 Test animals:

Male Balb/C mice were purchased from Shanghai Jiesijie Laboratory Animal Co., LTD, with Certificate No.: SCXK (Shanghai) 2013-0006 N0.311620400001794.

### 2.2.3 Administration:

Each group had 24 male Balb/C mice. After an overnight fast, Balb/C mice were administered p.o. with the test compound at an administration dose of 5 mg/kg and an administration volume of 10 mL/kg.

### 2.2.4 Sample collection:

0.2 ml of blood was taken from the heart before administration and at 0, 0.5, 1, 2, 4, 6, 8 and 24 hours after administration. The samples were stored in EDTA-K₂ tubes, and centrifuged for 6 minutes at 4°C, 6000 rpm to separate the plasma. The plasma samples were stored at -80°C. The mice were sacrificed with CO₂, and the whole brain tissue was taken out, weighed, placed in a 2 mL centrifuge tube and stored at -80°C.

### 2.2.5 Sample process:

1) 160 µL of acetonitrile was added to 40 µL of the plasma sample for precipitation, and then the mixture was centrifuged for 5-20 minutes at 3500 × g.
2) 90 µL of acetonitrile containing the internal standard (100 ng/mL) was added to 30 µL of the plasma and brain homogenate samples for precipitation, and then the mixture was centrifuged for 8 minutes at 13000 rpm.
3) 70 µL of the treated supernatant was taken and added to 70 µL of water, and mixed by vortex for 10 minutes. 20 µL of the mixture was taken to analyze the concentration of the test compound by LC/MS/MS. LC/MS/MS analysis instrument: AB Sciex API 4000 Qtrap.

### 2.2.6 Liquid chromatography analysis

• Liquid chromatography condition: Shimadzu LC-20AD pump
• Chromatographic column: Agilent ZORBAX XDB-C18 (50×2.1 mm, 3.5 µm)
• Mobile phase: Eluent A was 0.1% formic acid in water, and Eluent B was acetonitrile
• Flow rate: 0.4 mL/min
• Elution time: 0-4.0 minutes, the eluent is as follows:

| Time/minute | Eluent A | Eluent B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 2.3. Test results and analysis

The main parameters of pharmacokinetics were calculated by WinNonlin 6.1. The results of pharmacokinetic experiment in mice are shown in Table 6 below:

**Table 6: Results of pharmacokinetic experiment in mice**

| Example No. | Pharmacokinetic experiment (5 mg/kg) | | | | | |
|---|---|---|---|---|---|---|
| | Peak time | Plasma concentration | Area under curve | Area under curve | Half-life | Mean residence time |
| | tₘₐₓ(ng/mL) | Cₘₐₓ(ng/mL) | AUC₀₋ₜ(ng/mL×h) | AUC_{0-∞}(ng/mL×h) | t_{1/2}(h) | MRT(h) |
| 1 plasma | 0.5 | 1099.3 | 1360.5 | 1374.2 | 0.54 | 1.21 |
| 1 brain tissue | 0.5 | 636.7 | 839.7 | 913.0 | 2.84 | 2.39 |
| 2 plasma | 0.5 | 673.3 | 569.7 | 573.8 | 0.50 | 1.01 |
| 2 brain tissue | 0.5 | 690.3 | 718.9 | 721.8 | 0.50 | 1.10 |
| 3 plasma | 0.5 | 461.0 | 1241.5 | 1254.2 | 5.12 | 4.80 |
| 3 brain tissue | 0.5 | 1406.3 | 2303.0 | 3067.4 | 4.72 | 5.53 |
| 4 plasma | 0.5 | 219.0 | 409.0 | 483.8 | 3.19 | 4.03 |
| 4 brain tissue | 0.5 | 464.0 | 917.2 | 964.3 | 1.90 | 2.40 |
| 7 plasma | 0.5 | 663.7 | 774.9 | 778.0 | 0.9 | 1.1 |
| 7 brain tissue | 0.5 | 605.3 | 677.0 | 679.9 | 0.5 | 0.9 |
| 9 plasma | 1.0 | 1210 | 5057.2 | 5065.8 | 2.5 | 3.6 |
| 9 brain tissue | 1.0 | 1060.0 | 4731.4 | 4744.7 | 2.5 | 3.9 |
| 39 plasma | 0.5 | 553.7 | 1305.0 | 1316.9 | 2.99 | 4.15 |
| 39 brain tissue | 1.0 | 678.0 | 2642.7 | 2680.6 | 3.18 | 4.81 |
| 40 plasma | 0.5 | 719.7 | 1704.9 | 1772.1 | 1.64 | 1.43 |
| 40 brain tissue | 1.0 | 998.0 | 2403.0 | 2498.4 | 2.37 | 2.35 |
| 41 plasma | 0.5 | 524.7 | 588.5 | 604.6 | 3.27 | 1.48 |
| 41 brain tissue | 0.5 | 710.7 | 657.1 | 676.0 | 3.95 | 1.41 |
| 48 plasma | 1.0 | 508.3 | 1577.3 | 1758.8 | 2.04 | 3.50 |
| 48 brain tissue | 1.0 | 382.7 | 1127.8 | 1275.4 | 2.00 | 3.66 |
| 49 plasma | 0.5 | 1146.3 | 2967.0 | 2972.0 | 2.79 | 3.71 |
| 49 brain tissue | 0.5 | 723.7 | 2051.0 | 2237.3 | 2.12 | 3.17 |
| 50 plasma | 0.5 | 1303.3 | 2430.5 | 2482.0 | 1.40 | 2.06 |
| 50 brain tissue | 0.5 | 635.7 | 1098.1 | 1117.6 | 1.31 | 1.91 |
| 51 plasma | 1.0 | 2986.7 | 13411.8 | 13415.2 | 1.75 | 3.47 |
| 51 brain tissue | 1.0 | 1480.0 | 4821.6 | 5273.3 | 2.09 | 3.40 |
| 52 plasma | 0.5 | 2136.7 | 7156.4 | 8601.3 | 3.06 | 4.59 |
| 52 brain tissue | 1.0 | 1523.3 | 5846.2 | 6511.2 | 2.28 | 3.82 |
| 59 plasma | 1.0 | 1146.7 | 4643.8 | 5922.3 | 2.64 | 4.96 |
| 59 brain tissue | 1.0 | 296.2 | 1439.7 | 2234.7 | 5.59 | 7.97 |
| 73 plasma | 1.0 | 386 | 1034.8 | 1060.9 | 1.19 | 2.12 |
| 73 brain tissue | 1.0 | 626.3 | 1676.2 | 1706.2 | 1.12 | 2.07 |
| 88 plasma | 0.5 | 1750.0 | 4627.3 | 5132.2 | 1.78 | 3.31 |
| 88 brain tissue | 1.0 | 892.0 | 3205.7 | 3501.3 | 1.59 | 3.22 |
| 89 plasma | 2.0 | 640.3 | 4103.8 | 4110.3 | 2.06 | 4.38 |
| 89 brain tissue | 2.0 | 724.0 | 3736.7 | 4450.7 | 2.82 | 4.71 |
| 90 plasma | 1.0 | 762.7 | 2785.0 | 3792.4 | 3.85 | 5.88 |
| 90 brain tissue | 1.0 | 613.0 | 2126.5 | 3162.6 | 6.02 | 7.58 |
| 93 plasma | 0.5 | 2483.3 | 5982.0 | 6106.9 | 1.19 | 2.22 |
| 93 brain tissue | 0.5 | 1738.0 | 4327.1 | 4539.1 | 1.21 | 2.10 |
| 94 plasma | 0.5 | 1633.3 | 3545.3 | 3634.6 | 1.35 | 2.55 |
| 94 brain tissue | 0.5 | 1048.0 | 2153.2 | 2251.2 | 1.68 | 2.68 |
| 96 plasma | 0.5 | 1650.0 | 6287.8 | 6492.4 | 1.70 | 3.08 |
| 96 brain tissue | 2.0 | 812.0 | 8818.0 | 9215.1 | 3.95 | 7.81 |
| 98 plasma | 1.0 | 2086.7 | 7785.5 | 8000.6 | 1.45 | 2.97 |
| 98 brain tissue | 1.0 | 1966.0 | 8677.8 | 12488.1 | 4.66 | 6.73 |

It can be seen from the results of the pharmacokinetic experiment in mice in the table that the compounds of the examples of the present invention showed good metabolic properties, and both the exposure AUC and the maximum blood drug concentration Cₘₐₓ performed well. Compared with the existing compound SAGE-217, the compounds of the present invention can significantly increase the exposure in mice, while the maximum tolerated dose is equivalent or even better than that of SAGE-217. Therefore, the compounds of the present invention have good tolerance, wide safety window and high safety.

### 3. In vivo pharmacodynamic experiment in the forced swimming model in mice

### 3.1 Experimental objective

The antidepressant effect of the compound was evaluated by the forced swimming model in mice.

### 3.2 Main instruments and reagents of the experiment

### 3.2.1 Instruments

Forced swimming device (JLBehv-FSC-4, Shanghai Jiliang Software Technology Co., Ltd.).

### 3.2.2 Reagents

Sodium carboxymethyl cellulose (CMC-Na, SLBV9664, Sigma)
Tween 80 (BCBV8843, Sigma)

### 3.2.3 Test compounds

Compounds of Examples 1 to 3, Example 9, Example 40, Example 41, Examples 48 to 52, Example 90 and Example 98 of the present invention, prepared by the applicant.

### 3.3 Experimental procedures

### 3.3.1 Adaptation:

Male ICR mice (25-35 g) were adapted in the experiment environment for 3 days before the forced swimming test.

### 3.3.2 Grouping and administration:

According to the experiment design, the mice were randomly grouped on the day before the experiment according to body weight, with 12 mice in each group. Before the test, the compounds of each example were administered intragastrically according to the Tmax thereof in the brain in mice pharmacokinetic experiment.

Respectively:
1) Model group (0.5% CMC-Na -1% Tween 80 solution, *p.o.,* 10 mL/kg);
2) Compounds of Examples 1 to 3, Example 40, Example 41, Examples 48 to 51, Example 90 and Example 98 (10 mg/kg, *p.o.,* 10 mL/kg); Example 9 and Example 52 (5 mg/kg, *p.o*., 5 mL/kg).

When being administered, the compounds of each example were suspended in 0.5% CMC-Na +1% Tween 80 solution to the desired concentration.

### 3.3.2 Forced swimming test:

0.5-1 hour after administration, ICR mice were placed in a forced swimming device (transparent glass drum (water depth 18 cm, water temperature 25-26°C), one mouse per tank) and forced to swim for 6 minutes. The forced swimming device recorded the floating time of the ICR mice during the entire 6 minutes, and the data of the latter four minutes were used for data analysis. The mice were taken out immediately after the swimming experiment, wiped dry and put back in their original cages.

Note: The criterion for determining the immobility time is that the mouse stops struggling in water and floats, and there are only slight limb movements to keep the head floating on the water.

### 3.4 Data analysis

$Floating time percentage = 100 * floating time / 240 s .$

### 3.5 Test data:

**Table 7: Results of the forced swimming experiment in mice**

| **Example No.** | **Dose (mpk)** | **Mean (immobility, s)** | **Mean (immobility, %)** |
|---|---|---|---|
| Vehicle | / | 163.70 | 68.22 |
| Example 1 | 10 | 130.22 | 54.26 |
| Example 2 | 10 | 68.39 | 28.50 |
| Example 3 | 10 | 143.81 | 59.93 |
| Example 9 | 5 | 138.22 | 57.60 |
| Example 40 | 10 | 84.90 | 35.30 |
| Example 41 | 10 | 85.8 | 35.75 |
| Example 48 | 10 | 134.21 | 55.92 |
| Example 49 | 10 | 85.77 | 35.74 |
| Example 50 | 10 | 70.96 | 29.57 |
| Example 51 | 5 | 73.72 | 30.72 |
| Example 52 | 5 | 91.21 | 38.00 |
| Example 90 | 10 | 88.61 | 36.92 |
| Example 98 | 5 | 80.5 | 33.54 |

### 3.6 Experimental results

It can be seen from the above results that the compounds of the examples of the present application can significantly shorten the cumulative immobility time of the forced-swimming mice, and have a significant antidepressant effect.

The immobility time during the latter four minutes of the compounds of Example 2, Example 40, Example 41, Example 49, Example 50, Example 90 and Example 98 was very significantly different compared with that of the model group.

### 4. In vivo pharmacodynamic test in the PTZ-induced epilepsy model in mice

### 4.1 Test objective

The PTZ-induced epilepsy model in CD-1 mice was established, and the antiepileptic effect of the compounds of Example 2, Example 40, Example 41 and Example 52 was evaluated using this model.

### 4.2 Test method

### 4.2.1 Test animals

50 male CD-1 mice were purchased from Beijing Vital River Laboratory Animal Technology Co. Ltd. The test animals were adapted at the animal room in the third building of Shanghai ChemPartner Co., Ltd for 7 days before the experiment. The average body weight of the animals on the test day was 32.2±0.2 grams. Feeding environment: 5 animals/cage, room temperature 23±2°C, 12/12 hours of light and dark cycle, free acess to food and water.

The mice were randomly grouped for the experiment on the test day.

### 4.2.2 Test compounds

Compounds of Example 2, Example 40, Example 41 and Example 52 (prepared by the applicant). The test compounds were stored in a refrigerator at 4°C.

**Table 8: Test reagent information**

| **Name** | **Article numbe r** | **Batch number** | **Property** | **Supplier** | **Total weight** | **Purity** | **Store condition** |
|---|---|---|---|---|---|---|---|
| Pentylenetet razol (PTZ) | P6500 | SLBD387 6V | White crystal | Sigma | 25 g | 100% | -20°C refrigerati on |
| Sodium carboxymet hyl cellulose | 9004-32-4 | LAB0R36 | White solid | Beijing J&K Scientific Co., Ltd. | 100G | 800cps | Room temperatur e/dry/in the dark |
| Tween-80 | 9005-65-6 | P1279207 | Transpare nt liquid | GENERAL-REAGENT^{®} | 500 mL | 100% | Room temperatur e/dry |
| Hydroxypro pyl β-cyclodextrin | 19184C | OP1901A | White powder | Seebio Biotech | 500 g | ≥98% | 2-8°C refrigerati on |
| 0.9% sodium chloride injection | H37022 749 | H1801031 4 | Transpare nt liquid | Shandong Hualu Pharmaceutic al Co., Ltd. | 500 mL | 100% | Room temperatur e/dry |

### 4.2.3 Test equipments

- 1 ml sterile disposable syringe with needle (purchased from Zhejiang Kangdelai Medical Devices Co., Ltd.)
- Pipette: Eppendorf Research Plus (100-1000µL)
- Vortex mixer: Kylin-Bell Vortex 5
- Ultrasonic instrument: JL-360 ultrasonic cleaner
- Balance: METTLER TOLEDO XS204 precision balance
- Balance: METTLER TOLEDO XS6002S electronic balance
- Plexiglass box: 25 cm length * 15 cm width * 15 cm hight with one opaque side wall, custom made by Suzhou Fengshi Laboratory Animal Equipment Co., Ltd
- 3-channel timer: Oregon/Model NO.WB-388.

### 4.2.4 Test animal grouping

1) Vehicle/PTZ: 0.5% CMC-Na+1% Tween-80 (10 ml/kg, p.o.), administered 0.5 hr before the PTZ administration; PTZ (120 ml/kg, s.c.), administered before the experiment;
2) 3 mg/kg of the compounds of Examples/PTZ: the compounds of Example 2, Example 40, Example 41 and Example 52 (3 mg/kg, 10 ml/kg, p.o.), administered 0.5 hr before the PTZ administration; PTZ (120 ml/kg, s.c.), administered before the experiment.

### 4.3 Experimental procedures

### 4.3.1 Solvent formulation

1) 0.5% CMC-Na+1%Tween-80 (administration volume: 10 mL/kg):
   1 g of sodium carboxymethyl cellulose was precisely weighed and added to a 250 mL solvent bottle, then 150 mL of double-distilled water was added. The mixture was stirred at room temperature for 4 hours with a magnetic stirrer to obtain a uniform and clear solution. 2 mL of Tween-80 was slowly added, and the mixture was stirred at room temperature for 3 hours to obtain a uniform and clear solution. The solution was slowly transferred to a 200 mL volumetric flask, and double distilled water was added to the constant volume of 200 mL. The solution was transferred to a 250 mL solvent bottle, and stirred for 1 hour with a magnetic stirrer to obtain a uniform and clear solution.
2) 30% hydroxypropyl-β-cyclodextrin:
   30.6122 g of hydroxypropyl-β-cyclodextrin (purity: 98%) was precisely weighed and added to a 100 mL solvent bottle, then 60 mL of double-distilled water was added. The mixture was mixed by vortex for 3 minutes, and treated by ultrasound at room temperature for 15 minutes to obtain a uniform and clear solution. Double distilled water was added to the constant volume of 100 mL, mixed by vortex for 1 minute, and treated by ultrasound at room temperature for 5 minutes to obtain a uniform and clear solution.

### 4.3.2 Test compound formulation

1) 12 mg/mL PTZ (dose: 120 mg/kg; administration volume: 10 mL/kg): 248 mg of PTZ was precisely weighed and added to a 40 mL brown flask, then 20.667 mL of physiological saline was added. The mixture was mixed by vortex for 2 minutes, and treated by ultrasound at room temperature for 2 minutes to obtain a uniform and clear solution (concentration: 12 mg/mL).
2) 0.3 mg/mL of the compounds of Example 5 or Example 23 (dose: 3 mg/kg; administration volume: 10 mL/kg):
   A certain amount of 0.5% CMC-Na+1% Tween-80 was taken and added to a flask containing a certain amount of the compounds of Example 5 or Example 23. The mixture was mixed by vortex for 3 minutes, and treated by ultrasound at room temperature for 15 minutes to obtain a uniform suspension (concentration: 0.3 mg/mL).

### 4.3.3 Test method

1) The test animals were transferred to the operating room to adapt to the environment 1 hour before the test;
2) The animals were randomly grouped, marked and weighed;
3) The compounds of Example 2, Example 40, Example 41 and Example 52 were administered respectively 1 hour before the PTZ administration, or 0.5% CMC-Na+1% Tween-80, the compounds of Example 2, Example 40, Example 41 and Example 52 were administered respectively 0.5 hour before the PTZ administration;
4) PTZ (120 mg/kg) was administrated subcutaneously before the experiment observation, and this time point was recorded as the observation start point;
5) After the administration of PTZ, the animal was immediately placed in the observation box and observed for 30 minutes, and the followings were recorded: a) the incubation period of the first clonic seizure, b) the incubation period of the first generalized tonic seizure, c) the number of clonic seizures, d) the number of generalized tonic seizures, e) the time when the animal died, 6) if the animal did not have seizures during the 30-minute observation period, the incubation period was recorded as 1800 sec and the number of seizures was recorded as 0.
   - Clonic seizure: generalized clonic seizure in animals lasts for more than 3 seconds, and is accompanied by a fall;
   - Tonic seizure: the limbs straightens 90° to the body;
6) The possible side effects induced by the drug after the administration were observed and recorded, which can be divided into four levels:
   - None: normal
   - Mild sedation
   - Moderate sedation
   - Severe sedation
7) The test was carried out from 12:00 am to 16:30 pm.

### 4.4 Adaptation to the environment

The test animals were transferred to the operating room to adapt to the environment 1 hour before the test.

### 4.5 Grouping and administration

The mice were randomly grouped, marked and weighed; 10 mice per group. The test compound was administered orally at an administration volume of 10 mL/kg 30-60 minutes before the PTZ administration.

### 4.6 PTZ modeling and testing

PTZ (120 mg/kg) was administrated subcutaneously before the experiment observation, and this time point was recorded as the observation start point; after the administration of PTZ, the animal was immediately placed in the observation box and observed for 30 minutes, and the followings were recorded: a) the incubation period of the first clonic seizure, b) the incubation period of the first generalized tonic seizure, c) the number of clonic seizures, d) the number of generalized tonic seizures, e) the time when the animal died. If the animal did not have seizures during the 30-minute observation period, the incubation period was recorded as 1800 sec and the number of seizures was recorded as 0.

### 4.7 Data analysis

All measurement data were expressed as Mean ± SEM, and analysed with Prism 6.0 statistical software.

### 4.8 Test data:

**Table 9: Results of the in vivo pharmacodynamic experiment in the epilepsy model in mice**

| **Examp le No.** | **Dose (mpk)** | **Incubation period of the clonic seizure (sec)** | **Number of clonic seizures** | **Incubation period of the generalized tonic seizure (sec)** | **Number of generali zed tonic seizures** | **Time when the animal died (sec)** | **Mortali ty rate (%)** |
|---|---|---|---|---|---|---|---|
| | | **Mean±SEM** | **Mean** | **Mean** | **Mean** | **Mean** | |
| Vehicle | / | 331.4±61.2 | 2.1±0.2 | 821.6±107.7 | 1.0±0.0 | 839.8±108.0 | 100% |
| 2 | 3 | 902.9±200.8 | 1.3±0.3 | 1736.0±63.2 | 0.1±0.1 | 1739.8±60.2 | 10% |
| 40 | 3 | 1308.1±170.1 | 0.5±0.2 | 1800.0±0.0 | 0.0±0.0 | 1800.0±0.0 | 0% |
| 41 | 3 | 841.4±179.8 | 1.9±0.4 | 1497.4±124.3 | 0.5±0.2 | 1573.1±117.9 | 40% |
| 52 | 3 | 635.4±149.9 | 1.8±0.3 | 1506.8±149.8 | 0.3±0.2 | 1618.1±121.3 | 30% |

### 4.9 Experimental results

The compounds of the examples significantly prolonged the incubation period of clonic seizure and generalized tonic seizure and reduced the number of clonic seizures and generalized tonic seizures compared with the control group. The compounds of the examples can protect 60%-100% of animals from death, significantly prolong the incubation period of death, and have a good antiepileptic effect.

### 5. Positive regulatory effect of the compounds of the present invention on GABA_{A} receptor

### 5.1 Experimental objective:

The objective of this test example is to determine the positive regulatory effect of the compound on GABA_{A} receptor by the electrophysiological manual patch clamp method.

### 5.2 Experimental instruments:

Manual patch clamp system HEKA EPC10 USB signal amplifier and digital conversion system (purchased from HEKAElectronics, Germany), centrifuge (Eppendorf, etc.), carbon dioxide incubator (purchased from Thermo, etc.), biological safety cabinet (Thermo, etc.), pipette (purchased from Eppendorf, etc.).

### 5.3 Experimental method:

The cells used in this experiment came from the HEK293T cell line which was transfected with human GABA_{A} receptor α1, β2 and γ2 subunit cDNA and stably exhibited normal GABA_{A} receptor function. The cells were grown in a culture dish with DMEM medium (purchased from Invitrogen), and cultured in an incubator at 37°C and 5% CO₂. Before the electrophysiological experiment, the cells were transferred to a round glass slide placed in the culture dish, and grown in the same culture medium and under the same culture condition as above. The density of cells on each round glass slide should meet the requirement that most cells were independent and individual.

Each test compound was diluted in gradient with DMSO to obtain a stock solution, which was then diluted with extracellular fluid by 1,000-fold to obtain the final test concentration. The final concentration of DMSO in the compound solution with each concentration was 0.1%. The experiment used the manual patch clamp system HEKA EPC10 USB signal amplifier and digital conversion system for whole cell current recording. The round glass slide with HEK293T GABA_{A} cells grown on the surface was taken out of the culture dish, and placed in the electrophysiological recording tank under the inverted microscope. The recording tank was continuously perfused with extracellular fluid. During the experiment, the whole-cell current recording technology was used to record the chloride current of the GABA_{A} channel. 1 µM GABA was used to activate the chloride current of the GABA_{A} channel of each cell as an initial control. 1 µM GABA acted on the cell through the drug perfusion system to induce the chloride current of the GABA_{A} channel, the action time was about 3 to 5 seconds, and the current value was used as the initial control value. The test concentration of compound was perfused and incubated for 2 to 5 minutes. 1 µM GABA solution (mixed with the test concentration of compound) was given to the cells to observe the enhancement effect of the test compound on the current induced by 1 µM GABA. When the cell state was stable, the test compound acted on the same cell from low to high concentration to record the enhancement effect of the induced current.

### 5.4 Processing method of the experimental data:

The experimental data was analyzed by HEKA Patchmaster, Microsoft Excel and the data analysis software provided by Graphpad Prism.

### 5.5 Experimental conclusion:

The biological activity of the compounds of the present invention in the *in vitro* cell activity test obtained according to the above scheme is shown in Table 10:

**Table 10: EC50 values (nM) of the positive regulatory effect of GABA-A current**

| Example No. | EC50: nM | Example No. | EC50: nM |
|---|---|---|---|
| 9 | 60 | 86 | 74 |
| 40 | 57 | 88 | 49 |
| 41 | 130 | 89 | 43 |
| 52 | 150 | 96 | 84 |

| | | | |
|---|---|---|---|
| Conclusion: The compounds of the examples of the present invention have an obvious positive regulatory effect on GABA-A current. | | | |

### Screening Study on the Salt and Crystal Form of the Compound

### 1. Preparation of different crystal forms of the free base of formula (VI)

### 1.1 Preparation of crystal form I of the free base:

20.5 g of the free base compound of Example 40 (purity: ~95%) was dissolved in 125 mL of ethyl acetate, and the resulting solution was refluxed to clear. 125 mL of *n-*heptane was added dropwise, and granular solids precipitated during the addition process. The mixture was naturally cooled to 20°C in the oil bath, and then stirred at 10 to 20°C for 1 hour. The mixture was filtered, and the filter cake was dried to obtain 16.7 g of white solid. After detection and analysis, the solid was crystal form I of the free base, having the XRPD pattern as shown in Figure 1, the TGA-DSC spectrum as shown in Figure 2, and the DVS spectrum as shown in Figure 8.

### 1.2 Preparation of crystal form II of the free base:

0.2 g of crystal form I of the free base of Example 40 was added to a 20 mL dry clean glass flask, heated to 160°C for 5 minutes, and a sample was taken. After detection and analysis, the sample was crystal form II of the free base, having the XRPD pattern as shown in Figure 3, the TGA-DSC spectrum as shown in Figure 4, and the DVS spectrum as shown in Figure 9.

### 1.3 Preparation of crystal form III of the free base:

17.5 g of the free base compound of Example 40 (purity: ~86%) was dissolved in 96 mL of ethyl acetate, and the resulting solution was refluxed to clear. 96 mL of n-heptane was added dropwise. Flocculent solids precipitated during the addition process, and the precipitation rate was rapid. The mixture was naturally cooled to 20°C in the oil bath, and then stirred at 10 to 20°C for 1 hour. The mixture was filtered, and the filter cake was dried to obtain 12.1 g of white flocculent solid. After detection and analysis, the solid was crystal form III of the free base, having the XRPD pattern as shown in Figure 5, the TGA spectrum as shown in Figure 6, and the DSC spectrum as shown in Figure 10.

### 2. Screening of crystal form of the compound salt form

### 2.1 Experimental objective:

Different salts in crystal form were prepared with different counter ion acid by crystallization methods such as natural evaporation, solution crystallization, anti-solvent crystallization and the like.

### 2.2 Experimental instruments:

| Instrument name | Model | Manufacturer |
|---|---|---|
| Analytical Balance | BSA224S-CW | Sartorius |
| Analytical Balance | XPR2 | Mettler Toledo |
| Ultrasonic cleaner | SK5200LHC | Shanghai Kudos Ultrasonic instrument Co.,Ltd |
| Pipette | 5000µL,1000µL,200µL, 20µL | Eppendorf |

### 2.3 Screening of salt form of the compound of formula (VI)

0.5 mL of good solvent was added to 50 mg of the free base of Example 40 (by an 1 mL pipette), and the resulting mixture was subjected to ultrasound to obtain a solution of the free base (concentration: 100 mg/mL). Corresponding counter ion acids were weighed (the amount of the counter ion acid was 1 to 1.2 equivalents), and dissolved by 200 µL of the same good solvent (by an 1 mL pipette) respectively. The counter ion acids were added to the suspension of the free base respectively under stirring, and stirred overnight. If there was no precipitation, then an anti-solvent was added to precipitate a precipitation. The mixture was centrifuged at high speed, the supernatant was removed, and the resulting solid precipitate was vacuum dried at 40°C to obtain the corresponding salt of the compound.

The above good solvent was selected from the group consisting of methanol, ethanol, acetone, tetrahydrofuran, dichloromethane and 1,4-dioxane, and preferably ethyl acetate and ethanol.

The above poor solvent was selected from the group consisting of heptane, methyl tert-butyl ether, toluene, isopropyl ether and ethyl acetate, and preferably methyl *tert-*butyl ether and isopropyl ether.

The good solvent and poor solvent were miscible when used.

**Table 10: Screening experimental results of crystal form of salt form of Example 40**

| No. | Counter ion acid name | Counter ion acid weight mg (µL) | Solid state | XRPD result | Figure |
|---|---|---|---|---|---|
| 1 | Sulfuric acid | 14.85 mg | Dark brown oil | / | / |
| 2 | p-Toluenesulfonic acid | 26.87 mg | Dark brown oil | / | / |
| 3 | Methanesulfonic acid | 14.02 mg | White solid | Crystal form | 7 |
| 4 | 1,5-Naphthalenedisulfonic acid | 51.57 mg | Dark brown oil | / | / |

### 3. Solubility experiment

### 3.1 Experimental objective:

The solubility of the free base and salt of the compound in water, Fasted State Simulated Gastric Fluid (FaSGF), Fasted State Simulated Intestinal Fluid (FaSSIF) and Fed State Simulated Intestinal Fluid (FeSSIF) was compaed, so as to provide a basis for evaluating the druggability of the crystal form and salt salt.

### 3.2 Experimental scheme:

About 2 to 3 mg of crystal forms I, II and III of the free base of Example 40 were weighed, suspended in 1 mL of Fasted State Simulated Gastric Fluid (FaSSGF), Fasted State Simulated Intestinal Fluid (FaSSIF), Fed State Simulated Intestinal Fluid (FeSSIF) and pure water respectively, and shaked for 24 hours. Thermodynamic solubility of the compound at 37°C was determined using HPLC by external standard method.

### 3.3 Experimental results:

3.3.1 Solubility results of crystal forms I, II and III of the free base of Example 40 are as shown in Table 11 below:

**Table 11: Solubility experimental results of crystal form of the free base and salt of Example 40**

| Medium | Solubility (µg/mL) | | |
|---|---|---|---|
| | Crystal form I | Crystal form II | Crystal form III |
| Water | < 1 | < 1 | < 1 |
| FaSSGF | < 1 | < 1 | < 1 |
| FaSSIF | 3.262 | 3.705 | 4.914 |
| FeSSIF | 18.213 | 18.495 | 28.166 |

It can be seen from the solubility results of crystal forms I, II and III of the free base of Example 40 in the four media that the solubility of the three crystal forms of the free base of Example 40 is low, wherein the solubility of crystal form I of the free base is the lowest, indicating that crystal form I of the free base is the most stable crystal form under the current temperature condition.

### 4. Hygroscopicity experiment

### 4.1 Experimental objective:

The hygroscopicity of crystal forms I, II and III of the free base of Example 40 under different relative humidity conditions was investigated, so as to provide a basis for screening of the crystal form and salt of the compound, production and storage.

### 4.2 Experimental scheme:

10 to 15 mg of the free base of Example 40 was placed in the sample chamber of DVS. DVS was carried out, and the vapour sorption weight gain percentage of the compound at 80%RH was recorded at the same time.

### 4.3 Instrument parameters:

The DVS experiment parameters are as follows:

| | |
|---|---|
| Test temperature | 25°C |
| Mass change rate | dm/dt=0.02%, |
| Stable duration | Minimum 10 min, maximum 180 min |
| Drying duration | Equilibrating at 0%RH for 120 min |
| RH (%) measuring gradient | 10% |
| RH(%) measuring range | 0-95-0% |
| Cycles | 2 |

### 4.4 Experimental results:

4.4.1 Hygroscopicity results of crystal forms I, II and III of the free base of Example 40 are as shown in Table 12 below:

**Table 12: Hygroscopicity experimental results of different crystal forms of the free base of Example 40**

| No. | Free base | Hygroscopicity (25°C/80%RH) | Figure |
|---|---|---|---|
| 1 | Crystal form I | 0.07% | 8 |
| 2 | Crystal form II | 0.03% | 9 |
| 3 | Crystal form III | 0.10% | 10 |

Crystal forms I, II and III of the free base of Example 40 had no hygroscopicity.

### 5. Competitive experiment of different crystal forms of the free base

### 5.1 Experimental objective:

The stability among crystal forms I, II and III of the free base of Example 40 was investigated, so as to select the stable crystal form.

### 5.2 Experimental scheme:

Multiple portions of about 20 mg of crystal forms I, II and III of the free base of Example 40 were accurately weighed, and mixed in pairs in equal amounts. Methyl *tert-*butyl ether and n-heptane were added respectively, and the resulting mixture was stirred at 40°C for 7 days. The mixture was centrifuged at high speed, the supernatant was removed, and the resulting solid precipitate was vacuum dried in an oven at 40°C and characterized by XRPD.

### 5.3 Experimental results:

The competitive results of crystal forms I, II and III of the free base of Example 40 are as shown in Table 13 below:

**Table 13: Competitive test results of different crystal forms of the free base of Example 40**

| | XRPD result | | |
|---|---|---|---|
| Solvent | Crystal form I+crystal form II | Crystal form I+crystal form III | Crystal form II+crystal form III |
| *n*-heptane | Crystal form I+crystal form II | Crystal form I+crystal form III | Crystal form II+crystal form III |
| Methyl *tert-butyl* ether | Crystal form I | Crystal form I | Crystal form I |

It can be seen from the results in the table that crystal forms I, II and III of the free base of Example 40 were almost insoluble in n-heptane, and no crystal form conversion had occurred; in methyl *tert-butyl* ether, the other two crystal forms were eventually converted to crystal form I, indicating that crystal form I of the free base is the stable crystal form.

### 6. Solid stability experiment

### 6.1 Experimental objective:

The physicochemical stability of candidate crystal forms I, II and III of the free base of Example 40 under influencing factors such as high temperature, high humidity and strong light was investigated, so as to provide a basis for the production and storage of the compound.

### 6.2 Experimental scheme:

About 5 mg of crystal form I of the free base of Example 40 was accurately weighed, and placed in a 60°C oven (sealed) or light box (5000±500 lux, sealed), or placed under room temperature/90%RH (saturated aqueous KNO₃ solution, open) or high temperature and high humidity (50°C/75%RH, saturated aqueous sodium chloride solution, open) respectively to investigate for 5 and 10 days. The related substance change of the free base was calculated by chromatographic peak area normalization method.

### 6.3 Experimental results:

1) The physicochemical stability results of crystal form I of the free base of Example 40 are as shown in Table 14 below:

**Table 14: Stability experimental results of the free base of Example 40**

| Sample name | Initial sample | 60°C Sealed | | RT/90%RH Open | | Light 5k lux Sealed | | 50°C/75%RH Open | |
|---|---|---|---|---|---|---|---|---|---|
| | 0d | 5d | 10d | 5d | 5d | 10d | 10d | 5d | 10d |
| Investigated factor | Total impur ities (%) | Total impur ities (%) | Total impur ities (%) | Total impur ities (%) | Total impur ities (%) | Total impur ities (%) | Total impur ities (%) | Total impur ities (%) | Total impur ities (%) |
| Crystal form I of the free base | 0.59 | 0.55 | 0.56 | 0.56 | 0.56 | 0.57 | 0.56 | 0.56 | 0.59 |

After crystal form I of the free base was placed under influencing factors of high temperature, high humidity and light for 10 days, the impurities did not increase, indicating that it has good physicochemical stability.

### 7. Crystal form stability experiment

### 7.1 Experimental objective:

The crystal form stability of crystal form I of the free base of Example 40 under influencing factors such as high temperature, high humidity and strong light was investigated.

### 7.2 Experimental scheme:

About 5 mg of crystal form I of the free base of Example 40 was accurately weighed, and placed under influencing factors of light, 40°C, 60°C, 25°C/RH75% or 25°C/RH90% respectively for 5, 10 and 30 days. X-ray powder diffraction assay was carried out, and the obtained data was compared with the initial data.

### 7.3 Experimental results:

1) The crystal form stability results of crystal form I of the free base of Example 40 are as shown in Table 15 below:

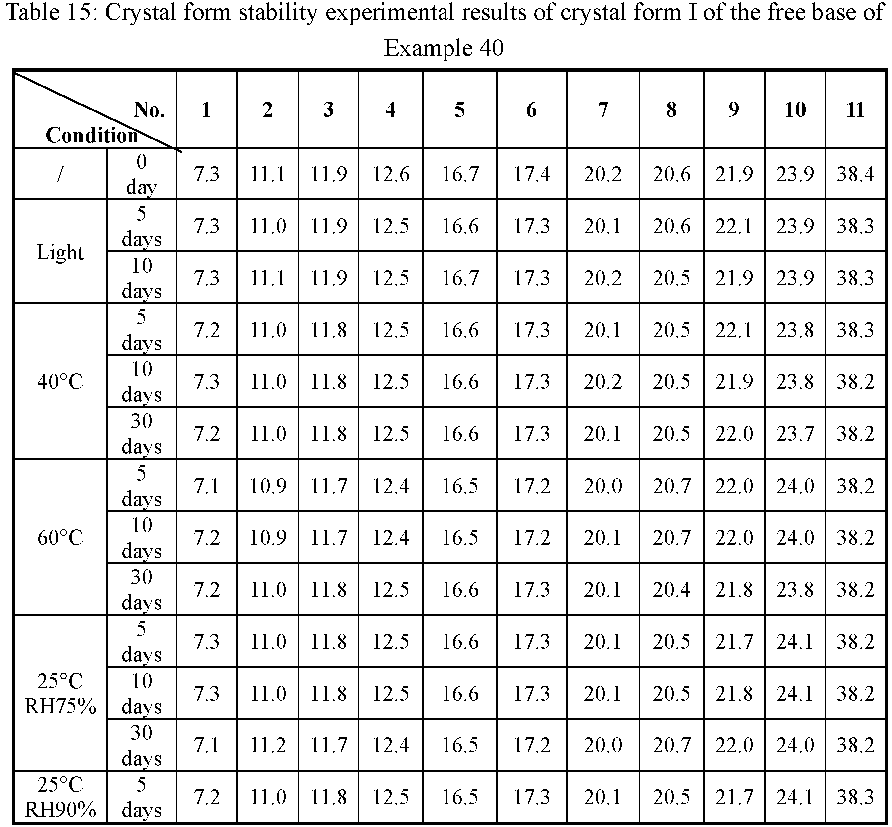

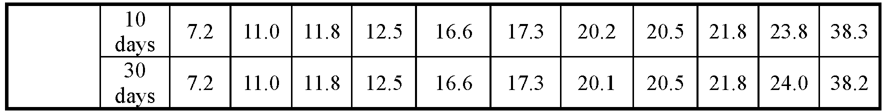

The results show that under different influencing factors, the X-ray powder diffraction assay data are consistent with the initial data. No crystal form conversion had occurred, indicating that crystal form I of the free base is stable. It should be emphasized that those skilled in the art know that the general diffraction angle error of a crystal form is within the range of ±0.2°. Individual peaks of a crystal form not within the scope of the present invention do not mean that such crystal form is a new crystal form. Those skilled in the art know that a crystal form still belongs to the crystal form of the present invention if there are differences in individual peaks.

### 8. Single crystal culture

### 8.1 Experimental objective:

A single crystal was cultured to resolve the structure of the free base of Example 40.

### 8.2 Experimental scheme:

About 20 mg of the free base of Example 40 was added into a 1.5 mL of glass flask, to which 1 mL of toluene was added. The mixture was heated to 50°C to dissolve the compound, and filtered through a 0.45 µm organic nylon membrane. The resulting filtrate was placed in a clean glass flask preheated to 50°C, and slowly cooled at room temperature. After about one day, colorless crystal particles, namely the single crystal of crystal form I of the free base of Example 40, were precipitated.

### 8.3 Experimental results:

The single crystal data of crystal form I of the free base of Example 40 is shown in Table 18 below:

**Table 16: Single crystal data of crystal form I of the free base of Example 40**

| | |
|---|---|
| C₂₆H₃₇N₃O₂ | *Dₓ* = 1.181 Mg m⁻³ |
| *Mᵣ* = 423.58 | Cu *K*□ radiation, □ = 1.54178 Å |
| Orthorhombic, *P*2₁2₁2₁ | Cell parameters from 9852 reflections |
| *a* = 9.376 (2) Å | □ = 4.6-74.4° |
| *b* = 10.533 (3) Å | □ = 0.59 mm⁻¹ |
| *c* = 24.117 (4) Å | *T* = 110 K |
| *V* = 2381.6 (9) Å³ | Block, colourless |
| *Z* = 4 | 0.19 × 0.08 × 0.03 mm |
| *F*(000) = 920 | |

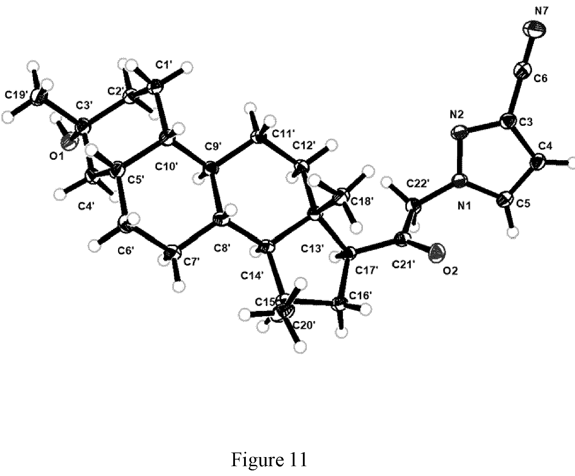

The single crystal results show that:
The unit cell structure of the single crystal of crystal form I of the free base of Example 40 is orthogonal, each unit cell contains 4 molecules, and the chiral molecular configuration optimized with single crystal characterization is consistent with the theoretical structure of chemical synthesis. The XRD pattern fitted from the single crystal data is consistent with that of crystal form I of the free base of Example 40, indicating that the single crystal of Example 40 formed at room temperature is crystal form I of the free base. The specific structure is shown in Figure 11.

## Claims

1. An acid addition salt of formula (Ia), having the following structure: 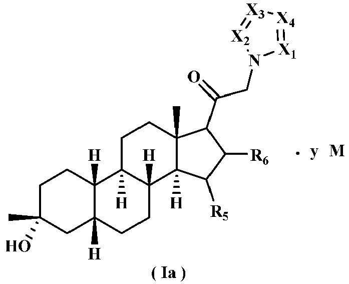 wherein:
X₁ is selected from the group consisting of CR₁ and N, and preferably N;
X₂ is selected from the group consisting of CR₂ and N;
X₃ is selected from the group consisting of CR₃ and N;
X₄ is selected from the group consisting of CR₄ and N;
R₁ and R₂ are each independently selected from the group consisting of hydrogen, deuterium, cyano, halogen, nitro, amino, C₁₋₈ alkyl, C₁₋₈ haloalkyl, C₁₋₈ alkoxy, C₁₋₈ hydroxyalkyl, cyano-substituted C₁₋₈ alkyl, C₃₋₈ cycloalkyl, cyano-substituted C₃₋₈ cycloalkyl, 3 to 8 membered heterocyclyl, C₆₋₁₀ aryl and 5 to 10 membered heteroaryl;
R₃ and R₄ are each independently selected from the group consisting of hydrogen, deuterium, cyano, halogen, nitro, amino, C₁₋₈ alkyl, C₁₋₈ haloalkyl, C₁₋₈ alkoxy, C₁₋₈ hydroxyalkyl, cyano-substituted C₁₋₈ alkyl, C₃₋₈ cycloalkyl, cyano-substituted C₃₋₈ cycloalkyl, 3 to 8 membered heterocyclyl, C₆₋₁₀ aryl, 5 to 10 membered heteroaryl, - (CH₂)ₙRₐ, -(CH₂)ₙORₐ, -(CH₂)ₙSRₐ, -(CH₂)ₙC(O)Rₐ, -S(O)Rₐ, -S(O)₂Rₐ, - S(O)(=NH)Rₐ, -C(O)ORₐ and -C(O)O(CH₂)ₙNRₐR_{b}, wherein the C₁₋₈ alkyl, C₁₋₈ haloalkyl, C₁₋₈ hydroxyalkyl, cyano-substituted C₁₋₈ alkyl, C₁₋₈ alkoxy, C₃₋₈ cycloalkyl, cyano-substituted C₃₋₈ cycloalkyl, 3 to 8 membered heterocyclyl, C₆₋₁₀ aryl and 5 to 10 membered heteroaryl can be each optionally further substituted;
R₅ is independently selected from the group consisting of C₁₋₈ alkyl, C₂₋₈ alkenyl, C₂₋₈ alkynyl, C₁₋₈ haloalkyl, C₁₋₈ hydroxyalkyl, cyano-substituted C₁₋₈ alkyl, C₁₋₈ alkoxy, C₁₋₈ alkylthio, C₃₋₈ cycloalkyl, cyano-substituted C₃₋₈ cycloalkyl, 3 to 8 membered heterocyclyl, C₆₋₁₀ aryl, 5 to 10 membered heteroaryl, -(CH₂)ₙRₐ, -(CH₂)ₙORₐ, - S(O)Rₐ, -S(O)₂Rₐ and -(CH₂)ₙNRₐR_{b}, wherein the C₁₋₈ alkyl, C₂₋₈ alkenyl, C₂₋₈ alkynyl, C₁₋₈ haloalkyl, C₁₋₈ hydroxyalkyl, cyano-substituted C₁₋₈ alkyl, C₁₋₈ alkoxy, C₃₋₈ cycloalkyl, cyano-substituted C₃₋₈ cycloalkyl, 3 to 8 membered heterocyclyl, C₆₋₁₀ aryl and 5 to 10 membered heteroaryl can be each optionally further substituted;
R₆ is hydrogen;
or, R₅ and R₆ together with the carbon atoms to which they are attached form a C₃₋₈ cycloalkyl or 3 to 8 membered heterocyclyl, wherein the C₃₋₈ cycloalkyl and 3 to 8 membered heterocyclyl can be each optionally further substituted;
Rₐ and R_{b} are each independently selected from the group consisting of hydrogen, deuterium, halogen, cyano, hydroxy, amino, nitro, C₁₋₈ alkyl, C₁₋₈ alkoxy, C₁₋₈ hydroxyalkyl, C₁₋₈ deuterated alkyl, C₁₋₈ haloalkyl, C₃₋₈ cycloalkyl, 3 to 8 membered heterocyclyl, C₆₋₁₀ aryl and 5 to 10 membered heteroaryl, wherein the C₁₋₈ alkyl, C₁₋₈ deuterated alkyl, C₁₋₈ haloalkyl, C₃₋₈ cycloalkyl, 3 to 8 membered heterocyclyl, C₆₋₁₀ aryl and 5 to 10 membered heteroaryl can be each optionally further substituted;
M is an inorganic acid or organic acid, wherein the inorganic acid is selected from the group consisting of hydrochloric acid, sulfuric acid, nitric acid, hydrobromic acid, hydrofluoric acid, hydroiodic acid and phosphoric acid, and the organic acid is selected from the group consisting of 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, capric acid, caproic acid, caprylic acid, cinnamic acid, citric acid, cyclamic acid, camphorsulfonic acid, aspartic acid, camphor acid, gluconic acid, glucuronic acid, glutamic acid, erythorbic acid, lactic acid, malic acid, mandelic acid, pyroglutamic acid, tartaric acid, lauryl sulfuric acid, dibenzoyl tartaric acid, ethane-1,2-disulfonic acid, ethanesulfonic acid, formic acid, fumaric acid, galactonic acid, gentisic acid, glutaric acid, 2-ketoglutaric acid, glycolic acid, hippuric acid, isethionic acid, lactobionic acid, ascorbic acid, asparagic acid, lauric acid, camphor acid, maleic acid, malonic acid, D-tartaric acid, pamoic acid, methanesulfonic acid, 1,5-naphthalenedisulfonic acid, naphthalene-2-sulfonic acid, nicotinic acid, oleic acid, orotic acid, oxalic acid, palmitic acid, embonic acid, propionic acid, salicylic acid, 4-aminosalicylic acid, sebacic acid, stearic acid, succinic acid, thiocyanic acid, undecylenic acid, trifluoroacetic acid, p-toluenesulfonic acid and L-malic acid; and
y is selected from the group consisting of 1, 2 and 3;
n is an integer from 0 to 6.

2. The acid addition salt according to claim 1,**characterized in that**,
R₁ and R₂ are each independently selected from the group consisting of hydrogen, fluorine, chlorine, cyano, methyl and cyclopropyl;
R₃ and R₄ are each independently selected from the group consisting of hydrogen, halogen, cyano, nitro, C₁₋₃ alkyl, cyano-substituted C₁₋₃ alkyl, halogen-substituted C₁₋₃ alkyl, C ₁₋₃ hydroxyalkyl, C₃₋₆ cycloalkyl, cyano-substituted C₃₋₆ cycloalkyl, oxazolyl, - C(O)Rₐ, -SC₁₋₃ alkyl, -S(O)C₁₋₃ alkyl, -S(O)₂C₁₋₃ alkyl, -C(O)OC₁₋₃ alkyl, - S(O)(=NH)C₁₋₃ alkyl and -C(O)O(CH₂)ₙN(CH₃)C₁₋₃ alkyl, and preferably hydrogen, fluorine, chlorine, cyano, nitro, methyl, trifluoromethyl, cyclopropyl, cyano-substituted cyclopropyl, cyano-substituted isopropyl, hydroxyisopropyl, oxazolyl, - C(O)Rₐ, -SCH₃, -S(O)CH₃, -S(O)₂CH₃, -C(O)OCH₂CH₃, -S(O)(=NH)CH₃ and - C(O)O(CH₂)₂NCH₃(CH₃);
R₅ is selected from the group consisting of C₁₋₃ alkyl, C₁₋₃ haloalkyl, C ₁₋₃ alkoxy, C₁₋₃ hydroxyalkyl, cyano-substituted C₁₋₃ alkyl, C₃₋₆ cycloalkyl, cyano-substituted C₃₋₆ cycloalkyl, 3 to 6 membered nitrogen-containing heterocyclyl, C₆₋₁₀ aryl, 5 to 10 membered heteroaryl, C ₁₋₃ alkylthio, -(CH₂)ₙRₐ, -(CH₂)ₙORₐ, -S(O)₂Rₐ and - (CH₂)ₙN(Rₐ)₂, and preferably hydrogen, fluorine, chlorine, cyano, methyl, ethyl, isopropyl, fluoromethyl, trifluoromethyl, methoxy, cyclopropyl, cyclobutyl, methyl-substituted pyrazolyl, phenyl, -SCH₃, -(CH₂)ₙRₐ, -NC(CH₃)₂, -S(O)₂C(CH₃)₂ and - CH₂N(CH₃)₂;
R₆ is hydrogen;
or, R₅ and R₆ together with the carbon atoms to which they are attached form a C₃₋₆ cycloalkyl, and preferably cyclopropyl, wherein the cyclopropyl is optionally substituted by halogen, and preferably by fluorine;
Rₐ is selected from the group consisting of hydrogen, nitro, C₁₋₃ alkyl, azetidinyl and pyrrolidinyl, wherein the azetidinyl and pyrrolidinyl are each optionally substituted by halogen, and preferably by fluorine;
M is selected from the group consisting of hydrochloric acid, sulfuric acid, methanesulfonic acid, p-toluenesulfonic acid, hydrobromic acid, nitric acid and 1,5-naphthalenedisulfonic acid, and preferably methanesulfonic acid;
y is 1 or 2;
n is independently selected from the group consisting of 0, 1 and 2.

3. The acid addition salt according to claim 1 or 2, having a structure as shown in formula (IIa): 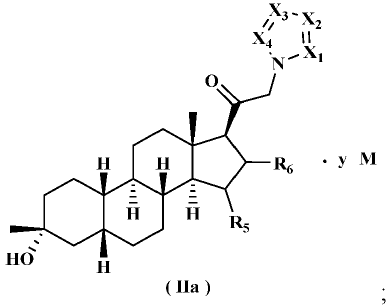 or, having a structure as shown in formula (IIIa): 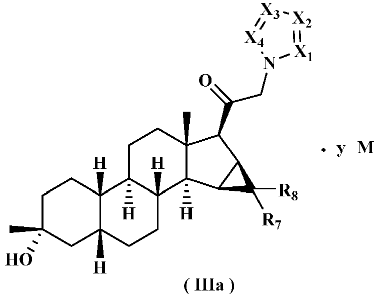 wherein:
R₇ and R₈ are each independently selected from the group consisting of hydrogen, fluorine, chlorine, bromine and methyl, and preferably hydrogen and fluorine;
or, having a structure as shown in formula (IVa): 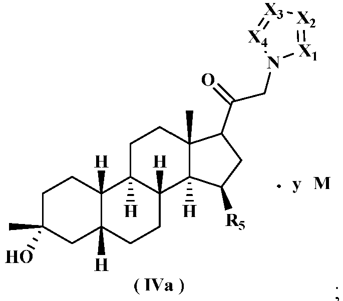
or, having a structure as shown in formula (Va):

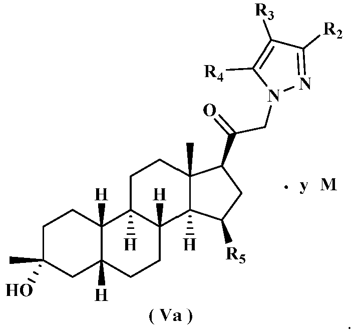

4. The acid addition salt according to any one of claims 1 to 3, **characterized in that** the structure thereof is as follows:

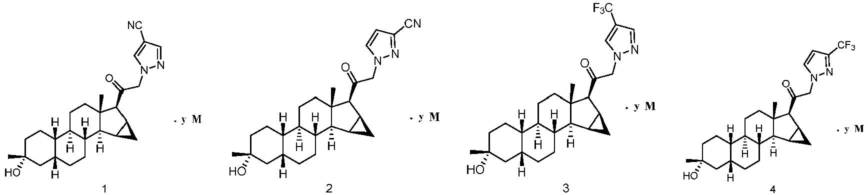

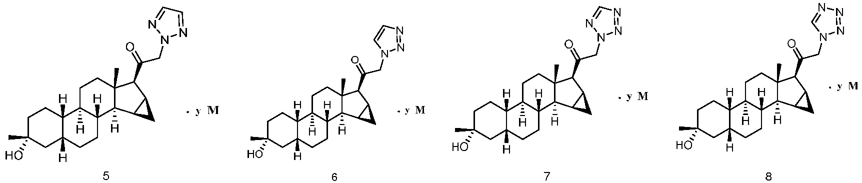

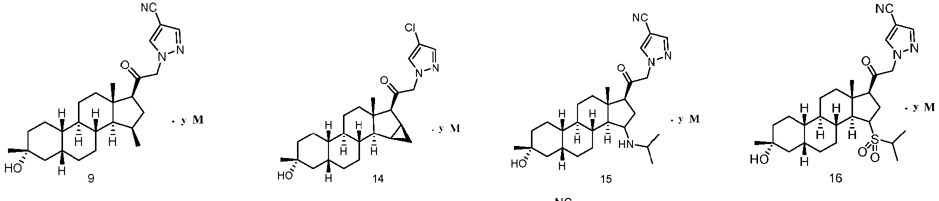

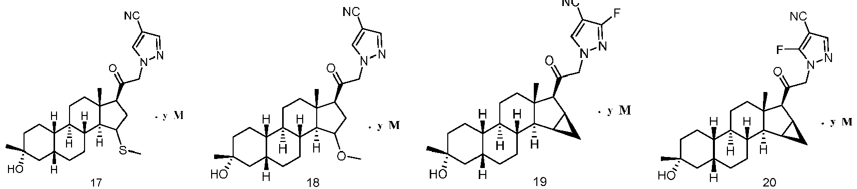

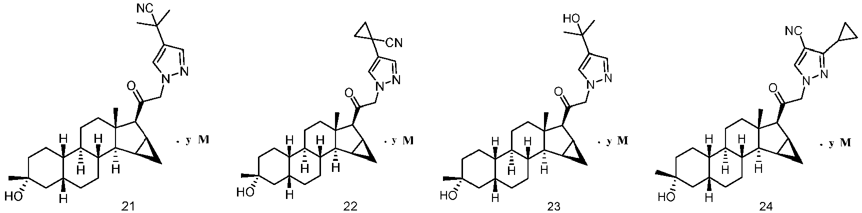

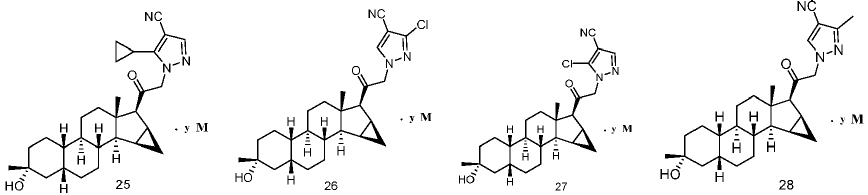

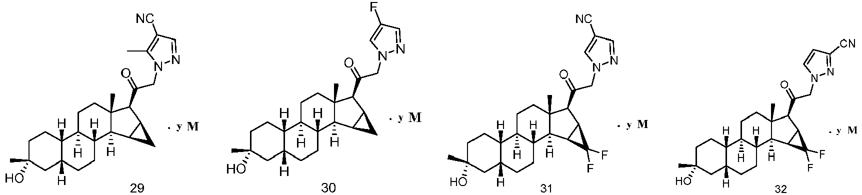

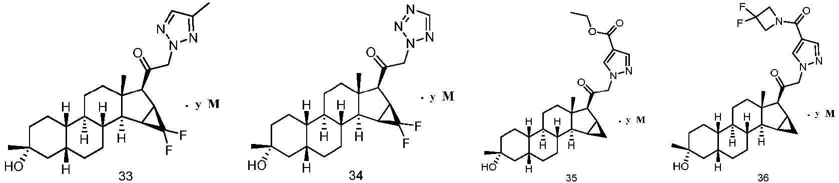

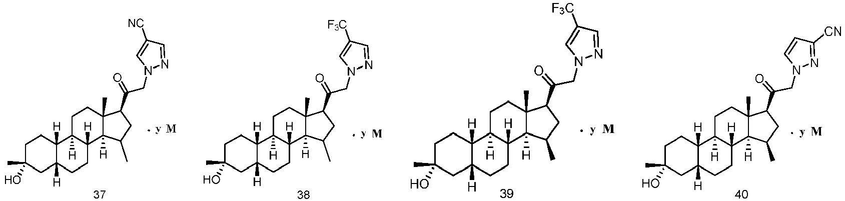

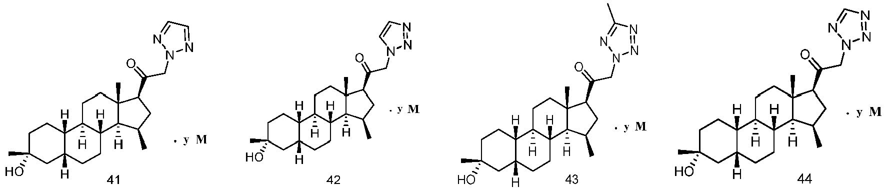

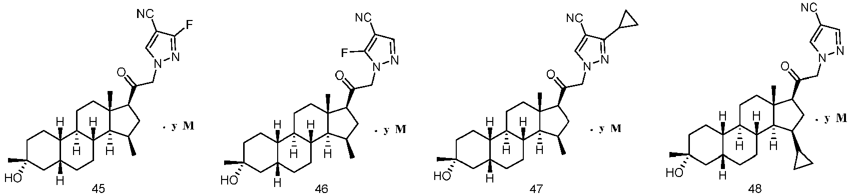

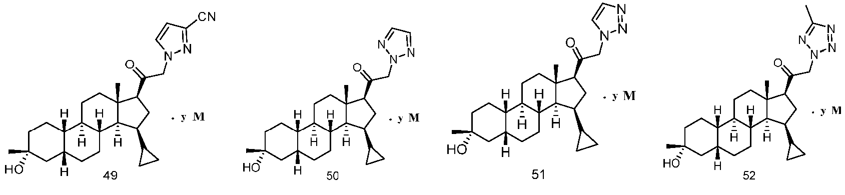

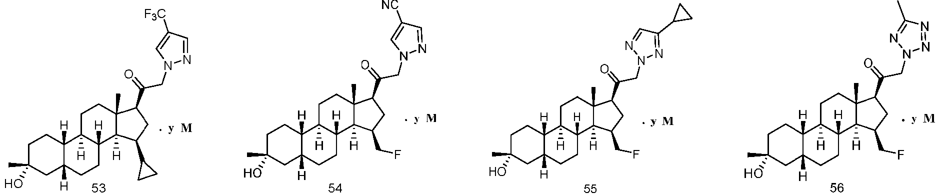

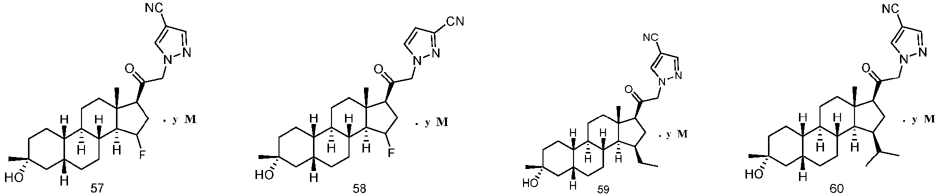

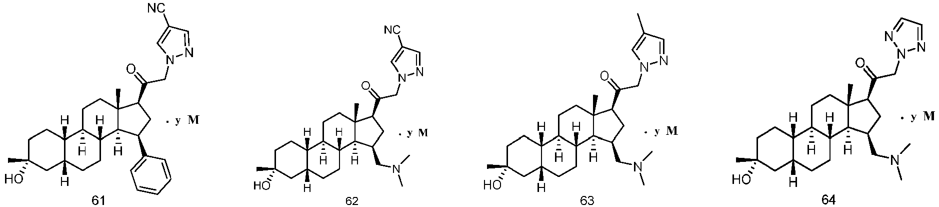

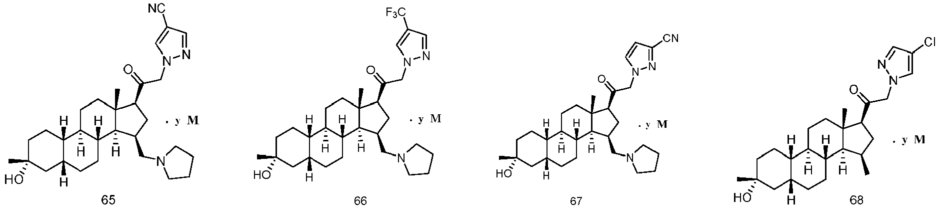

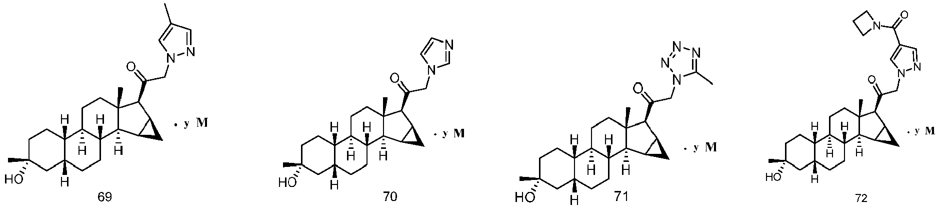

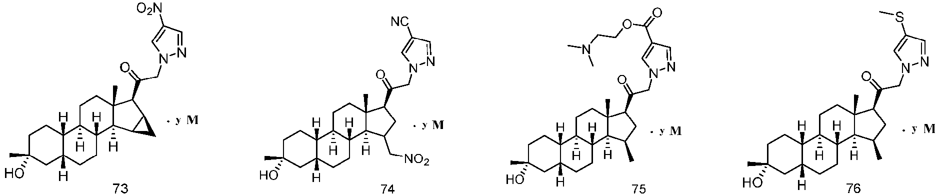

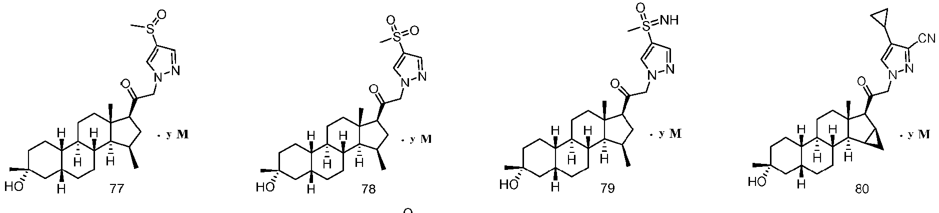

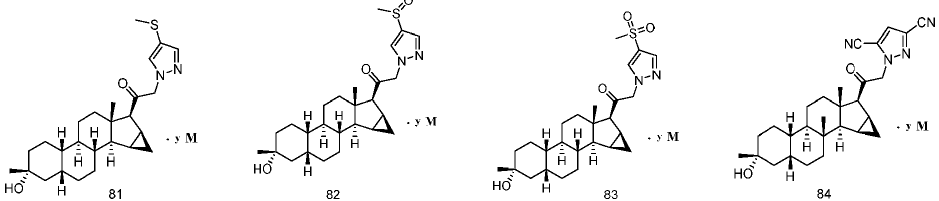

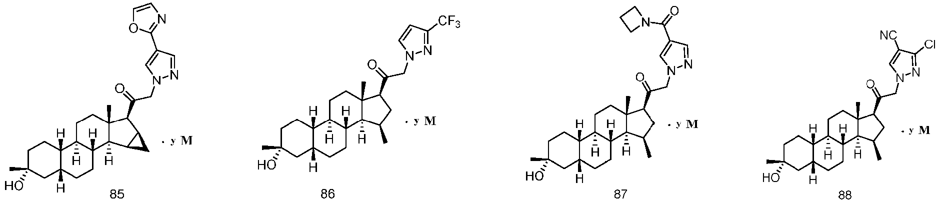

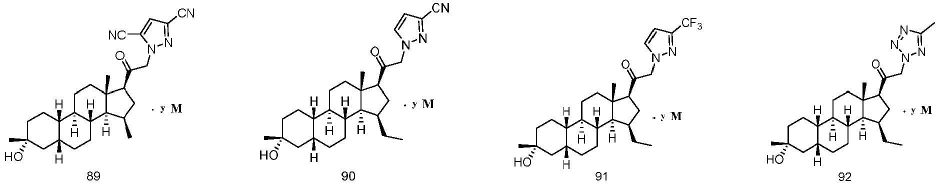

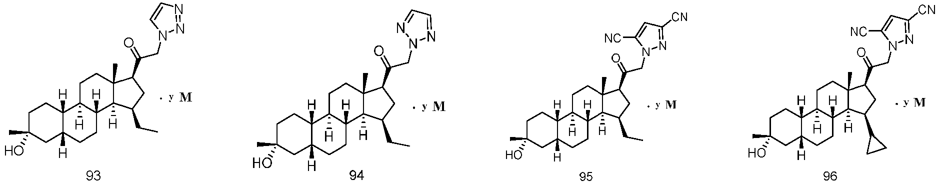

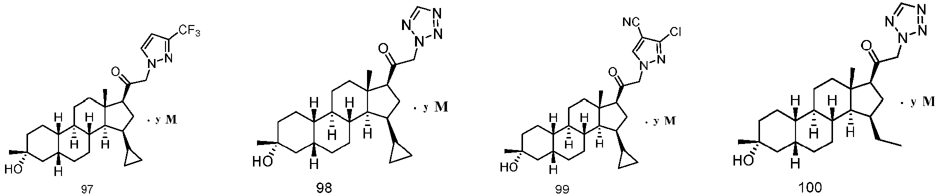

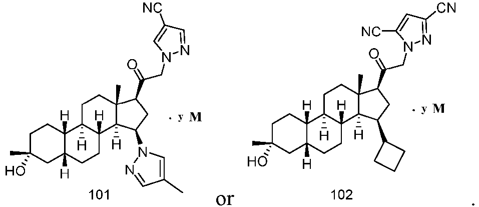

5. The acid addition salt according to claim 4, **characterized in that** the structure thereof is as follows:

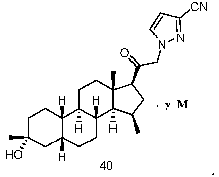

6. The acid addition salt according to claim 4, **characterized in that** the structure thereof is as follows: 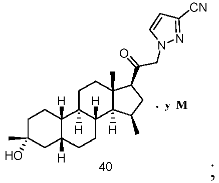 wherein M is methanesulfonic acid and y is 1.

7. The acid addition salt according to any one of claims 1 to 6, **characterized in that** the acid addition salt is crystalline or amorphous.

8. A crystal form of a compound of formula (VI), having the following structure: 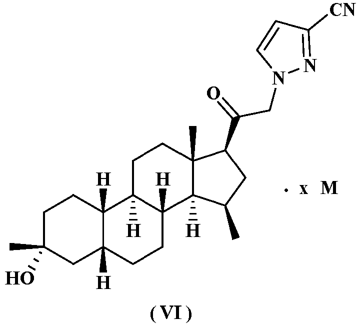
M is an inorganic acid or organic acid, wherein the inorganic acid is selected from the group consisting of hydrochloric acid, sulfuric acid, nitric acid, hydrobromic acid, hydrofluoric acid, hydroiodic acid and phosphoric acid, and the organic acid is selected from the group consisting of 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, capric acid, caproic acid, caprylic acid, cinnamic acid, citric acid, cyclamic acid, camphorsulfonic acid, aspartic acid, camphor acid, gluconic acid, glucuronic acid, glutamic acid, erythorbic acid, lactic acid, malic acid, mandelic acid, pyroglutamic acid, tartaric acid, lauryl sulfuric acid, dibenzoyl tartaric acid, ethane-1,2-disulfonic acid, ethanesulfonic acid, formic acid, fumaric acid, galactonic acid, gentisic acid, glutaric acid, 2-ketoglutaric acid, glycolic acid, hippuric acid, isethionic acid, lactobionic acid, ascorbic acid, asparagic acid, lauric acid, camphor acid, maleic acid, malonic acid, D-tartaric acid, pamoic acid, methanesulfonic acid, 1,5-naphthalenedisulfonic acid, naphthalene-2-sulfonic acid, nicotinic acid, oleic acid, orotic acid, oxalic acid, palmitic acid, embonic acid, propionic acid, salicylic acid, 4-aminosalicylic acid, sebacic acid, stearic acid, succinic acid, thiocyanic acid, undecylenic acid, trifluoroacetic acid, p-toluenesulfonic acid and L-malic acid; and
x is 0, 1 or 2.

9. The crystal form according to claim 8, **characterized in that**, the compound of formula (VI) is a hydrate or an anhydrate.

10. The crystal form according to any one of claims 8 to 9, **characterized in that**,
wherein x is 0, the crystal form is crystal form I of free base, the X-ray powder diffraction pattern thereof has a diffraction peak at 2θ (±0.2°) of 16.7, 12.6 and 14.6; preferably has diffraction peaks at 2θ (±0.2°) of 7.3, 13.2, 17.4, 19.4, 20.2 and 20.6, and more preferably has diffraction peaks at 2θ (±0.2°) of 9.2, 11.1, 11.9, 19.6, 22.3 and 25.5;
or,
wherein x is 0, the crystal form is crystal form II of free base, the X-ray powder diffraction pattern thereof has diffraction peaks at 2θ (±0.2°) of 11.7, 13.4, 13.6, 16.6 and 18.9, preferably further has diffraction peaks at 2θ (±0.2°) of 9.5, 10.1, 14.7 and 19.3, more preferably further has diffraction peaks at 2θ (±0.2°) of 19.6, 20.6, 20.9, 21.6, 22.1, 22.5, 22.7 and 24.4, and further preferably, the X-ray powder diffraction pattern thereof is substantially as shown in Figure 3;
or,
wherein x is 0, the crystal form is crystal form III of free base, the X-ray powder diffraction pattern thereof has diffraction peaks at 2θ (±0.2°) of 10.0, 11.7, 13.7, 16.6, 18.9 and 19.2, preferably further has diffraction peaks at 2θ (±0.2°) of 13.4, 19.6, 20.6, 20.9, 22.0, 22.7, 23.4 and 25.6, and further preferably, the X-ray powder diffraction pattern thereof is substantially as shown in Figure 5;
or,
wherein M is methanesulfonic acid, x is 1, the X-ray powder diffraction pattern thereof has diffraction peaks at 2θ (±0.2°) of 12.5, 13.5, 19.4 and 19.9, preferably further has diffraction peaks at 2θ (±0.2°) of 15.1, 15.8, 16.5, 17.3, 18.7 and 23.1, more preferably further has diffraction peaks at 2θ (±0.2°) of 11.1, 11.5, 13.9, 18.5, 21.3, 21.7, 26.5 and 28.9, and further preferably, the X-ray powder diffraction pattern thereof is substantially as shown in Figure 7.

11. The crystal form according to any one of claims 8 to 10, **characterized in that**, the X-ray powder diffraction pattern of the crystal form I is substantially as shown in Figure 1.

12. The crystal form according to any one of claims 8 to 10, **characterized in that**,
the DSC spectrum of the crystal form I has an endothermic peak at 151.4±0.5°C;
or, the DSC spectrum of the crystal form II has an endothermic peak at 193.5±0.5°C;
or, the DSC spectrum of the crystal form III has an endothermic peak at 206.4±0.5°C.

13. A method for preparing the crystal form of the compound according to any one of claims 8 to 12, specifically comprising the following steps of:
1) weighing an appropriate amount of the free base or the acid addition salt, followed by adding a good solvent and heating the mixture until dissolved;
2) after stirring for several hours, adding dropwise a poor solvent until turbidity appears;
3) stirring and cooling the mixture, followed by precipitating a crystal to obtain the target product;
preferably:
the good solvent is selected from the group consisting of methanol, ethanol, ethyl acetate, dichloromethane, acetone, toluene, acetonitrile, tetrahydrofuran, n-heptane, methyl tert-butyl ether, isopropyl ether and N,N-dimethylformamide; and preferably 88% acetone, tetrahydrofuran and ethanol; and
the poor solvent is selected from the group consisting of n-heptane, methyl *tert-butyl* ether and isopropyl ether; and preferably n-heptane and methyl tert-butyl ether.

14. A pharmaceutical composition, comprising a therapeutically effective amount of the acid addition salt of formula (Ia) according to any one of claims 1 to 7 or a therapeutically effective amount of the crystal form of the compound of formula (I) according to any one of claims 8 to 12, and one or more pharmaceutically acceptable carriers.

15. The pharmaceutical composition according to claim 14, **characterized in that** the pharmaceutical composition is an injection or an oral preparation, and preferably a tablet or a capsule.

16. The pharmaceutical composition according to claim 14, **characterized in that** the unit dose is 5 to 200 mg, preferably 10 to 100 mg, and more preferably 10 to 50 mg.

17. The acid addition salt of the compound of formula (Ia) according to any one of claims 1 to 7, the crystal form of the compound of formula (I) according to any one of claims 8 to 12 or the pharmaceutical composition according to any one of claims 14 to 16 for use in treating a related disease mediated by GABA_{A} receptor regulator, wherein the related disease mediated by GABA_{A} receptor regulator preferably is a Central Nervous System (CNS)-related disease; the CNS-related disease is selected from the group consisting of sleep disorder, mood disorder, schizophrenia spectrum disorder, spasmodic disorder, memory disorder, cognitive disorder, dyskinesia, personality disorder, autism spectrum disorder, pain, traumatic brain injury, vascular disease, substance abuse disorder and/or withdrawal syndrome or tinnitus.

## Patentansprüche

1. Säureadditionssalz der Formel (Ia) mit der folgenden Struktur: 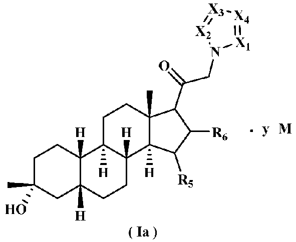 wobei:
X₁ aus der Gruppe bestehend aus CR₁ und N und vorzugsweise N ausgewählt ist;
X₂ aus der Gruppe bestehend aus CR₂ und N ausgewählt ist;
X₃ aus der Gruppe bestehend aus CR₃ und N ausgewählt ist;
X₄ aus der Gruppe bestehend aus CR₄ und N ausgewählt ist;
R₁ und R₂ jeweils unabhängig aus der Gruppe bestehend aus Wasserstoff, Deuterium, Cyano, Halogen, Nitro, Amino, C₁₋₈-Alkyl, C₁₋₈-Halogenalkyl, C₁₋₈-Alkoxy, C₁₋₈-Hydroxyalkyl, cyanosubstituiertem C₁₋₈-Alkyl, C₃₋₈-Cycloalkyl, cyanosubstituiertem C₃₋₈-Cycloalkyl, 3- bis 8-gliedrigem Heterocyclyl, C₆₋₁₀-Aryl und 5- bis 10-gliedrigem Heteroaryl ausgewählt sind;
R₃ und R₄ jeweils unabhängig aus der Gruppe bestehend aus Wasserstoff, Deuterium, Cyano, Halogen, Nitro, Amino, C₁₋₈-Alkyl, C₁₋₈-Halogenalkyl, C₁₋₈-Alkoxy, C₁₋₈-Hydroxyalkyl, cyanosubstituiertem C₁₋₈-Alkyl, C₃₋₈-Cycloalkyl, cyanosubstituiertem C₃₋₈-Cycloalkyl, 3- bis 8-gliedrigem Heterocyclyl, C₆₋₁₀-Aryl, 5- bis 10-gliedrigem Heteroaryl, -(CH₂)ₙR_{a,} - (CH₂)ₙOR_{a,} -(CH₂)ₙSR_{a,} -(CH₂)ₙC(O)R_{a,} -S(O)R_{a,} -S(O)₂R_{a,} -S(O)(=NH)R_{a,} - C(O)ORₐ und -C(O)O(CH₂)ₙNRₐR_{b} ausgewählt sind, wobei das C₁₋₈-Alkyl, C₁₋₈ -Halogenalkyl, C₁₋₈-Hydroxyalkyl, cyanosubstituierte C₁₋₈-Alkyl, C₁₋₈-Alkoxy, C₃₋₈-Cycloalkyl, cyanosubstituierte C₃₋₈-Cycloalkyl, 3- bis 8-gliedrige Heterocyclyl, C₆₋₁₀-Aryl und 5- bis 10-gliedrige Heteroaryl jeweils gegebenenfalls weiter substituiert sein können;
R₅ unabhängig aus der Gruppe bestehend aus C₁₋₈-Alkyl, C₂₋₈-Alkenyl, C₂₋₈-Alkinyl, C₁₋₈-Halogenalkyl, C₁₋₈-Hydroxyalkyl, cyanosubstituiertem C₁₋₈-Alkyl, C₁₋₈-Alkoxy, C₁₋₈-Alkylthio, C₃₋₈-Cycloalkyl, cyanosubstituiertem C₃₋₈-Cycloalkyl, 3- bis 8-gliedrigem Heterocyclyl, C₆₋₁₀-Aryl, 5- bis 10-gliedrigem Heteroaryl, -(CH₂)ₙR_{a,} -(CH₂)ₙOR_{a,} -S(O)R_{a,} -S(O)₂Rₐ und - (CH₂)ₙNRₐR_{b} ausgewählt ist, wobei das C₁₋₈-Alkyl, C₂₋₈-Alkenyl, C₂₋₈-Alkinyl, C₁₋₈-Halogenalkyl, C₁₋₈-Hydroxyalkyl, cyanosubstituierte C₁₋₈-Alkyl, C₁₋₈-Alkoxy, C₃₋₈-Cycloalkyl, cyanosubstituierte C₃₋₈-Cycloalkyl, 3-bis 8-gliedrige Heterocyclyl, C₆₋₁₀-Aryl und 5- bis 10-gliedrige Heteroaryl jeweils gegebenenfalls weiter substituiert sein können;
R₆ für Wasserstoff steht;
oder R₅ und R₆ zusammen mit den Kohlenstoffatomen, an die sie gebunden sind, ein C₃₋₈-Cycloalkyl oder 3- bis 8-gliedriges Heterocyclyl bilden, wobei das C₃₋₈-Cycloalkyl und 3- bis 8-gliedrige Heterocyclyl jeweils gegebenenfalls weiter substituiert sein können;
Rₐ und R_{b} jeweils unabhängig aus der Gruppe bestehend aus Wasserstoff, Deuterium, Halogen, Cyano, Hydroxy, Amino, Nitro, C₁₋₈-Alkyl, C₁₋₈-Alkoxy, C₁₋₈-Hydroxyalkyl, deuteriertem C₁₋₈-Alkyl, C₁₋₈-Halogenalkyl, C₃₋₈-Cycloalkyl, 3- bis 8-gliedrigem Heterocyclyl, C₆₋₁₀-Aryl und 5- bis 10-gliedrigem Heteroaryl ausgewählt sind, wobei das C₁₋₈-Alkyl, deuterierte C₁₋₈-Alkyl, C₁₋₈-Halogenalkyl, C₃₋₈-Cycloalkyl, 3- bis 8-gliedrige Heterocyclyl, C₆₋₁₀-Aryl und 5- bis 10-gliedrige Heteroaryl jeweils gegebenenfalls weiter substituiert sein können;
M für eine anorganische Säure oder organische Säure steht, wobei die anorganische Säure aus der Gruppe bestehend aus Chlorwasserstoffsäure, Schwefelsäure, Salpetersäure, Bromwasserstoffsäure, Fluorwasserstoffsäure, Iodwasserstoffsäure und Phosphorsäure ausgewählt ist und die organische Säure aus der Gruppe bestehend aus 2,5-Dihydroxybenzoesäure, 1-Hydroxy-2-naphthoesäure, Essigsäure, Dichloressigsäure, Trichloressigsäure, Acetohydroxamsäure, Adipinsäure, Benzolsulfonsäure, 4-Chlorbenzolsulfonsäure, Benzoesäure, 4-Acetylaminobenzoesäure, 4-Aminobenzoesäure, Caprinsäure, Capronsäure, Caprylsäure, Zimtsäure, Citronensäure, Cyclamsäure, Camphersulfonsäure, Asparaginsäure, Camphersäure, Gluconsäure, Glucuronsäure, Glutaminsäure, Erythorbinsäure, Milchsäure, Äpfelsäure, Mandelsäure, Pyroglutaminsäure, Weinsäure, Laurylschwefelsäure, Dibenzoylweinsäure, Ethan-1,2-disulfonsäure, Ethansulfonsäure, Ameisensäure, Fumarsäure, Galactonsäure, Gentisinsäure, Glutarsäure, 2-Ketoglutarsäure, Glykolsäure, Hippursäure, Isethionsäure, Lactobionsäure, Ascorbinsäure, Asparaginsäure, Laurinsäure, Camphersäure, Maleinsäure, Malonsäure, D-Weinsäure, Pamoasäure, Methansulfonsäure, 1,5-Naphthalindisulfonsäure, Naphthalin-2-sulfonsäure, Nicotinsäure, Ölsäure, Orotsäure, Oxalsäure, Palmitinsäure, Embonsäure, Propionsäure, Salicylsäure, 4-Aminosalicylsäure, Sebacinsäure, Stearinsäure, Bernsteinsäure, Thiocyansäure, Undecylensäure, Trifluoressigsäure, p-Toluolsulfonsäure und L-Äpfelsäure ausgewählt ist; und
y aus der Gruppe bestehend aus 1, 2 und 3 ausgewählt ist;
n für eine ganze Zahl von 0 bis 6 steht.

2. Säureadditionssalz nach Anspruch 1, **dadurch gekennzeichnet, dass**
R₁ und R₂ jeweils unabhängig aus der Gruppe bestehend aus Wasserstoff, Fluor, Chlor, Cyano, Methyl und Cyclopropyl ausgewählt sind;
R₃ und R₄ jeweils unabhängig aus der Gruppe bestehend aus Wasserstoff, Halogen, Cyano, Nitro, C₁₋₃-Alkyl, cyanosubstituiertem C₁₋₃-Alkyl, halogensubstituiertem C₁₋₃-Alkyl, C₁₋₃-Hydroxyalkyl, C₃₋₆-Cycloalkyl, cyanosubstituiertem C₃₋₆-Cycloalkyl, Oxazolyl, -C(O)R_{a,} -SC₁₋₃-Alkyl, - S(O)C₁₋₃-Alkyl, -S(O)₂C₁₋₃-Alkyl, -C(O)OC₁₋₃-Alkyl, -S(O)(=NH)C₁₋₃-Alkyl und -C(O)O(CH₂)ₙN(CH₃)C₁₋₃-Alkyl und vorzugsweise Wasserstoff, Fluor, Chlor, Cyano, Nitro, Methyl, Trifluormethyl, Cyclopropyl, cyanosubstituiertem Cyclopropyl, cyanosubstituiertem Isopropyl, Hydroxyisopropyl, Oxazolyl, -C(O)R_{a,} -SCH₃, -S(O)CH₃, -S(O)₂CH₃, - C(O)OCH₂CH₃, -S(O)(=NH)CH₃ und -C(O)O(CH₂)₂NCH₃(CH₃) ausgewählt sind;
R₅ aus der Gruppe bestehend aus C₁₋₃-Alkyl, C₁₋₃-Halogenalkyl, C₁₋₃-Alkoxy, C₁₋₃-Hydroxyalkyl, cyanosubstituiertem C₁₋₃-Alkyl, C₃₋₆-Cycloalkyl, cyanosubstituiertem C₃₋₆-Cycloalkyl, 3- bis 6-gliedrigem stickstoffhaltigem Heterocyclyl, C₆₋₁₀-Aryl, 5- bis 10-gliedrigem Heteroaryl, C₁₋₃-Alkylthio, - (CH₂)ₙR_{a,} -(CH₂)ₙOR_{a,} -S(O)₂Rₐ und -(CH₂)ₙN(Rₐ)₂ und vorzugsweise Wasserstoff, Fluor, Chlor, Cyano, Methyl, Ethyl, Isopropyl, Fluormethyl, Trifluormethyl, Methoxy, Cyclopropyl, Cyclobutyl, methylsubstituiertem Pyrazolyl, Phenyl, -SCH₃, -(CH₂)ₙRₐ, -NC(CH₃)₂, -S(O)₂C(CH₃)₂ und - CH₂N(CH₃)₂ ausgewählt ist;
R₆ für Wasserstoff steht;
oder R₅ und R₆ zusammen mit den Kohlenstoffatomen, an die sie gebunden sind, ein C₃₋₆-Cycloalkyl und vorzugsweise Cyclopropyl bilden, wobei das Cyclopropyl gegebenenfalls durch Halogen und vorzugsweise durch Fluor substituiert ist;
Rₐ aus der Gruppe bestehend aus Wasserstoff, Nitro, C₁₋₃-Alkyl, Azetidinyl und Pyrrolidinyl ausgewählt ist, wobei das Azetidinyl und Pyrrolidinyl jeweils gegebenenfalls durch Halogen und vorzugsweise durch Fluor substituiert sind;
M aus der Gruppe bestehend aus Chlorwasserstoffsäure, Schwefelsäure, Methansulfonsäure, p-Toluolsulfonsäure, Bromwasserstoffsäure, Salpetersäure und 1,5-Naphthalindisulfonsäure und vorzugsweise Methansulfonsäure ausgewählt ist;
y für 1 oder 2 steht;
n unabhängig aus der Gruppe bestehend aus 0, 1 und 2 ausgewählt ist.

3. Säureadditionssalz nach Anspruch 1 oder 2 mit einer Struktur gemäß Formel (IIa): 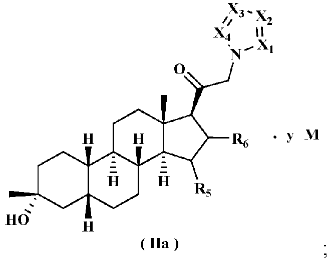 oder mit einer Struktur gemäß Formel (IIIa): 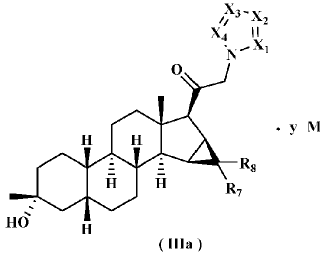 wobei:
R₇ und R₈ jeweils unabhängig aus der Gruppe bestehend aus Wasserstoff, Fluor, Chlor, Brom und Methyl und vorzugsweise Wasserstoff und Fluor ausgewählt sind;
oder mit einer Struktur gemäß Formel (IVa): 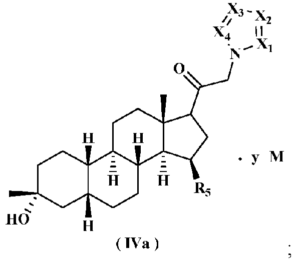
oder mit einer Struktur gemäß Formel (Va):

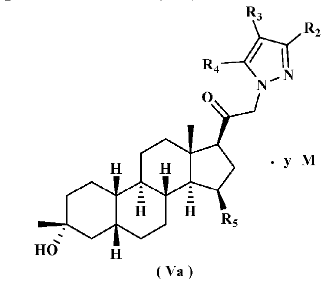

4. Säureadditionssalz nach einem der Ansprüche 1 bis 3, **dadurch gekennzeichnet, dass** es die folgende Struktur aufweist:

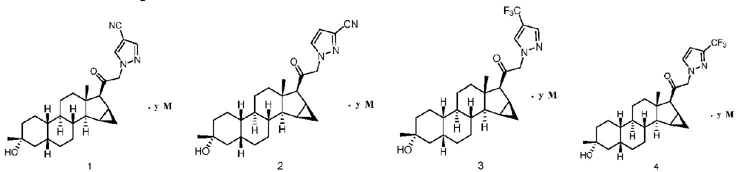

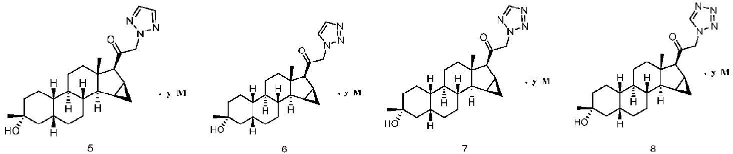

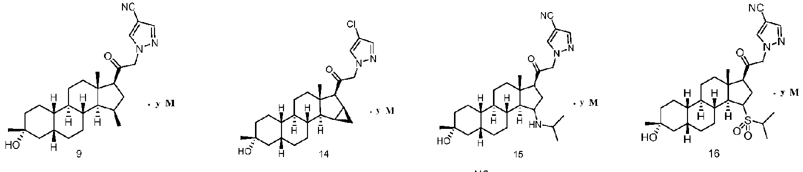

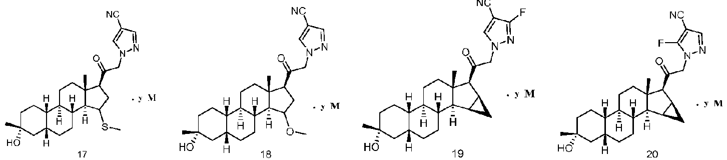

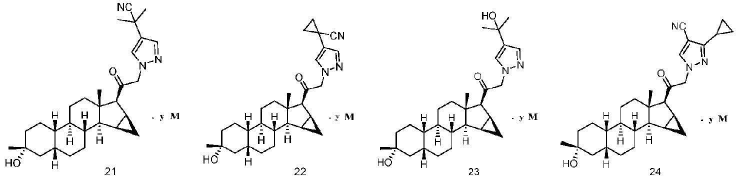

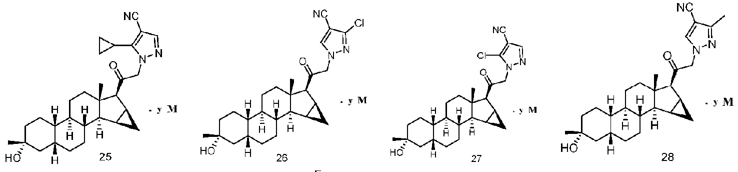

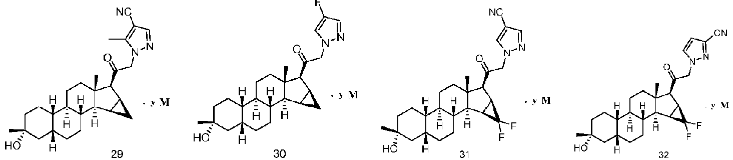

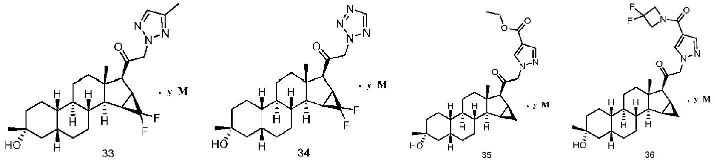

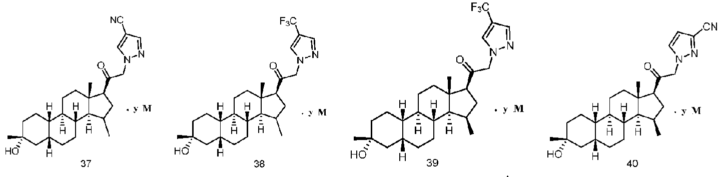

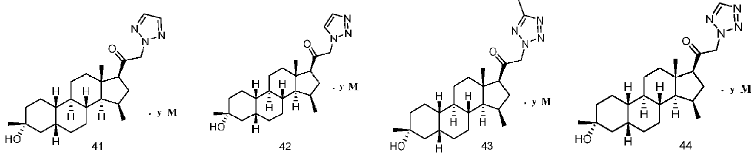

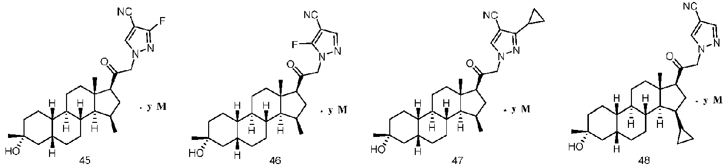

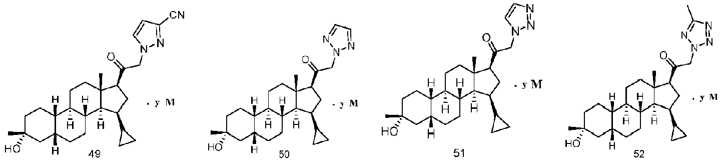

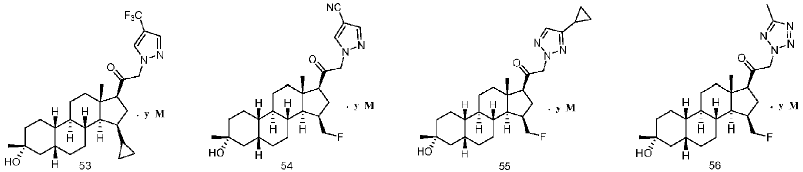

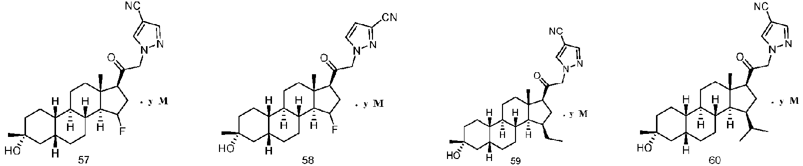

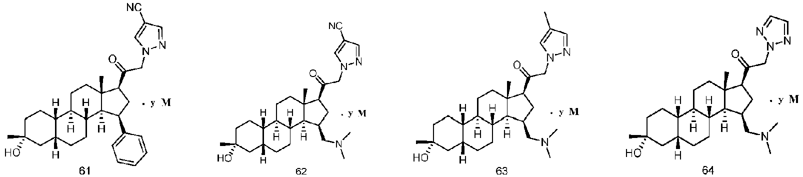

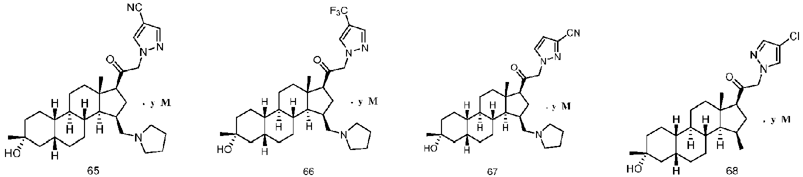

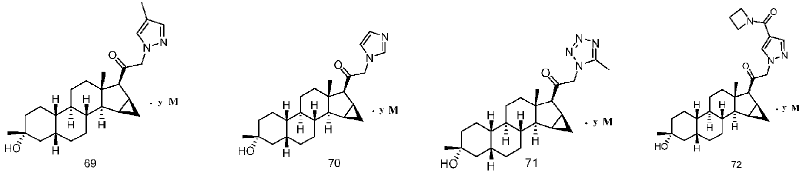

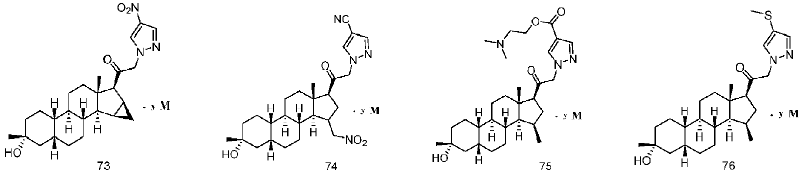

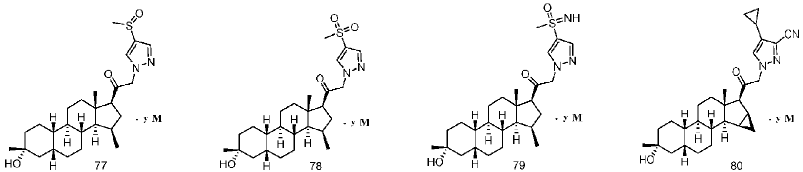

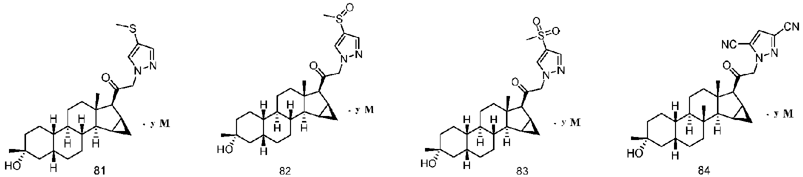

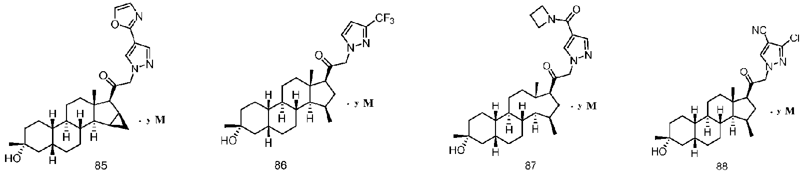

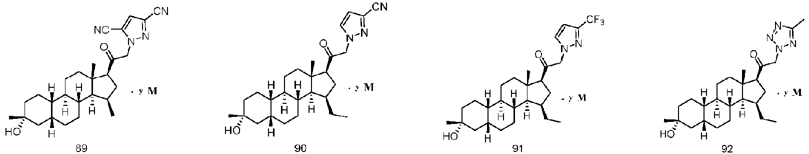

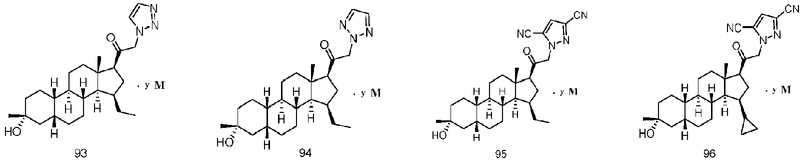

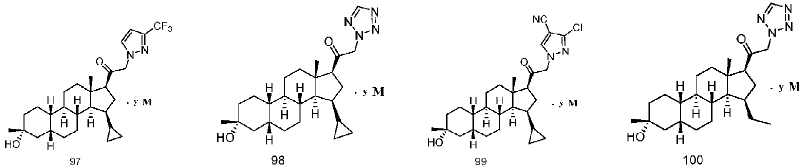

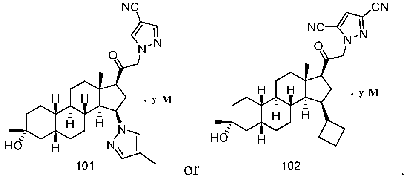

5. Säureadditionssalz nach Anspruch 4, **dadurch gekennzeichnet, dass** es die folgende Struktur aufweist:

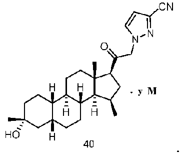

6. Säureadditionssalz nach Anspruch 4, **dadurch gekennzeichnet, dass** es die folgende Struktur aufweist: 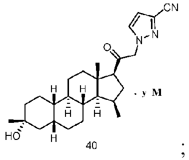 wobei M für Methansulfonsäure steht und y für 1 steht.

7. Säureadditionssalz nach einem der Ansprüche 1 bis 6, **dadurch gekennzeichnet, dass** das Säureadditionssalz kristallin oder amorph ist.

8. Kristallform einer Verbindung der Formel (VI) mit der folgenden Struktur: 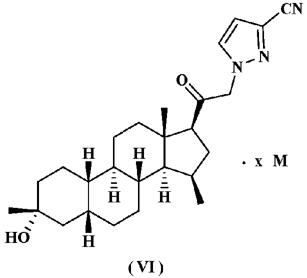
M für eine anorganische Säure oder organische Säure steht, wobei die anorganische Säure aus der Gruppe bestehend aus Chlorwasserstoffsäure, Schwefelsäure, Salpetersäure, Bromwasserstoffsäure, Fluorwasserstoffsäure, Iodwasserstoffsäure und Phosphorsäure ausgewählt ist und die organische Säure aus der Gruppe bestehend aus 2,5-Dihydroxybenzoesäure, 1-Hydroxy-2-naphthoesäure, Essigsäure, Dichloressigsäure, Trichloressigsäure, Acetohydroxamsäure, Adipinsäure, Benzolsulfonsäure, 4-Chlorbenzolsulfonsäure, Benzoesäure, 4-Acetylaminobenzoesäure, 4-Aminobenzoesäure, Caprinsäure, Capronsäure, Caprylsäure, Zimtsäure, Citronensäure, Cyclamsäure, Camphersulfonsäure, Asparaginsäure, Camphersäure, Gluconsäure, Glucuronsäure, Glutaminsäure, Erythorbinsäure, Milchsäure, Äpfelsäure, Mandelsäure, Pyroglutaminsäure, Weinsäure, Laurylschwefelsäure, Dibenzoylweinsäure, Ethan-1,2-disulfonsäure, Ethansulfonsäure, Ameisensäure, Fumarsäure, Galactonsäure, Gentisinsäure, Glutarsäure, 2-Ketoglutarsäure, Glykolsäure, Hippursäure, Isethionsäure, Lactobionsäure, Ascorbinsäure, Asparaginsäure, Laurinsäure, Camphersäure, Maleinsäure, Malonsäure, D-Weinsäure, Pamoasäure, Methansulfonsäure, 1,5-Naphthalindisulfonsäure, Naphthalin-2-sulfonsäure, Nicotinsäure, Ölsäure, Orotsäure, Oxalsäure, Palmitinsäure, Embonsäure, Propionsäure, Salicylsäure, 4-Aminosalicylsäure, Sebacinsäure, Stearinsäure, Bernsteinsäure, Thiocyansäure, Undecylensäure, Trifluoressigsäure, p-Toluolsulfonsäure und L-Äpfelsäure ausgewählt ist; und
x für 0, 1 oder 2 steht.

9. Kristallform nach Anspruch 8, **dadurch gekennzeichnet, dass** es sich bei der Verbindung der Formel (VI) um ein Hydrat oder ein Anhydrat handelt.

10. Kristallform nach einem der Ansprüche 8 bis 9, **dadurch gekennzeichnet, dass**
wobei x für 0 steht, die Kristallform Kristallform I der freien Base ist, das Röntgenpulverbeugungsmuster davon einen Beugungspeak bei 2θ (±0,2°) von 16,7, 12,6 und 14,6 aufweist; vorzugsweise Beugungspeaks bei 2θ (±0,2°) von 7,3, 13,2, 17,4, 19,4, 20,2 und 20,6 aufweist und weiter bevorzugt Beugungspeaks bei 2θ (±0,2°) von 9,2, 11,1, 11,9, 19,6, 22,3 und 25,5 aufweist;
oder
wobei x für 0 steht, die Kristallform Kristallform II der freien Base ist, das Röntgenpulverbeugungsmuster davon einen Beugungspeak bei 2θ (±0,2°) von 11,7, 13,4, 13,6, 16,6 und 18,9 aufweist, vorzugsweise ferner Beugungspeaks bei 2θ (±0,2°) von 9,5, 10,1, 14,7 und 19,3 aufweist, weiter bevorzugt ferner Beugungspeaks bei 2θ (±0,2°) von 19,6, 20,6, 20,9, 21,6, 22,1, 22,5, 22,7 und 24,4 aufweist und ferner bevorzugt das Röntgenpulverbeugungsmuster davon weitgehend wie in Figur 3 gezeigt ist;
oder
wobei x für 0 steht, die Kristallform Kristallform III der freien Base ist, das Röntgenpulverbeugungsmuster davon einen Beugungspeak bei 2θ (±0,2°) von 10,0, 11,7, 13,7, 16,6, 18,9 und 19,2 aufweist, vorzugsweise ferner Beugungspeaks bei 2θ (±0,2°) von 13,4, 19,6, 20,6, 20,9, 22,0, 22,7, 23,4 und 25,6 aufweist und ferner bevorzugt das Röntgenpulverbeugungsmuster davon weitgehend wie in Figur 5 gezeigt ist;
oder
wobei M für Methansulfonsäure steht, x für 1 steht, das Röntgenpulverbeugungsmuster davon einen Beugungspeak bei 2θ (±0,2°) von 12,5, 13,5, 19,4 und 19,9 aufweist, vorzugsweise ferner Beugungspeaks bei 2θ (±0,2°) von 15,1, 15,8, 16,5, 17,3, 18,7 und 23,1 aufweist, weiter bevorzugt ferner Beugungspeaks bei 2θ (±0,2°) von 11,1, 11,5, 13,9, 18,5, 21,3, 21,7, 26,5 und 28,9 aufweist und ferner bevorzugt das Röntgenpulverbeugungsmuster davon weitgehend wie in Figur 7 gezeigt ist.

11. Kristallform nach einem der Ansprüche 8 bis 10, **dadurch gekennzeichnet, dass** das Röntgenpulverbeugungsmuster der Kristallform I weitgehend wie in Figur 1 gezeigt ist.

12. Kristallform nach einem der Ansprüche 8 bis 10, **dadurch gekennzeichnet, dass** das DSC-Spektrum der Kristallform I einen endothermen Peak bei 151,4±0,5 °C aufweist;
oder das DSC-Spektrum der Kristallform II einen endothermen Peak bei 193,5±0,5 °C aufweist;
oder das DSC-Spektrum der Kristallform III einen endothermen Peak bei 206,4±0,5 °C aufweist.

13. Verfahren zur Herstellung der Kristallform der Verbindung nach einem der Ansprüche 8 bis 12, das im Einzelnen die folgenden Schritte umfasst:
1) Abwiegen einer geeigneten Menge der freien Base oder des Säureadditionssalzes, anschließendes Zugeben eines guten Lösungsmittels und Erhitzen der Mischung bis zur Auflösung;
2) nach mehrstündigem Rühren Zutropfen eines schlechten Lösungsmittels, bis Trübung auftritt;
3) Rühren und Abkühlen der Mischung und anschließendes Ausfällen eines Kristalls zum Erhalt des Zielprodukts;
vorzugsweise:
das gute Lösungsmittel aus der Gruppe bestehend aus Methanol, Ethanol, Essigsäureethylester, Dichlormethan, Aceton, Toluol, Acetonitril, Tetrahydrofuran, n-Heptan, Methyl-tert.-butylether, Isopropylether und N,N-Dimethylformamid und vorzugsweise 88%igem Aceton, Tetrahydrofuran und Ethanol ausgewählt wird; und
das schlechte Lösungsmittel aus der Gruppe bestehend aus n-Heptan, Methyl-tert.-butylether und Isopropylether und vorzugsweise n-Heptan und Methyl-tert.-butylether ausgewählt wird.

14. Pharmazeutische Zusammensetzung, umfassend eine therapeutisch wirksame Menge des Säureadditionssalzes der Formel (Ia) nach einem der Ansprüche 1 bis 7 oder eine therapeutisch wirksame Menge der Kristallform der Verbindung der Formel (I) nach einem der Ansprüche 8 bis 12 und einen oder mehrere pharmazeutisch unbedenkliche Träger.

15. Pharmazeutische Zusammensetzung nach Anspruch 14, **dadurch gekennzeichnet, dass** es sich bei der pharmazeutischen Zusammensetzung um eine Injektion oder eine orale Zubereitung und vorzugsweise eine Tablette oder eine Kapsel handelt.

16. Pharmazeutische Zusammensetzung nach Anspruch 14, **dadurch gekennzeichnet, dass** die Einheitsdosis 5 bis 200 mg, bevorzugt 10 bis 100 mg und weiter bevorzugt 10 bis 50 mg beträgt.

17. Säureadditionssalz der Verbindung der Formel (Ia) nach einem der Ansprüche 1 bis 7, Kristallform der Verbindung der Formel (I) nach einem der Ansprüche 8 bis 12 oder pharmazeutische Zusammensetzung nach einem der Ansprüche 14 bis 16 zur Verwendung bei der Behandlung einer durch GABA_{A}-Rezeptor-Regulator vermittelten verwandten Erkrankung, wobei es sich bei der durch GABA_{A}-Rezeptor-Regulator vermittelten verwandten Erkrankung vorzugsweise um eine mit dem Zentralnervensystem (ZNS) in Zusammenhang stehende Erkrankung handelt; die mit dem ZNS in Zusammenhang stehende Erkrankung aus der Gruppe bestehend aus Schlafstörung, Stimmungsstörung, Schizophrenie-Spektrum-Störung, spasmodischer Störung, Gedächtnisstörung, kognitiver Störung, Dyskinesie, Persönlichkeitsstörung, Autismus-Spektrum-Störung, Schmerzen, traumatischer Hirnverletzungen, Gefäßerkrankung, Stoffmissbrauchsstörung und/oder Entzugssyndrom oder Tinnitus ausgewählt ist.

## Revendications

1. Sel d'addition d'acide de formule (Ia), ayant la structure suivante : 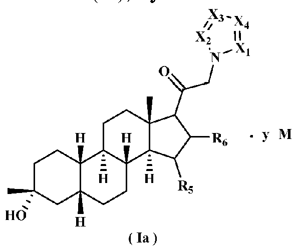 dans laquelle :
X₁ est choisi dans le groupe constitué par CR₁ et N, et de préférence N ;
X₂ est choisi dans le groupe constitué par CR₂ et N ;
X₃ est choisi dans le groupe constitué par CR₃ et N ;
X₄ est choisi dans le groupe constitué par CR₄ et N ;
R₁ et R₂ sont chacun indépendamment choisis dans le groupe constitué par hydrogène, deutérium, cyano, halogène, nitro, amino, C₁₋₈ alkyle, C₁₋₈ halogénoalkyle, C₁₋₈ alcoxy, C₁₋₈ hydroxyalkyle, C₁₋₈ alkyle substitué par cyano, C₃₋₈ cycloalkyle, C₃₋₈ cycloalkyle substitué par cyano, hétérocyclyle à 3 à 8 chaînons, C₆₋₁₀ aryle et hétéroaryle à 5 à 10 chaînons ;
R₃ et R₄ sont chacun indépendamment choisis dans le groupe constitué par hydrogène, deutérium, cyano, halogène, nitro, amino, C₁₋₈ alkyle, C₁₋₈ halogénoalkyle, C₁₋₈ alcoxy, C₁₋₈ hydroxyalkyle, C₁₋₈ alkyle substitué par cyano, C₃₋₈ cycloalkyle, C₃₋₈ cycloalkyle substitué par cyano, hétérocyclyle à 3 à 8 chaînons, C₆₋₁₀ aryle, hétéroaryle à 5 à 10 chaînons, -(CH₂)ₙR_{a,} - (CH₂)ₙOR_{a,} -(CH₂)ₙSR_{a,} -(CH₂)ₙC(O)R_{a,} -S(O)R_{a,} -S(O)₂R_{a,} -S(O)(=NH)R_{a,} - C(O)ORₐ et -C(O)O(CH₂)ₙNRₐR_{b}, les C₁₋₈ alkyle, C₁₋₈ halogénoalkyle, C₁₋₈ hydroxyalkyle, C₁₋₈ alkyle substitué par cyano, C₁₋₈ alcoxy, C₃₋₈ cycloalkyle, C₃₋₈ cycloalkyle substitué par cyano, hétérocyclyle à 3 à 8 chaînons, C₆₋₁₀ aryle et hétéroaryle à 5 à 10 chaînons pouvant être chacun en outre éventuellement substitués ;
R₅ est indépendamment choisi dans le groupe constitué par C₁₋₈ alkyle, C₂₋₈ alcényle, C₂₋₈ alcynyle, C₁₋₈ halogénoalkyle, C₁₋₈ hydroxyalkyle, C₁₋₈ alkyle substitué par cyano, C₁₋₈ alcoxy, C₁₋₈ alkylthio, C₃₋₈ cycloalkyle, C₃₋₈ cycloalkyle substitué par cyano, hétérocyclyle à 3 à 8 chaînons, C₆₋₁₀ aryle, hétéroaryle à 5 à 10 chaînons, -(CH₂)ₙR_{a,} -(CH₂)ₙOR_{a,} -S(O)R_{a,} -S(O)₂Rₐ et -(CH₂)ₙNRₐR_{b}, les C₁₋₈ alkyle, C₂₋₈ alcényle, C₂₋₈ alcynyle, C₁₋₈ halogénoalkyle, C₁₋₈ hydroxyalkyle, C₁₋₈ alkyle substitué par cyano, C₁₋₈ alcoxy, C₃₋₈ cycloalkyle, C₃₋₈ cycloalkyle substitué par cyano, hétérocyclyle à 3 à 8 chaînons, C₆₋₁₀ aryle et hétéroaryle à 5 à 10 chaînons pouvant être chacun en outre éventuellement substitués ;
R₆ est hydrogène ;
ou, R₅ et R₆ conjointement avec les atomes de carbone auxquels ils sont fixés forment un C₃₋₈ cycloalkyle ou hétérocyclyle à 3 à 8 chaînons, les C₃₋₈ cycloalkyle et hétérocyclyle à 3 à 8 chaînons pouvant être chacun en outre éventuellement substitués ;
Rₐ et R_{b} sont chacun indépendamment choisis dans le groupe constitué par hydrogène, deutérium, halogène, cyano, hydroxy, amino, nitro, C₁₋₈ alkyle, C₁₋₈ alcoxy, C₁₋₈ hydroxyalkyle, C₁₋₈ alkyle deutéré, C₁₋₈ halogénoalkyle, C₃₋8 cycloalkyle, hétérocyclyle à 3 à 8 chaînons, C₆₋₁₀ aryle et hétéroaryle à 5 à 10 chaînons, les C₁₋₈ alkyle, C₁₋₈ alkyle deutéré, C₁₋₈ halogénoalkyle, C₃₋₈ cycloalkyle, hétérocyclyle à 3 à 8 chaînons, C₆₋₁₀ aryle et hétéroaryle à 5 à 10 chaînons pouvant être chacun en outre éventuellement substitués ;
M est un acide inorganique ou un acide organique, dans laquelle l'acide inorganique est choisi dans le groupe constitué par l'acide chlorhydrique, l'acide sulfurique, l'acide nitrique, l'acide bromhydrique, l'acide fluorhydrique, l'acide iodhydrique et l'acide phosphorique, et l'acide organique est choisi dans le groupe constitué par l'acide 2,5-dihydroxybenzoïque, l'acide 1-hydroxy-2-naphtoïque, l'acide acétique, l'acide dichloroacétique, l'acide trichloroacétique, l'acide acétohydroxamique, l'acide adipique, l'acide benzènesulfonique, l'acide 4-chlorobenzènesulfonique, l'acide benzoïque, l'acide 4-acétylaminobenzoïque, l'acide 4-aminobenzoïque, l'acide caprique, l'acide caproïque, l'acide caprylique, l'acide cinnamique, l'acide citrique, l'acide cyclamique, l'acide camphorsulfonique, l'acide aspartique, l'acide camphrénique, l'acide gluconique, l'acide glucuronique, l'acide glutamique, l'acide érythorbique, l'acide lactique, l'acide malique, l'acide mandélique, l'acide pyroglutamique, l'acide tartrique, l'acide laurylsulfurique, l'acide dibenzoyltartrique, l'acide éthane-1,2-disulfonique, l'acide éthanesulfonique, l'acide formique, l'acide fumarique, l'acide galactonique, l'acide gentisique, l'acide glutarique, l'acide 2-cétoglutarique, l'acide glycolique, l'acide hippurique, l'acide iséthionique, l'acide lactobionique, l'acide ascorbique, l'acide asparagique, l'acide laurique, l'acide camphrénique, l'acide maléique, l'acide malonique, l'acide D-tartrique, l'acide pamoïque, l'acide méthanesulfonique, l'acide 1,5-naphtalènedisulfonique, l'acide naphtalène-2-sulfonique, l'acide nicotinique, l'acide oléique, l'acide orotique, l'acide oxalique, l'acide palmitique, l'acide embonique, l'acide propionique, l'acide salicylique, l'acide 4-aminosalicylique, l'acide sébacique, l'acide stéarique, l'acide succinique, l'acide thiocyanique, l'acide undécylénique, l'acide trifluoroacétique, l'acide p-toluènesulfonique et l'acide L-malique ; et
y est choisi dans le groupe constitué par 1, 2 et 3 ;
n est un entier de 0 à 6.

2. Sel d'addition d'acide selon la revendication 1, **caractérisé en ce que**,
R₁ et R₂ sont chacun indépendamment choisis dans le groupe constitué par hydrogène, fluor, chlore, cyano, méthyle et cyclopropyle ;
R₃ et R₄ sont chacun indépendamment choisis dans le groupe constitué par hydrogène, halogène, cyano, nitro, C₁₋₃ alkyle, C₁₋₃ alkyle substitué par cyano, C₁₋₃ alkyle substitué par halogène, C₁₋₃ hydroxyalkyle, C₃₋₆ cycloalkyle, C₃₋₆ cycloalkyle substitué par cyano, oxazolyle, -C(O)R_{a,} -SC₁₋₃ alkyle, -S(O)C₁₋₃ alkyle, -S(O)₂C₁₋₃ alkyle, -C(O)OC₁₋₃ alkyle, - S(O)(=NH)C₁₋₃ alkyle et -C(O)O(CH₂)ₙN(CH₃)C₁₋₃ alkyle, et de préférence hydrogène, fluor, chlore, cyano, nitro, méthyle, trifluorométhyle, cyclopropyle, cyclopropyle substitué par cyano, isopropyle substitué par cyano, hydroxyisopropyle, oxazolyle, -C(O)R_{a,} -SCH₃, -S(O)CH₃, - S(O)₂CH₃, -C(O)OCH₂CH₃, -S(O)(=NH)CH₃ et -C(O)O(CH₂)₂NCH₃(CH₃) ;
R₅ est choisi dans le groupe constitué par C₁₋₃ alkyle, C₁₋₃ halogénoalkyle, C₁₋₃ alcoxy, C₁₋₃ hydroxyalkyle, C₁₋₃ alkyle substitué par cyano, C₃₋₆ cycloalkyle, C₃₋₆ cycloalkyle substitué par cyano, hétérocyclyle contenant azote à 3 à 6 chaînons, C₆₋₁₀ aryle, hétéroaryle à 5 à 10 chaînons, C₁₋₃ alkylthio, -(CH₂)ₙR_{a,} -(CH₂)ₙOR_{a,} -S(O)₂Rₐ et -(CH₂)ₙN(Rₐ)₂, et de préférence hydrogène, fluor, chlore, cyano, méthyle, éthyle, isopropyle, fluorométhyle, trifluorométhyle, méthoxy, cyclopropyle, cyclobutyle, pyrazolyle substitué par méthyle, phényle, -SCH₃, -(CH₂)ₙRₐ, -NC(CH₃)₂, -S(O)₂C(CH₃)₂ et -CH₂N(CH₃)₂ ;
R₆ est hydrogène ;
ou, R₅ et R₆ conjointement avec les atomes de carbone auxquels ils sont fixés forment un C₃₋₆ cycloalkyle, et de préférence cyclopropyle, le cyclopropyle étant éventuellement substitué par halogène, et de préférence par fluor ;
Rₐ est choisi dans le groupe constitué par hydrogène, nitro, C₁₋₃ alkyle, azétidinyle et pyrrolidinyle, les azétidinyle et pyrrolidinyle étant chacun éventuellement substitués par halogène, et de préférence par fluor ;
M est choisi dans le groupe constitué par l'acide chlorhydrique, l'acide sulfurique, l'acide méthanesulfonique, l'acide p-toluènesulfonique, l'acide bromhydrique, l'acide nitrique et l'acide 1,5-naphtalènedisulfonique, et de préférence l'acide méthanesulfonique ;
y est 1 ou 2 ;
n est indépendamment choisi dans le groupe constitué par 0, 1 et 2.

3. Sel d'addition d'acide selon la revendication 1 ou 2, ayant une structure telle que présentée dans la formule (IIa) : 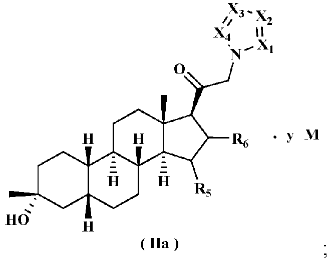 ou, ayant une structure telle que présentée dans la formule (IIIa) : 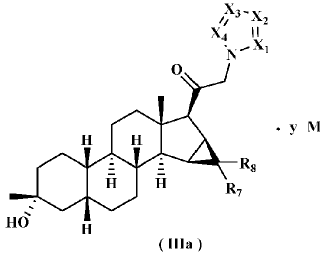 dans laquelle :
R₇ et R₈ sont chacun choisis indépendamment dans le groupe constitué par hydrogène, fluor, chlore, brome et méthyle, et de préférence hydrogène et fluor ;
ou ayant une structure telle que présentée dans la formule (IVa) : 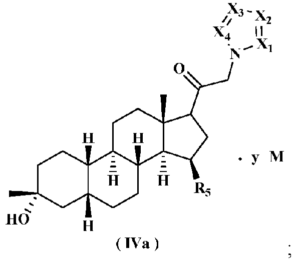
ou ayant une structure telle que présentée dans la formule (Va) :

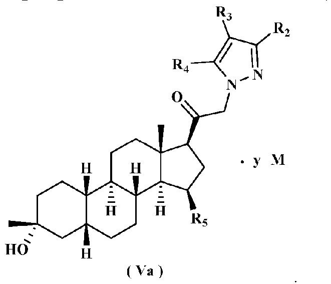

4. Sel d'addition d'acide selon l'une quelconque des revendications 1 à 3, **caractérisé en ce que** sa structure est comme suit :

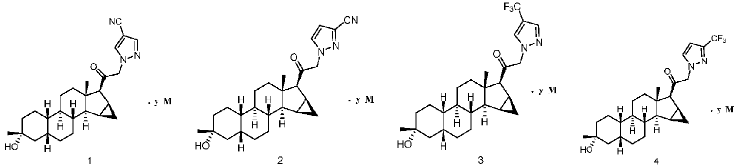

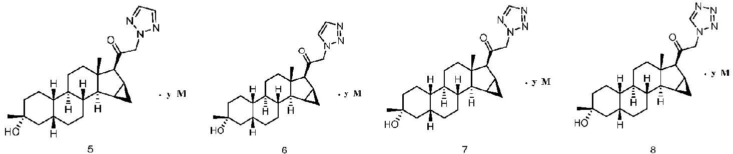

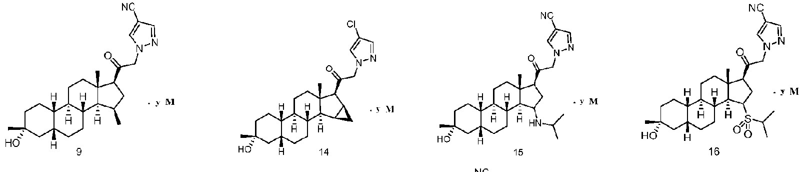

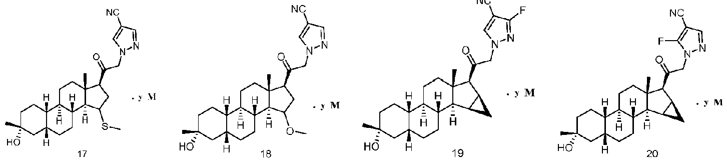

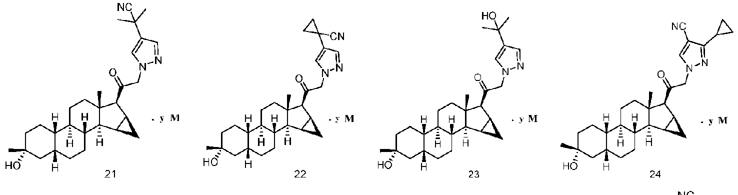

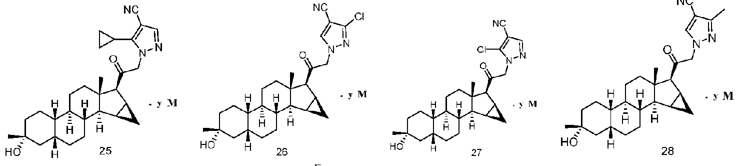

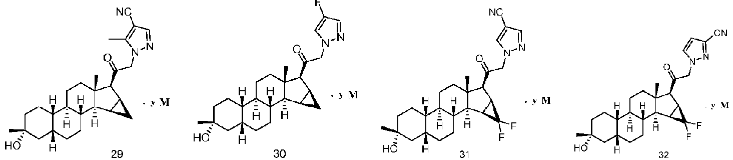

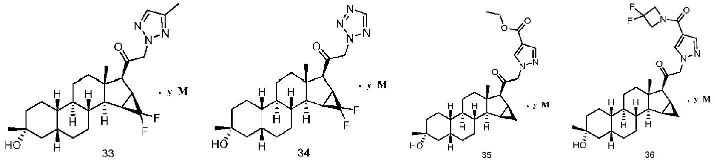

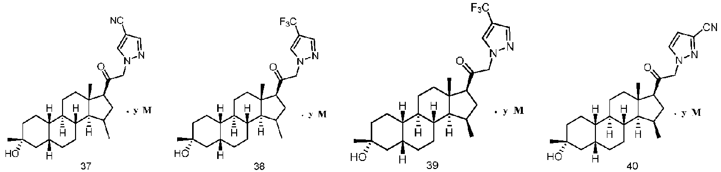

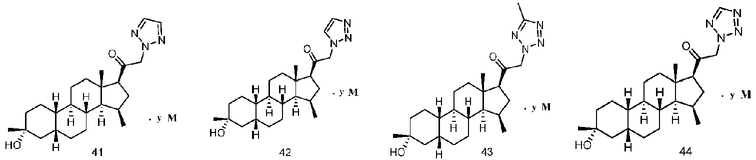

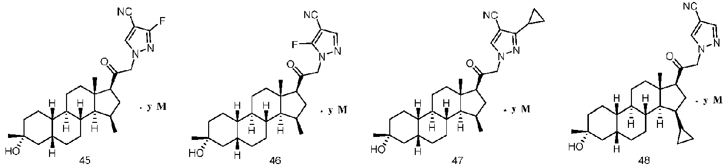

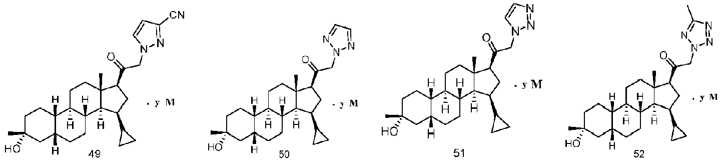

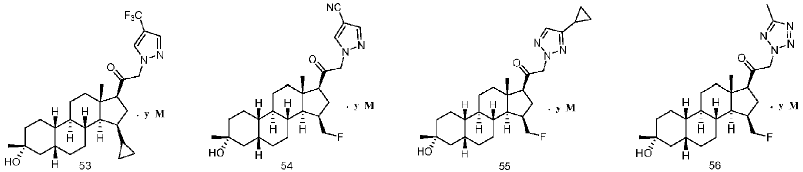

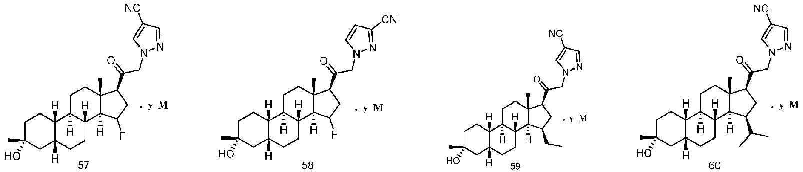

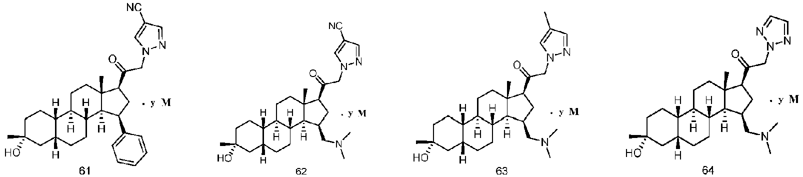

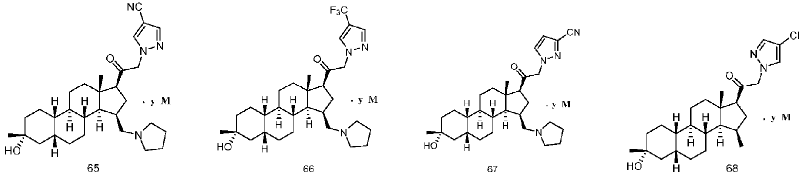

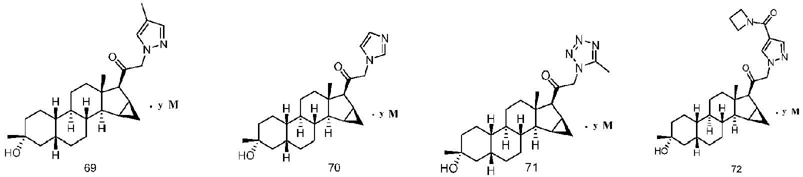

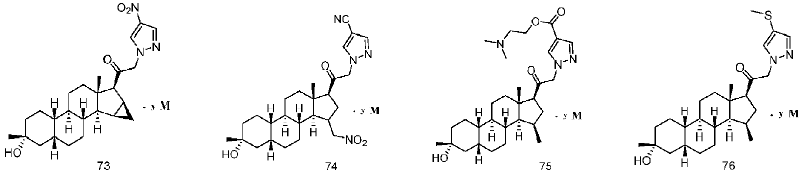

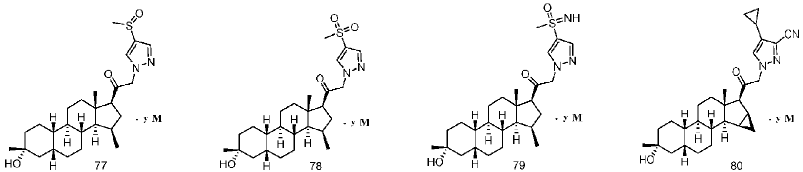

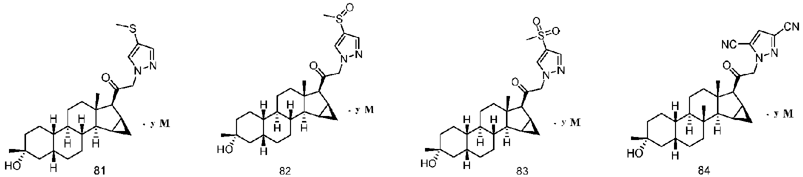

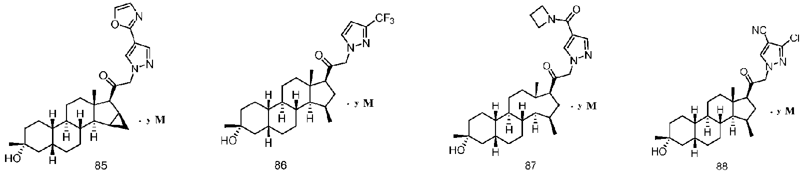

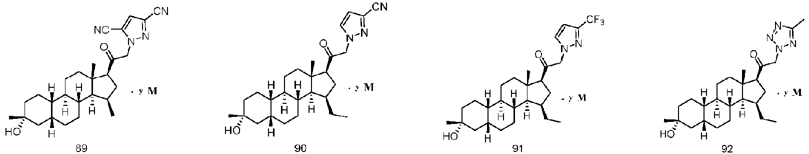

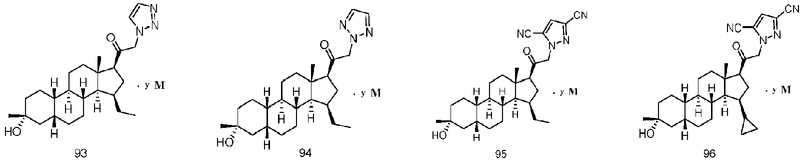

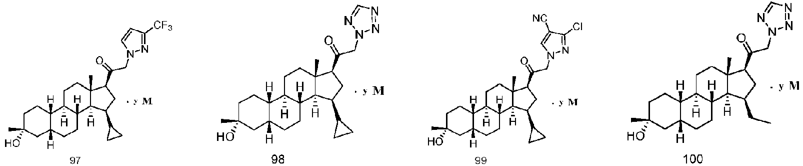

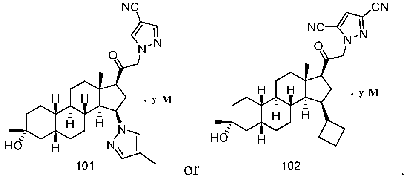

5. Sel d'addition d'acide selon la revendication 4, **caractérisé en ce que** sa structure est comme suit :

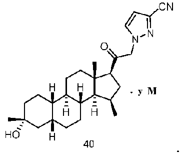

6. Sel d'addition d'acide selon la revendication 4, **caractérisé en ce que** sa structure est comme suit : 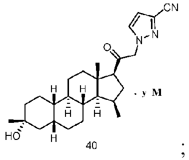 dans laquelle M est l'acide méthanesulfonique et y est 1.

7. Sel d'addition d'acide selon l'une quelconque des revendications 1 à 6, **caractérisé en ce que** le sel d'addition d'acide est cristallin ou amorphe.

8. Forme cristalline d'un composé de formule (VI), ayant la structure suivante : 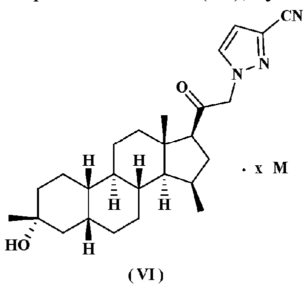
M est un acide inorganique ou un acide organique, dans laquelle l'acide inorganique est choisi dans le groupe constitué par l'acide chlorhydrique, l'acide sulfurique, l'acide nitrique, l'acide bromhydrique, l'acide fluorhydrique, l'acide iodhydrique et l'acide phosphorique, et l'acide organique est choisi dans le groupe constitué par l'acide 2,5-dihydroxybenzoïque, l'acide 1-hydroxy-2-naphtoïque, l'acide acétique, l'acide dichloroacétique, l'acide trichloroacétique, l'acide acétohydroxamique, l'acide adipique, l'acide benzènesulfonique, l'acide 4-chlorobenzènesulfonique, l'acide benzoïque, l'acide 4-acétylaminobenzoïque, l'acide 4-aminobenzoïque, l'acide caprique, l'acide caproïque, l'acide caprylique, l'acide cinnamique, l'acide citrique, l'acide cyclamique, l'acide camphorsulfonique, l'acide aspartique, l'acide camphrénique, l'acide gluconique, l'acide glucuronique, l'acide glutamique, l'acide érythorbique, l'acide lactique, l'acide malique, l'acide mandélique, l'acide pyroglutamique, l'acide tartrique, l'acide laurylsulfurique, l'acide dibenzoyltartrique, l'acide éthane-1,2-disulfonique, l'acide éthanesulfonique, l'acide formique, l'acide fumarique, l'acide galactonique, l'acide gentisique, l'acide glutarique, l'acide 2-cétoglutarique, l'acide glycolique, l'acide hippurique, l'acide iséthionique, l'acide lactobionique, l'acide ascorbique, l'acide asparagique, l'acide laurique, l'acide camphrénique, l'acide maléique, l'acide malonique, l'acide D-tartrique, l'acide pamoïque, l'acide méthanesulfonique, l'acide 1,5-naphtalènedisulfonique, l'acide naphtalène-2-sulfonique, l'acide nicotinique, l'acide oléique, l'acide orotique, l'acide oxalique, l'acide palmitique, l'acide embonique, l'acide propionique, l'acide salicylique, l'acide 4-aminosalicylique, l'acide sébacique, l'acide stéarique, l'acide succinique, l'acide thiocyanique, l'acide undécylénique, l'acide trifluoroacétique, l'acide p-toluènesulfonique et l'acide L-malique ; et
x est 0, 1 ou 2.

9. Forme cristalline selon la revendication 8, **caractérisée en ce que** le composé de formule (VI) est un hydrate ou un anhydrate.

10. Forme cristalline selon l'une quelconque des revendications 8 à 9, **caractérisée en ce que**,
dans laquelle x est 0, la forme cristalline est la forme cristalline I de base libre, son diagramme de diffraction des rayons X sur poudre présente un pic de diffraction à 2θ (±0,2°) de 16,7, 12,6 et 14,6 ; présente de préférence des pics de diffraction à 2θ (±0,2°) de 7,3, 13,2, 17,4, 19,4, 20,2 et 20,6, et présente, plus préférablement, des pics de diffraction à 2θ (±0,2°) de 9,2, 11,1, 11,9, 19,6, 22,3 et 25,5 ;
ou,
dans laquelle x est 0, la forme cristalline est la forme cristalline II de base libre, son diagramme de diffraction des rayons X sur poudre présente des pics de diffraction à 2θ (±0,2°) de 11,7, 13,4, 13,6, 16,6 et 18,9, de préférence a en outre des pics de diffraction à 2θ (±0,2°) de 9,5, 10,1, 14,7 et 19,3, plus préférablement a en outre des pics de diffraction à 2θ (±0,2°) de 19,6, 20,6, 20,9, 21,6, 22,1, 22,5, 22,7 et 24,4, et de préférence encore, son diagramme de diffraction des rayons X sur poudre est sensiblement tel que présenté sur la figure 3 ;
ou,
dans laquelle x est 0, la forme cristalline est la forme cristalline III de base libre, son diagramme de diffraction des rayons X sur poudre présente des pics de diffraction à 2θ (±0,2°) de 10,0, 11,7, 13,7, 16,6, 18,9 et 19,2, de préférence présente en outre des pics de diffraction à 2θ (±0,2°) de 13,4, 19,6, 20,6, 20,9, 22,0, 22,7, 23,4 et 25,6, et de préférence encore, son diagramme de diffraction des rayons X sur poudre est sensiblement tel que présenté sur la figure 5 ;
ou,
dans laquelle M est l'acide méthanesulfonique, x est 1, son diagramme de diffraction des rayons X sur poudre présente des pics de diffraction à 2θ (±0,2°) de 12,5, 13,5, 19,4 et 19,9, de préférence présente en outre des pics de diffraction à 2θ (±0,2°) de 15,1, 15,8, 16,5, 17,3, 18,7 et 23,1, plus préférablement présente en outre des pics de diffraction à 2θ (±0,2°) de 11,1, 11,5, 13,9, 18,5, 21,3, 21,7, 26,5 et 28,9, et de préférence encore, le diagramme de diffraction des rayons X sur poudre de celui-ci est sensiblement tel que présenté sur la figure 7.

11. Forme cristalline selon l'une quelconque des revendications 8 à 10, **caractérisée en ce que** le diagramme de diffraction des rayons X sur poudre de la forme cristalline I est sensiblement tel que présenté sur la figure 1.

12. Forme cristalline selon l'une quelconque des revendications 8 à 10, **caractérisée en ce que**,
le spectre DSC de la forme cristalline I présente un pic endothermique à 151,4±0,5 °C ;
ou, le spectre DSC de la forme cristalline II présente un pic endothermique à 193,5±0,5 °C ;
ou, le spectre DSC de la forme cristalline III présente un pic endothermique à 206,4±0,5 °C.

13. Procédé de préparation de la forme cristalline du composé selon l'une quelconque des revendications 8 à 12, comprenant spécifiquement les étapes suivantes de :
1) pesée d'une quantité appropriée de la base libre ou du sel d'addition d'acide, puis ajout d'un bon solvant et chauffage du mélange jusqu'à dissolution ;
2) après plusieurs heures d'agitation, ajout goutte à goutte d'un mauvais solvant jusqu'à apparition de turbidité ;
3) agitation et refroidissement du mélange, suivi par précipitation d'un cristal pour obtenir le produit cible ;
de préférence :
le bon solvant est sélectionné dans le groupe constitué par le méthanol, l'éthanol, l'acétate d'éthyle, le dichlorométhane, l'acétone, le toluène, l'acétonitrile, le tétrahydrofurane, le n-heptane, l'éther méthylique tert-butylique, l'éther isopropylique et le N,N-diméthylformamide ; et de préférence 88 % acétone, tétrahydrofurane et éthanol ; et
le mauvais solvant est choisi dans le groupe constitué par le n-heptane, le méthyltert-butyléther et l'isopropyléther ; et de préférence le n-heptane et le méthyltert-butyléther.

14. Composition pharmaceutique, comprenant une quantité thérapeutiquement efficace du sel d'addition d'acide de formule (Ia) selon l'une quelconque des revendications 1 à 7 ou une quantité thérapeutiquement efficace de la forme cristalline du composé de formule (I) selon l'une quelconque des revendications 8 à 12, et un ou plusieurs supports pharmaceutiquement acceptables.

15. Composition pharmaceutique selon la revendication 14, **caractérisée en ce que** la composition pharmaceutique est une injection ou une préparation orale, et de préférence un comprimé ou une capsule.

16. Composition pharmaceutique selon la revendication 14, **caractérisée en ce que** la dose unitaire est de 5 à 200 mg, de préférence de 10 à 100 mg, et plus préférablement de 10 à 50 mg.

17. Sel d'addition d'acide du composé de formule (Ia) selon l'une quelconque des revendications 1 à 7, forme cristalline du composé de formule (I) selon l'une quelconque des revendications 8 à 12 ou composition pharmaceutique selon l'une quelconque des revendications 14 à 16 pour une utilisation dans le traitement d'une maladie liée médiée par le régulateur des récepteurs GABA_{A}, où la maladie liée médiée par le régulateur des récepteurs GABA_{A} est de préférence une maladie liée au système nerveux central (SNC) ; la maladie liée au SNC est choisie dans le groupe constitué par un trouble du sommeil, un trouble de l'humeur, un trouble du spectre de la schizophrénie, un trouble spasmodique, un trouble de la mémoire, un trouble cognitif, une dyskinésie, un trouble de la personnalité, un trouble du spectre autistique, une douleur, un traumatisme crânien, une maladie vasculaire, un trouble lié à l'abus de substances et/ou un syndrome de sevrage ou des acouphènes.
